# EUROPEAN PATENT APPLICATION

(11) **EP 2 305 791 A1**
(43) Date of publication of application: **06.04.2011**
(21) Application number: 10011467.7
(22) Date of filing: 14.04.2006
(51) Int. Cl.: C12N 1/36, C12P 9/00, C12N 1/20, A23L 1/30, A61K 35/74

(54) **Methods and compositions to modulate adhesion and stress tolerance in bacteria**

(30) Priority: 15.04.2005 US 671887 P
(62) Divisional of application: 06750233.6
(71) Applicant: NORTH CAROLINA STATE UNIVERSITY, Raleigh, NC 27695-8210 (US)
(72) Inventor: Buck, Logan B., Banner Elk North Carolina 28604 (US); Azcarate-Peril, Andrea, Raleigh North Carolina 27607 (US); Klaenhammer, Todd R., Raleigh North Carolina 27606 (US); Altermann, Eric, Palmerston North (NZ)
(74) Representative: Blance, Stephen John

(57) **Abstract**

Methods and compositions are provided which improve the adhesion of a bacteria to target substrates and/or improve the stress tolerance of a bacteria. Methods comprise exposing bacteria to adhesion adaptive conditions and thereby increasing the adhesion activity and/or stress tolerance of the bacteria. Further provided are bacteria having a modulated production of autoinducer-2. Compositions include recombinant bacteria expressing nucleic acid molecules involved in the pathway of autoinducer-2 production. Further provided are autoinducer-2-related fusion proteins, antigenic peptides, antibodies, and vectors. Methods of screening compounds or environmental conditions which will stimulate the production of autoinducer-2 and/or produce an adhesion adaptive response are further provided, as are various methods of use for the bacteria having increased adhesion and/or stress tolerance.

SEQUENCE LISTING

<110> North Carolina State University

<120> Methods and compositions to modulate adhesion and stress tolerance in
bacteria

<130> P024139EPB

<150> US 60/671,887
<151> 2005-04-15

<160> 38

<170> FastSEQ for Windows Version 4.0

<210> 1
<211> 693
<212> DNA
<213> Lactobacillus acidophilus

<220>
<221> CDS
<222> (1)...(693)
<223> MTA/SAH nucleosidase (E.C.3.2.2.9) ORF Lba820

<400> 1
atg aag att gcg att atc gtt cca atg gct gaa gaa gct gaa ttt tat     48
Met Lys Ile Ala Ile Ile Val Pro Met Ala Glu Glu Ala Glu Phe Tyr
 1               5                  10                  15

cgt aat cat ttc cat tca gaa aat aaa gaa atg ttc gga tca act gaa     96
Arg Asn His Phe His Ser Glu Asn Lys Glu Met Phe Gly Ser Thr Glu
            20                  25                  30

ttc gaa cat ttt tct gtt aat ggc aat gat gta tat ttg ggt tta agt     144
Phe Glu His Phe Ser Val Asn Gly Asn Asp Val Tyr Leu Gly Leu Ser
        35                  40                  45

ggt att ggt aaa gtc caa gca gct atg aat ctt tca agt ttg tta aca     192
Gly Ile Gly Lys Val Gln Ala Ala Met Asn Leu Ser Ser Leu Leu Thr
    50                  55                  60

agt gtt gac ata gat gta ata ttt atg act ggt tca gct ggt gca ctt     240
Ser Val Asp Ile Asp Val Ile Phe Met Thr Gly Ser Ala Gly Ala Leu
65                  70                  75                  80

aag gaa gat gtt cat caa gaa gat cta gtt tta cca aat gca ttt gcc     288
Lys Glu Asp Val His Gln Glu Asp Leu Val Leu Pro Asn Ala Phe Ala
                85                  90                  95

tat tat gat gcc cat aat act gca gct ggt gat tat gta gaa gga caa     336
Tyr Tyr Asp Ala His Asn Thr Ala Ala Gly Asp Tyr Val Glu Gly Gln
            100                 105                 110

att cct caa cag cca gct caa ttt gta ctt gat tca cca gag cgt gca     384
Ile Pro Gln Gln Pro Ala Gln Phe Val Leu Asp Ser Pro Glu Arg Ala
        115                 120                 125

gct ttt gca aat tac tta aag aag caa aag gtt gct ttt cgt gaa gga     432
Ala Phe Ala Asn Tyr Leu Lys Lys Gln Lys Val Ala Phe Arg Glu Gly
    130                 135                 140

ttg gtt gta aca ggt gat tca ttt att gct tca aat gag caa aaa gat     480
Leu Val Val Thr Gly Asp Ser Phe Ile Ala Ser Asn Glu Gln Lys Asp
145                 150                 155                 160

atg ata aag aag aac ttt cca gat gcc tta tgt gtt gaa atg gaa gga     528
Met Ile Lys Lys Asn Phe Pro Asp Ala Leu Cys Val Glu Met Glu Gly
                165                 170                 175

gca gct ttt gcg caa gtc gct aat gca ttt aag aaa cca tta gtt gct     576
Ala Ala Phe Ala Gln Val Ala Asn Ala Phe Lys Lys Pro Leu Val Ala
            180                 185                 190

atg cga gca att tct gat aat ggt gat ggc tca gct aac gaa gac ttt     624
Met Arg Ala Ile Ser Asp Asn Gly Asp Gly Ser Ala Asn Glu Asp Phe
        195                 200                 205

gat act ttt gta aag aaa gta gga gct aag gca gct aaa tta att agt     672
Asp Thr Phe Val Lys Lys Val Gly Ala Lys Ala Ala Lys Leu Ile Ser
    210                 215                 220

gat tac gta gaa gag ttg aat                                         693
Asp Tyr Val Glu Glu Leu Asn
225                 230

<210> 2
<211> 231
<212> PRT
<213> Lactobacillus acidophilus

<400> 2
Met Lys Ile Ala Ile Ile Val Pro Met Ala Glu Glu Ala Glu Phe Tyr
 1               5                  10                  15
Arg Asn His Phe His Ser Glu Asn Lys Glu Met Phe Gly Ser Thr Glu
            20                  25                  30
Phe Glu His Phe Ser Val Asn Gly Asn Asp Val Tyr Leu Gly Leu Ser
        35                  40                  45
Gly Ile Gly Lys Val Gln Ala Ala Met Asn Leu Ser Ser Leu Leu Thr
    50                  55                  60
Ser Val Asp Ile Asp Val Ile Phe Met Thr Gly Ser Ala Gly Ala Leu
65                  70                  75                  80
Lys Glu Asp Val His Gln Glu Asp Leu Val Leu Pro Asn Ala Phe Ala
                85                  90                  95
Tyr Tyr Asp Ala His Asn Thr Ala Ala Gly Asp Tyr Val Glu Gly Gln
            100                 105                 110
Ile Pro Gln Gln Pro Ala Gln Phe Val Leu Asp Ser Pro Glu Arg Ala
        115                 120                 125
Ala Phe Ala Asn Tyr Leu Lys Lys Gln Lys Val Ala Phe Arg Glu Gly
    130                 135                 140
Leu Val Val Thr Gly Asp Ser Phe Ile Ala Ser Asn Glu Gln Lys Asp
145                 150                 155                 160
Met Ile Lys Lys Asn Phe Pro Asp Ala Leu Cys Val Glu Met Glu Gly
                165                 170                 175
Ala Ala Phe Ala Gln Val Ala Asn Ala Phe Lys Lys Pro Leu Val Ala
            180                 185                 190
Met Arg Ala Ile Ser Asp Asn Gly Asp Gly Ser Ala Asn Glu Asp Phe
        195                 200                 205
Asp Thr Phe Val Lys Lys Val Gly Ala Lys Ala Ala Lys Leu Ile Ser
    210                 215                 220
Asp Tyr Val Glu Glu Leu Asn
225                 230

<210> 3

<211> 942
<212> DNA
<213> Lactobacillus acidophilus

<220>
<221> CDS
<222> (1)...(942)
<223> SAM-dependent methyltransferase ORF LBA931

<400> 3
atg aaa tta ttg att att aaa att gat act agt tat gaa gta gag gat 48
Met Lys Leu Leu Ile Ile Lys Ile Asp Thr Ser Tyr Glu Val Glu Asp
1               5                   10                  15

gca tta ggc gtt ttt gca act gat aat tta aaa gcg tta ggc att gag 96
Ala Leu Gly Val Phe Ala Thr Asp Asn Leu Lys Ala Leu Gly Ile Glu
            20                  25                  30

tcc aga aaa cgt agt gat ttt gaa caa gct ggt tgg tta cat gat tca 144
Ser Arg Lys Arg Ser Asp Phe Glu Gln Ala Gly Trp Leu His Asp Ser
        35                  40                  45

act gtt gta gaa atg gat gac att aaa gac ttg cct aaa gat act tat 192
Thr Val Val Glu Met Asp Asp Ile Lys Asp Leu Pro Lys Asp Thr Tyr
    50                  55                  60

ttt tat gct tac ttt gat gaa gaa gct gat aaa gat gag ctt gta gag 240
Phe Tyr Ala Tyr Phe Asp Glu Glu Ala Asp Lys Asp Glu Leu Val Glu
65                  70                  75                  80

aaa ttt caa gca aaa ctt gaa gag ctt aag agt tat ggt tta aat act 288
Lys Phe Gln Ala Lys Leu Glu Glu Leu Lys Ser Tyr Gly Leu Asn Thr
                85                  90                  95

ggt gaa ggt aaa att aca act tct tat att gaa gat caa gat tgg aat 336
Gly Glu Gly Lys Ile Thr Thr Ser Tyr Ile Glu Asp Gln Asp Trp Asn
            100                 105                 110

aca gct tgg caa aag tat tat cat gta ata gac ttt tcg cgg cac tta 384
Thr Ala Trp Gln Lys Tyr Tyr His Val Ile Asp Phe Ser Arg His Leu
        115                 120                 125

gct att gtt cct gaa tgg gaa gat tat caa cca gct ttt agc gat caa 432
Ala Ile Val Pro Glu Trp Glu Asp Tyr Gln Pro Ala Phe Ser Asp Gln
    130                 135                 140

caa ctc att aag ctt gat cca ggt tta gca ttc ggt aca gga aat cat 480
Gln Leu Ile Lys Leu Asp Pro Gly Leu Ala Phe Gly Thr Gly Asn His
145                 150                 155                 160

aaa aca act caa ttg gca atg atg gga att gag cgt gct atg gta aaa 528
Lys Thr Thr Gln Leu Ala Met Met Gly Ile Glu Arg Ala Met Val Lys
                165                 170                 175

cca atg agt gta gtt gat gtt ggt act ggc tct ggt att tta gct att 576
Pro Met Ser Val Val Asp Val Gly Thr Gly Ser Gly Ile Leu Ala Ile
            180                 185                 190

gcc gct tct aaa tta ggt gca aca aat gtt tta gca act gat att tct 624
Ala Ala Ser Lys Leu Gly Ala Thr Asn Val Leu Ala Thr Asp Ile Ser
        195                 200                 205

gat gaa tct atg act gct gct aag caa aat tca gca ttg aat aac cta 672
Asp Glu Ser Met Thr Ala Ala Lys Gln Asn Ser Ala Leu Asn Asn Leu

210                 215                 220

act aat att aag gtt caa aaa act agc cta ctt gca ggc gta gaa ggt      720
Thr Asn Ile Lys Val Gln Lys Thr Ser Leu Leu Ala Gly Val Glu Gly
225                 230                 235                 240

aaa ttt gat att att gtt gcc aat att tta gct gaa att tta tta gat      768
Lys Phe Asp Ile Ile Val Ala Asn Ile Leu Ala Glu Ile Leu Leu Asp
                245                 250                 255

tta att cct caa atg gat gcc cat ttg aat aag gat gga caa gta atc      816
Leu Ile Pro Gln Met Asp Ala His Leu Asn Lys Asp Gly Gln Val Ile
            260                 265                 270

ttt tcg ggt atc gat tat ttg caa tta cct aaa att aaa aaa tca ttg      864
Phe Ser Gly Ile Asp Tyr Leu Gln Leu Pro Lys Ile Lys Lys Ser Leu
        275                 280                 285

gat gaa aac aac ttt aag ata gat ttg act atg aaa caa gga cgg tgg      912
Asp Glu Asn Asn Phe Lys Ile Asp Leu Thr Met Lys Gln Gly Arg Trp
    290                 295                 300

ata ggt cta gca att act aga aaa gaa aaa                              942
Ile Gly Leu Ala Ile Thr Arg Lys Glu Lys
305                 310


<210> 4
<211> 314
<212> PRT
<213> Lactobacillus acidophilus

<400> 4
Met Lys Leu Leu Ile Ile Lys Ile Asp Thr Ser Tyr Glu Val Glu Asp
1               5                   10                  15
Ala Leu Gly Val Phe Ala Thr Asp Asn Leu Lys Ala Leu Gly Ile Glu
            20                  25                  30
Ser Arg Lys Arg Ser Asp Phe Glu Gln Ala Gly Trp Leu His Asp Ser
        35                  40                  45
Thr Val Val Glu Met Asp Asp Ile Lys Asp Leu Pro Lys Asp Thr Tyr
    50                  55                  60
Phe Tyr Ala Tyr Phe Asp Glu Glu Ala Asp Lys Asp Glu Leu Val Glu
65                  70                  75                  80
Lys Phe Gln Ala Lys Leu Glu Glu Leu Lys Ser Tyr Gly Leu Asn Thr
                85                  90                  95
Gly Glu Gly Lys Ile Thr Thr Ser Tyr Ile Glu Asp Gln Asp Trp Asn
            100                 105                 110
Thr Ala Trp Gln Lys Tyr Tyr His Val Ile Asp Phe Ser Arg His Leu
        115                 120                 125
Ala Ile Val Pro Glu Trp Glu Asp Tyr Gln Pro Ala Phe Ser Asp Gln
    130                 135                 140
Gln Leu Ile Lys Leu Asp Pro Gly Leu Ala Phe Gly Thr Gly Asn His
145                 150                 155                 160
Lys Thr Thr Gln Leu Ala Met Met Gly Ile Glu Arg Ala Met Val Lys
                165                 170                 175
Pro Met Ser Val Val Asp Val Gly Thr Gly Ser Gly Ile Leu Ala Ile
            180                 185                 190
Ala Ala Ser Lys Leu Gly Ala Thr Asn Val Leu Ala Thr Asp Ile Ser
        195                 200                 205
Asp Glu Ser Met Thr Ala Ala Lys Gln Asn Ser Ala Leu Asn Asn Leu
    210                 215                 220
Thr Asn Ile Lys Val Gln Lys Thr Ser Leu Leu Ala Gly Val Glu Gly
225                 230                 235                 240
Lys Phe Asp Ile Ile Val Ala Asn Ile Leu Ala Glu Ile Leu Leu Asp

245                 250                 255
Leu Ile Pro Gln Met Asp Ala His Leu Asn Lys Asp Gly Gln Val Ile
            260                 265                 270
Phe Ser Gly Ile Asp Tyr Leu Gln Leu Pro Lys Ile Lys Lys Ser Leu
        275                 280                 285
Asp Glu Asn Asn Phe Lys Ile Asp Leu Thr Met Lys Gln Gly Arg Trp
    290                 295                 300
Ile Gly Leu Ala Ile Thr Arg Lys Glu Lys
305                 310

<210> 5
<211> 657
<212> DNA
<213> Lactobacillus acidophilus

<220>
<221> CDS
<222> (1)...(657)
<223> ABC transporter membrane-spanning permease ORF
      LBA1042

<400> 5
atg aat tgg acg att atc cag cag gca atg cct gca ttt atg gca ggt      48
Met Asn Trp Thr Ile Ile Gln Gln Ala Met Pro Ala Phe Met Ala Gly
 1               5                  10                  15

ttc aag cta aca ctt tgg tta tcg tta gtt gga atc att gga tca ata      96
Phe Lys Leu Thr Leu Trp Leu Ser Leu Val Gly Ile Ile Gly Ser Ile
            20                  25                  30

ata gtg gga att ata gtc agt tta ttt caa tat ttt aaa gta cct gtt     144
Ile Val Gly Ile Ile Val Ser Leu Phe Gln Tyr Phe Lys Val Pro Val
        35                  40                  45

tta agt caa ata tct acc gca tat gta gaa ctt gca cga aac aca cca     192
Leu Ser Gln Ile Ser Thr Ala Tyr Val Glu Leu Ala Arg Asn Thr Pro
    50                  55                  60

tta tta att caa cta ttt ttc tta tat tat gcg ttt cca atc ttc ggt     240
Leu Leu Ile Gln Leu Phe Phe Leu Tyr Tyr Ala Phe Pro Ile Phe Gly
65                  70                  75                  80

tta aag atg tcc gca gaa gtc tgc ggt att atc ggc ttg atc ttt tta     288
Leu Lys Met Ser Ala Glu Val Cys Gly Ile Ile Gly Leu Ile Phe Leu
                85                  90                  95

ggt ggg gca tat atg gca gaa ggt ttt acc ggt gga ttt aat ggt gta     336
Gly Gly Ala Tyr Met Ala Glu Gly Phe Thr Gly Gly Phe Asn Gly Val
            100                 105                 110

act aag agc caa att aat agt ggt aag gct tta ggg atg aat aga tta     384
Thr Lys Ser Gln Ile Asn Ser Gly Lys Ala Leu Gly Met Asn Arg Leu
        115                 120                 125

caa ctt gct aga tat gtt gtc ttt cca cag ggg gta gca ctc agt gta     432
Gln Leu Ala Arg Tyr Val Val Phe Pro Gln Gly Val Ala Leu Ser Val
    130                 135                 140

cca gca ctt gcc gcc aat ata atc ttt tta att aaa gaa aca tca atc     480
Pro Ala Leu Ala Ala Asn Ile Ile Phe Leu Ile Lys Glu Thr Ser Ile
145                 150                 155                 160

ttt tct gtc att gca att cca gaa ttg act aac aca gcg atg gac ttg     528

Phe Ser Val Ile Ala Ile Pro Glu Leu Thr Asn Thr Ala Met Asp Leu
                165                 170                 175

atc gga atg tat tat cgt tca aac gaa tac tta ttt atg ctg gta gtt     576
Ile Gly Met Tyr Tyr Arg Ser Asn Glu Tyr Leu Phe Met Leu Val Val
            180                 185                 190

gca tat gca gta att ttg att cca tta att tta att tta aat tgg tta     624
Ala Tyr Ala Val Ile Leu Ile Pro Leu Ile Leu Ile Leu Asn Trp Leu
        195                 200                 205

gaa aag aga gtt cgt tat ggt gca ttc ggg aat                         657
Glu Lys Arg Val Arg Tyr Gly Ala Phe Gly Asn
    210                 215


<210> 6
<211> 219
<212> PRT
<213> Lactobacillus acidophilus

<400> 6
Met Asn Trp Thr Ile Ile Gln Gln Ala Met Pro Ala Phe Met Ala Gly
 1               5                  10                  15
Phe Lys Leu Thr Leu Trp Leu Ser Leu Val Gly Ile Ile Gly Ser Ile
            20                  25                  30
Ile Val Gly Ile Ile Val Ser Leu Phe Gln Tyr Phe Lys Val Pro Val
        35                  40                  45
Leu Ser Gln Ile Ser Thr Ala Tyr Val Glu Leu Ala Arg Asn Thr Pro
    50                  55                  60
Leu Leu Ile Gln Leu Phe Phe Leu Tyr Tyr Ala Phe Pro Ile Phe Gly
65                  70                  75                  80
Leu Lys Met Ser Ala Glu Val Cys Gly Ile Ile Gly Leu Ile Phe Leu
                85                  90                  95
Gly Gly Ala Tyr Met Ala Glu Gly Phe Thr Gly Gly Phe Asn Gly Val
            100                 105                 110
Thr Lys Ser Gln Ile Asn Ser Gly Lys Ala Leu Gly Met Asn Arg Leu
        115                 120                 125
Gln Leu Ala Arg Tyr Val Val Phe Pro Gln Gly Val Ala Leu Ser Val
    130                 135                 140
Pro Ala Leu Ala Ala Asn Ile Ile Phe Leu Ile Lys Glu Thr Ser Ile
145                 150                 155                 160
Phe Ser Val Ile Ala Ile Pro Glu Leu Thr Asn Thr Ala Met Asp Leu
                165                 170                 175
Ile Gly Met Tyr Tyr Arg Ser Asn Glu Tyr Leu Phe Met Leu Val Val
            180                 185                 190
Ala Tyr Ala Val Ile Leu Ile Pro Leu Ile Leu Ile Leu Asn Trp Leu
        195                 200                 205
Glu Lys Arg Val Arg Tyr Gly Ala Phe Gly Asn
    210                 215


<210> 7
<211> 672
<212> DNA
<213> Lactobacillus acidophilus

<220>
<221> CDS
<222> (1)...(672)
<223> ABC transporter ORF LBA1044

<400> 7
atg gtg cat tcg gga att aat gtt tta ttt gaa gga aca aat cta gca     48

Met Val His Ser Gly Ile Asn Val Leu Phe Glu Gly Thr Asn Leu Ala
1               5                   10                  15

cgc tta atg gct gga tta tgg gca tct att tgg att gca gca att tca     96
Arg Leu Met Ala Gly Leu Trp Ala Ser Ile Trp Ile Ala Ala Ile Ser
            20                  25                  30

tta gtt att ggt tta ctt tta ggt aca att cta gga atc tta cgt act     144
Leu Val Ile Gly Leu Leu Leu Gly Thr Ile Leu Gly Ile Leu Arg Thr
        35                  40                  45

atg cct aat aaa att gtt cgt ttt gtt ttg aga cta tat ctt gaa ttt     192
Met Pro Asn Lys Ile Val Arg Phe Val Leu Arg Leu Tyr Leu Glu Phe
    50                  55                  60

ttt aga ata gtg cca aca att gta ctt tta tat ttg gtt tat tat att     240
Phe Arg Ile Val Pro Thr Ile Val Leu Leu Tyr Leu Val Tyr Tyr Ile
65                  70                  75                  80

ttg cca aga aca ttc cat gtt aat tgg tcg gca act tgg atg gca gtt     288
Leu Pro Arg Thr Phe His Val Asn Trp Ser Ala Thr Trp Met Ala Val
                85                  90                  95

ttg gcc ttt gca tta tgg gta gct gct gaa ttt agt gat att gta cgt     336
Leu Ala Phe Ala Leu Trp Val Ala Ala Glu Phe Ser Asp Ile Val Arg
            100                 105                 110

gga gca cta gaa tca gtg cca aag aac cag cga gaa tca ggt ttg gca     384
Gly Ala Leu Glu Ser Val Pro Lys Asn Gln Arg Glu Ser Gly Leu Ala
        115                 120                 125

ctg ggt tta agt aat att caa ctc ttt aga tat gtt tta tta cca caa     432
Leu Gly Leu Ser Asn Ile Gln Leu Phe Arg Tyr Val Leu Leu Pro Gln
    130                 135                 140

gca atc aaa tta gag ctt cct gca aca att aat ctt gca aca cgt gtg     480
Ala Ile Lys Leu Glu Leu Pro Ala Thr Ile Asn Leu Ala Thr Arg Val
145                 150                 155                 160

att aaa aca act tca ctt ttg atg gtg atc aat att atg gaa gta atc     528
Ile Lys Thr Thr Ser Leu Leu Met Val Ile Asn Ile Met Glu Val Ile
                165                 170                 175

aat gta gga caa caa att att gaa gct aat aat caa aaa tat cca aca     576
Asn Val Gly Gln Gln Ile Ile Glu Ala Asn Asn Gln Lys Tyr Pro Thr
            180                 185                 190

ggt gta ttt tgg att tat ggt ttt att ttc att tta tat ttc att tta     624
Gly Val Phe Trp Ile Tyr Gly Phe Ile Phe Ile Leu Tyr Phe Ile Leu
        195                 200                 205

gat tat cca tta tca gca tgg gca aag cgg tta aca gca aat aaa gag     672
Asp Tyr Pro Leu Ser Ala Trp Ala Lys Arg Leu Thr Ala Asn Lys Glu
    210                 215                 220

<210> 8
<211> 224
<212> PRT
<213> Lactobacillus acidophilus

<400> 8
Met Val His Ser Gly Ile Asn Val Leu Phe Glu Gly Thr Asn Leu Ala

1                   5                   10                  15
Arg Leu Met Ala Gly Leu Trp Ala Ser Ile Trp Ile Ala Ala Ile Ser
            20                  25                  30
Leu Val Ile Gly Leu Leu Leu Gly Thr Ile Leu Gly Ile Leu Arg Thr
        35                  40                  45
Met Pro Asn Lys Ile Val Arg Phe Val Leu Arg Leu Tyr Leu Glu Phe
    50                  55                  60
Phe Arg Ile Val Pro Thr Ile Val Leu Leu Tyr Leu Val Tyr Tyr Ile
65                  70                  75                  80
Leu Pro Arg Thr Phe His Val Asn Trp Ser Ala Thr Trp Met Ala Val
                85                  90                  95
Leu Ala Phe Ala Leu Trp Val Ala Ala Glu Phe Ser Asp Ile Val Arg
            100                 105                 110
Gly Ala Leu Glu Ser Val Pro Lys Asn Gln Arg Glu Ser Gly Leu Ala
        115                 120                 125
Leu Gly Leu Ser Asn Ile Gln Leu Phe Arg Tyr Val Leu Leu Pro Gln
    130                 135                 140
Ala Ile Lys Leu Glu Leu Pro Ala Thr Ile Asn Leu Ala Thr Arg Val
145                 150                 155                 160
Ile Lys Thr Thr Ser Leu Leu Met Val Ile Asn Ile Met Glu Val Ile
                165                 170                 175
Asn Val Gly Gln Gln Ile Ile Glu Ala Asn Asn Gln Lys Tyr Pro Thr
            180                 185                 190
Gly Val Phe Trp Ile Tyr Gly Phe Ile Phe Ile Leu Tyr Phe Ile Leu
        195                 200                 205
Asp Tyr Pro Leu Ser Ala Trp Ala Lys Arg Leu Thr Ala Asn Lys Glu
    210                 215                 220

<210> 9
<211> 738
<212> DNA
<213> Lactobacillus acidophilus

<220>
<221> CDS
<222> (1)...(738)
<223> ABC transporter ATP-binding protein ORF LBA1045

<400> 9
atg aca gaa gaa att tta aag gta gaa cat tta gat aaa ttt tac ggt     48
Met Thr Glu Glu Ile Leu Lys Val Glu His Leu Asp Lys Phe Tyr Gly
 1               5                  10                  15

gat tgg cgg gct tta cat gat att aac ttc aat ctt aaa aag ggt gaa     96
Asp Trp Arg Ala Leu His Asp Ile Asn Phe Asn Leu Lys Lys Gly Glu
            20                  25                  30

gtt tta acc ctt ctc ggc cca tca ggc tcg ggc aag agt acg ctt tta    144
Val Leu Thr Leu Leu Gly Pro Ser Gly Ser Gly Lys Ser Thr Leu Leu
        35                  40                  45

cgt act tta aat ggc tta gaa gat tat caa aag ggt agt att tat ttt    192
Arg Thr Leu Asn Gly Leu Glu Asp Tyr Gln Lys Gly Ser Ile Tyr Phe
    50                  55                  60

cat gat aaa aaa att gat cct act cca aaa gaa tgg caa tta tta aga    240
His Asp Lys Lys Ile Asp Pro Thr Pro Lys Glu Trp Gln Leu Leu Arg
65                  70                  75                  80

caa aag att ggt atg gtt ttc cag agc tat gat cta ttt cct aac tta    288
Gln Lys Ile Gly Met Val Phe Gln Ser Tyr Asp Leu Phe Pro Asn Leu
                85                  90                  95

acc gta atg gac aat att tta ctt gct cca gta aaa gtg caa cat cga     336
Thr Val Met Asp Asn Ile Leu Leu Ala Pro Val Lys Val Gln His Arg
            100                 105                 110

aaa gaa gat gaa gtt aga aag caa gct gaa gaa ttg cta aaa cgg gta     384
Lys Glu Asp Glu Val Arg Lys Gln Ala Glu Glu Leu Leu Lys Arg Val
        115                 120                 125

ggg atg caa gat tat gca tca tca tat cca aga gaa tta tcc ggt ggt     432
Gly Met Gln Asp Tyr Ala Ser Ser Tyr Pro Arg Glu Leu Ser Gly Gly
    130                 135                 140

caa aaa caa aga gta gca att gtc cgt gca atg gct atg aat ccc gaa     480
Gln Lys Gln Arg Val Ala Ile Val Arg Ala Met Ala Met Asn Pro Glu
145                 150                 155                 160

att ttg cta tta gac gaa att act gca tct ctt gat cca gaa atg gtt     528
Ile Leu Leu Leu Asp Glu Ile Thr Ala Ser Leu Asp Pro Glu Met Val
                165                 170                 175

cgc ggt gtt caa gaa att gta gag cat tta tct aat aga gat cat atg     576
Arg Gly Val Gln Glu Ile Val Glu His Leu Ser Asn Arg Asp His Met
            180                 185                 190

acc atg att gtc gta act cac cag atg aat ttc gct gaa aag att gca     624
Thr Met Ile Val Val Thr His Gln Met Asn Phe Ala Glu Lys Ile Ala
        195                 200                 205

gat gaa gta cta ttc ctt gaa aat ggt aaa atc ttg gaa gat aca cca     672
Asp Glu Val Leu Phe Leu Glu Asn Gly Lys Ile Leu Glu Asp Thr Pro
    210                 215                 220

gga aaa gag ttc ttt aag aac ccc caa act gct cgt gct cgt gaa ttc     720
Gly Lys Glu Phe Phe Lys Asn Pro Gln Thr Ala Arg Ala Arg Glu Phe
225                 230                 235                 240

cta gac agc atg gat ttc                                             738
Leu Asp Ser Met Asp Phe
                245


<210> 10
<211> 246
<212> PRT
<213> Lactobacillus acidophilus

<400> 10
Met Thr Glu Glu Ile Leu Lys Val Glu His Leu Asp Lys Phe Tyr Gly
1               5                   10                  15
Asp Trp Arg Ala Leu His Asp Ile Asn Phe Asn Leu Lys Lys Gly Glu
            20                  25                  30
Val Leu Thr Leu Leu Gly Pro Ser Gly Ser Gly Lys Ser Thr Leu Leu
        35                  40                  45
Arg Thr Leu Asn Gly Leu Glu Asp Tyr Gln Lys Gly Ser Ile Tyr Phe
    50                  55                  60
His Asp Lys Lys Ile Asp Pro Thr Pro Lys Glu Trp Gln Leu Leu Arg
65                  70                  75                  80
Gln Lys Ile Gly Met Val Phe Gln Ser Tyr Asp Leu Phe Pro Asn Leu
                85                  90                  95
Thr Val Met Asp Asn Ile Leu Leu Ala Pro Val Lys Val Gln His Arg
            100                 105                 110
Lys Glu Asp Glu Val Arg Lys Gln Ala Glu Glu Leu Leu Lys Arg Val
        115                 120                 125
Gly Met Gln Asp Tyr Ala Ser Ser Tyr Pro Arg Glu Leu Ser Gly Gly

130                 135                 140
Gln Lys Gln Arg Val Ala Ile Val Arg Ala Met Ala Met Asn Pro Glu
145                 150                 155                 160
Ile Leu Leu Leu Asp Glu Ile Thr Ala Ser Leu Asp Pro Glu Met Val
                165                 170                 175
Arg Gly Val Gln Glu Ile Val Glu His Leu Ser Asn Arg Asp His Met
            180                 185                 190
Thr Met Ile Val Val Thr His Gln Met Asn Phe Ala Glu Lys Ile Ala
        195                 200                 205
Asp Glu Val Leu Phe Leu Glu Asn Gly Lys Ile Leu Glu Asp Thr Pro
    210                 215                 220
Gly Lys Glu Phe Phe Lys Asn Pro Gln Thr Ala Arg Ala Arg Glu Phe
225                 230                 235                 240
Leu Asp Ser Met Asp Phe
                245

<210> 11
<211> 858
<212> DNA
<213> Lactobacillus acidophilus

<220>
<221> CDS
<222> (1)...(858)
<223> ABC transporter substrate-binding protein ORF
      LBA1046

<400> 11
atg aaa gtt aag aac att ttc aat tgg act atc agc ata cta gct att    48
Met Lys Val Lys Asn Ile Phe Asn Trp Thr Ile Ser Ile Leu Ala Ile
 1               5                  10                  15

tta ctc gtg att atc tct gct tat ttt ggt ttt gcg caa aaa ggt tta    96
Leu Leu Val Ile Ile Ser Ala Tyr Phe Gly Phe Ala Gln Lys Gly Leu
            20                  25                  30

aat agt ggt act tct gct agc cga tgg agt aat caa aac gga gtt tct    144
Asn Ser Gly Thr Ser Ala Ser Arg Trp Ser Asn Gln Asn Gly Val Ser
        35                  40                  45

gaa att aaa aga cgc ggc tac tta aga gta gcg gtt ttt ggt gat tta    192
Glu Ile Lys Arg Arg Gly Tyr Leu Arg Val Ala Val Phe Gly Asp Leu
    50                  55                  60

cca cct tat ggc tgg gtt aat tca aat ggt aag cgt gta ggt tat gat    240
Pro Pro Tyr Gly Trp Val Asn Ser Asn Gly Lys Arg Val Gly Tyr Asp
65                  70                  75                  80

gtt tac ttg gca cac cgt atg acc cag gat tta ggg gtg aaa att aga    288
Val Tyr Leu Ala His Arg Met Thr Gln Asp Leu Gly Val Lys Ile Arg
                85                  90                  95

ttt gtt caa gtt aat gct aac aat cgt gtt gac act ttg aat tca aat    336
Phe Val Gln Val Asn Ala Asn Asn Arg Val Asp Thr Leu Asn Ser Asn
            100                 105                 110

aaa gct gat atc att ttg gct aac ttt aca gtt aca cct gaa cga aaa    384
Lys Ala Asp Ile Ile Leu Ala Asn Phe Thr Val Thr Pro Glu Arg Lys
        115                 120                 125

gat gta gta gac ttt gct aaa cca tat atg aag gtt tct gtt ggt gtc    432
Asp Val Val Asp Phe Ala Lys Pro Tyr Met Lys Val Ser Val Gly Val
    130                 135                 140

att tcg cct aag agt gca cca ata act tct gct aat cag tta aag aat     480
Ile Ser Pro Lys Ser Ala Pro Ile Thr Ser Ala Asn Gln Leu Lys Asn
145                 150                 155                 160

aaa aaa tta att gtt act aag ggt acg act gct gaa aac tac ttc tca     528
Lys Lys Leu Ile Val Thr Lys Gly Thr Thr Ala Glu Asn Tyr Phe Ser
                165                 170                 175

caa caa aag aat gta aac tta tta aaa ttc gat tct aaa acg cag caa     576
Gln Gln Lys Asn Val Asn Leu Leu Lys Phe Asp Ser Lys Thr Gln Gln
            180                 185                 190

ttc aac gct ctt aag aat ggt cgt ggt gca gct tta gct gat gat aac     624
Phe Asn Ala Leu Lys Asn Gly Arg Gly Ala Ala Leu Ala Asp Asp Asn
        195                 200                 205

tca tac ctt tac gct tgg gta aaa caa aat cct aaa tat act gta gga     672
Ser Tyr Leu Tyr Ala Trp Val Lys Gln Asn Pro Lys Tyr Thr Val Gly
    210                 215                 220

att aag agt att ggt ccc aaa caa tat att tct cca gca gtt aaa aaa     720
Ile Lys Ser Ile Gly Pro Lys Gln Tyr Ile Ser Pro Ala Val Lys Lys
225                 230                 235                 240

ggg aat cgt tca cta tta aaa tgg aca aat aaa gag att act aaa ttg     768
Gly Asn Arg Ser Leu Leu Lys Trp Thr Asn Lys Glu Ile Thr Lys Leu
                245                 250                 255

aat aag gaa agc ttt ttc gta gaa gat tac aat aaa gaa tta cgt cca     816
Asn Lys Glu Ser Phe Phe Val Glu Asp Tyr Asn Lys Glu Leu Arg Pro
            260                 265                 270

tac ttt ggt aag gat atc aag cca act gat att att ttg gct             858
Tyr Phe Gly Lys Asp Ile Lys Pro Thr Asp Ile Ile Leu Ala
        275                 280                 285

<210> 12
<211> 286
<212> PRT
<213> Lactobacillus acidophilus

<400> 12
Met Lys Val Lys Asn Ile Phe Asn Trp Thr Ile Ser Ile Leu Ala Ile
1               5                   10                  15
Leu Leu Val Ile Ile Ser Ala Tyr Phe Gly Phe Ala Gln Lys Gly Leu
            20                  25                  30
Asn Ser Gly Thr Ser Ala Ser Arg Trp Ser Asn Gln Asn Gly Val Ser
        35                  40                  45
Glu Ile Lys Arg Arg Gly Tyr Leu Arg Val Ala Val Phe Gly Asp Leu
    50                  55                  60
Pro Pro Tyr Gly Trp Val Asn Ser Asn Gly Lys Arg Val Gly Tyr Asp
65                  70                  75                  80
Val Tyr Leu Ala His Arg Met Thr Gln Asp Leu Gly Val Lys Ile Arg
                85                  90                  95
Phe Val Gln Val Asn Ala Asn Asn Arg Val Asp Thr Leu Asn Ser Asn
            100                 105                 110
Lys Ala Asp Ile Ile Leu Ala Asn Phe Thr Val Thr Pro Glu Arg Lys
        115                 120                 125
Asp Val Val Asp Phe Ala Lys Pro Tyr Met Lys Val Ser Val Gly Val
    130                 135                 140
Ile Ser Pro Lys Ser Ala Pro Ile Thr Ser Ala Asn Gln Leu Lys Asn

145                 150                 155                 160
Lys Lys Leu Ile Val Thr Lys Gly Thr Thr Ala Glu Asn Tyr Phe Ser
                165                 170                 175
Gln Gln Lys Asn Val Asn Leu Leu Lys Phe Asp Ser Lys Thr Gln Gln
            180                 185                 190
Phe Asn Ala Leu Lys Asn Gly Arg Gly Ala Ala Leu Ala Asp Asp Asn
        195                 200                 205
Ser Tyr Leu Tyr Ala Trp Val Lys Gln Asn Pro Lys Tyr Thr Val Gly
    210                 215                 220
Ile Lys Ser Ile Gly Pro Lys Gln Tyr Ile Ser Pro Ala Val Lys Lys
225                 230                 235                 240
Gly Asn Arg Ser Leu Leu Lys Trp Thr Asn Lys Glu Ile Thr Lys Leu
                245                 250                 255
Asn Lys Glu Ser Phe Phe Val Glu Asp Tyr Asn Lys Glu Leu Arg Pro
            260                 265                 270
Tyr Phe Gly Lys Asp Ile Lys Pro Thr Asp Ile Ile Leu Ala
        275                 280                 285


<210> 13
<211> 1116
<212> DNA
<213> Lactobacillus acidophilus

<220>
<221> CDS
<222> (1)...(1116)
<223> metE ORF LBA1080

<400> 13
atg agt aaa act tta gta cat tat gac att gtt ggt agt ttc tta aga    48
Met Ser Lys Thr Leu Val His Tyr Asp Ile Val Gly Ser Phe Leu Arg
 1               5                  10                  15

cca gaa gaa ttg aaa aag gca cgt gct gat ttt gca gcg ggt aac att    96
Pro Glu Glu Leu Lys Lys Ala Arg Ala Asp Phe Ala Ala Gly Asn Ile
            20                  25                  30

tca aaa act gac ttg aaa aag gtt gaa gat gaa gaa atc gct aaa tta    144
Ser Lys Thr Asp Leu Lys Lys Val Glu Asp Glu Glu Ile Ala Lys Leu
        35                  40                  45

gtt aaa aaa gaa gaa aaa gct ggt tta aag att gta act gat ggt gaa    192
Val Lys Lys Glu Glu Lys Ala Gly Leu Lys Ile Val Thr Asp Gly Glu
    50                  55                  60

ttc aga aga agt tat tgg cac ctt gat act ttt tgg ggc ttc ggt gga    240
Phe Arg Arg Ser Tyr Trp His Leu Asp Thr Phe Trp Gly Phe Gly Gly
65                  70                  75                  80

att aag cac act act caa gaa cat ggc tac ttc ttc cac gat gaa gaa    288
Ile Lys His Thr Thr Gln Glu His Gly Tyr Phe Phe His Asp Glu Glu
                85                  90                  95

act cgt aat gat tct gct caa gtt gag gga aag att aaa ttt aca ggt    336
Thr Arg Asn Asp Ser Ala Gln Val Glu Gly Lys Ile Lys Phe Thr Gly
            100                 105                 110

gat cat cca gat tta gaa gca ttt aag ttt ttg aag agt tta acc gat    384
Asp His Pro Asp Leu Glu Ala Phe Lys Phe Leu Lys Ser Leu Thr Asp
        115                 120                 125

ggc agt gat gta act cca cgt caa agc att cct tca cca gcc caa ttt    432
Gly Ser Asp Val Thr Pro Arg Gln Ser Ile Pro Ser Pro Ala Gln Phe

130                 135                 140

tac gca gaa ctc gtc cgt ggc cca gaa aat gtt gca gca gtg aag aaa     480
Tyr Ala Glu Leu Val Arg Gly Pro Glu Asn Val Ala Ala Val Lys Lys
145                 150                 155                 160

gtt tac gat acc gaa gac gaa ctt tta aac gat att tca aaa gca tat     528
Val Tyr Asp Thr Glu Asp Glu Leu Leu Asn Asp Ile Ser Lys Ala Tyr
                165                 170                 175

tat gat tta atc atc gca ctt tac aaa gca ggt tgt cgc gat gtg aaa     576
Tyr Asp Leu Ile Ile Ala Leu Tyr Lys Ala Gly Cys Arg Asp Val Lys
            180                 185                 190

ttg gat gac tgt act tgg gga atg gtc gta gat gat gat ttc tgg gca     624
Leu Asp Asp Cys Thr Trp Gly Met Val Val Asp Asp Asp Phe Trp Ala
        195                 200                 205

aca atg gtt aaa caa ggt ttt gac cgt gac gaa ctt caa gaa aaa tac     672
Thr Met Val Lys Gln Gly Phe Asp Arg Asp Glu Leu Gln Glu Lys Tyr
    210                 215                 220

ctt cgt gtt aac aat ggc gca ctt aaa gat cta cca gct gat tta aga     720
Leu Arg Val Asn Asn Gly Ala Leu Lys Asp Leu Pro Ala Asp Leu Arg
225                 230                 235                 240

act tca act cat att tgt cga ggc aat tac cac tca act tgg gct gct     768
Thr Ser Thr His Ile Cys Arg Gly Asn Tyr His Ser Thr Trp Ala Ala
                245                 250                 255

aaa ggt ggt tat gga ccg gtt gcc aaa tac gtt ttt gca caa gaa aat     816
Lys Gly Gly Tyr Gly Pro Val Ala Lys Tyr Val Phe Ala Gln Glu Asn
            260                 265                 270

gtc gat gca ttc tat ctt gaa ttt gat aat gaa aga tca ggt aac ttc     864
Val Asp Ala Phe Tyr Leu Glu Phe Asp Asn Glu Arg Ser Gly Asn Phe
        275                 280                 285

gat cca atc aag gaa att cct gct gat aaa gaa gta gta ctt ggt tta     912
Asp Pro Ile Lys Glu Ile Pro Ala Asp Lys Glu Val Val Leu Gly Leu
    290                 295                 300

gta act agt aag aaa cct gaa ttg gaa aag cca gag gat ttg att gct     960
Val Thr Ser Lys Lys Pro Glu Leu Glu Lys Pro Glu Asp Leu Ile Ala
305                 310                 315                 320

cgt atc aat gaa gca agt aag ttc cac gat tta gcc aac tta gcc tta     1008
Arg Ile Asn Glu Ala Ser Lys Phe His Asp Leu Ala Asn Leu Ala Leu
                325                 330                 335

agc act caa tgt ggt ttt gca tca acc gag gaa gga aac caa ttg aca     1056
Ser Thr Gln Cys Gly Phe Ala Ser Thr Glu Glu Gly Asn Gln Leu Thr
            340                 345                 350

gaa gac gat gaa tgg aaa aag att ggc tta gta atc gat act gct aag     1104
Glu Asp Asp Glu Trp Lys Lys Ile Gly Leu Val Ile Asp Thr Ala Lys
        355                 360                 365

caa gtt tgg aag                                                     1116
Gln Val Trp Lys
    370

<210> 14

<211> 372
<212> PRT
<213> Lactobacillus acidophilus

<400> 14
Met Ser Lys Thr Leu Val His Tyr Asp Ile Val Gly Ser Phe Leu Arg
1               5                   10                  15
Pro Glu Glu Leu Lys Lys Ala Arg Ala Asp Phe Ala Ala Gly Asn Ile
            20                  25                  30
Ser Lys Thr Asp Leu Lys Lys Val Glu Asp Glu Glu Ile Ala Lys Leu
        35                  40                  45
Val Lys Lys Glu Glu Lys Ala Gly Leu Lys Ile Val Thr Asp Gly Glu
    50                  55                  60
Phe Arg Arg Ser Tyr Trp His Leu Asp Thr Phe Trp Gly Phe Gly Gly
65                  70                  75                  80
Ile Lys His Thr Thr Gln Glu His Gly Tyr Phe Phe His Asp Glu Glu
                85                  90                  95
Thr Arg Asn Asp Ser Ala Gln Val Glu Gly Lys Ile Lys Phe Thr Gly
            100                 105                 110
Asp His Pro Asp Leu Glu Ala Phe Lys Phe Leu Lys Ser Leu Thr Asp
        115                 120                 125
Gly Ser Asp Val Thr Pro Arg Gln Ser Ile Pro Ser Pro Ala Gln Phe
    130                 135                 140
Tyr Ala Glu Leu Val Arg Gly Pro Glu Asn Val Ala Ala Val Lys Lys
145                 150                 155                 160
Val Tyr Asp Thr Glu Asp Glu Leu Leu Asn Asp Ile Ser Lys Ala Tyr
                165                 170                 175
Tyr Asp Leu Ile Ile Ala Leu Tyr Lys Ala Gly Cys Arg Asp Val Lys
            180                 185                 190
Leu Asp Asp Cys Thr Trp Gly Met Val Val Asp Asp Asp Phe Trp Ala
        195                 200                 205
Thr Met Val Lys Gln Gly Phe Asp Arg Asp Glu Leu Gln Glu Lys Tyr
    210                 215                 220
Leu Arg Val Asn Asn Gly Ala Leu Lys Asp Leu Pro Ala Asp Leu Arg
225                 230                 235                 240
Thr Ser Thr His Ile Cys Arg Gly Asn Tyr His Ser Thr Trp Ala Ala
                245                 250                 255
Lys Gly Gly Tyr Gly Pro Val Ala Lys Tyr Val Phe Ala Gln Glu Asn
            260                 265                 270
Val Asp Ala Phe Tyr Leu Glu Phe Asp Asn Glu Arg Ser Gly Asn Phe
        275                 280                 285
Asp Pro Ile Lys Glu Ile Pro Ala Asp Lys Glu Val Val Leu Gly Leu
    290                 295                 300
Val Thr Ser Lys Lys Pro Glu Leu Glu Lys Pro Glu Asp Leu Ile Ala
305                 310                 315                 320
Arg Ile Asn Glu Ala Ser Lys Phe His Asp Leu Ala Asn Leu Ala Leu
                325                 330                 335
Ser Thr Gln Cys Gly Phe Ala Ser Thr Glu Glu Gly Asn Gln Leu Thr
            340                 345                 350
Glu Asp Asp Glu Trp Lys Lys Ile Gly Leu Val Ile Asp Thr Ala Lys
        355                 360                 365
Gln Val Trp Lys
    370


<210> 15
<211> 471
<212> DNA
<213> Lactobacillus acidophilus

<220>
<221> CDS
<222> (1)...(471)
<223> luxS ORF LBA1081

<400> 15
atg gca aaa gtt gaa agt ttt aca tta gac cac act aaa gtt aag gca     48
Met Ala Lys Val Glu Ser Phe Thr Leu Asp His Thr Lys Val Lys Ala
1               5                   10                  15

cct tac gtt cgt tta att act gtt gaa gaa ggt cct aaa ggc gac aag     96
Pro Tyr Val Arg Leu Ile Thr Val Glu Glu Gly Pro Lys Gly Asp Lys
            20                  25                  30

att tct aac tat gac tta cgt tta gtt caa ccg aac gaa aat gca att     144
Ile Ser Asn Tyr Asp Leu Arg Leu Val Gln Pro Asn Glu Asn Ala Ile
        35                  40                  45

cct acc ggc gga ttg cat act att gaa cac tta ctt gcc agc tta ctt     192
Pro Thr Gly Gly Leu His Thr Ile Glu His Leu Leu Ala Ser Leu Leu
    50                  55                  60

cgt gac cgt ctt gat ggt gta atc gat tgt tca cca ttt ggt tgc cga     240
Arg Asp Arg Leu Asp Gly Val Ile Asp Cys Ser Pro Phe Gly Cys Arg
65                  70                  75                  80

aca gga ttc cac cta atc gtt tgg ggt gaa cat tca act act gaa gtt     288
Thr Gly Phe His Leu Ile Val Trp Gly Glu His Ser Thr Thr Glu Val
                85                  90                  95

gct aaa gca ttg aag tct tca tta gag gaa att cgt gac aca att act     336
Ala Lys Ala Leu Lys Ser Ser Leu Glu Glu Ile Arg Asp Thr Ile Thr
            100                 105                 110

tgg gaa gat gta cca ggt aca act att aag act tgt ggt aac tac cgt     384
Trp Glu Asp Val Pro Gly Thr Thr Ile Lys Thr Cys Gly Asn Tyr Arg
        115                 120                 125

gat cac tca ttg ttc acc gca aaa gaa tgg tgt cgt gat att ctt gaa     432
Asp His Ser Leu Phe Thr Ala Lys Glu Trp Cys Arg Asp Ile Leu Glu
    130                 135                 140

aaa gga att agt gat gac cca ttc gaa aga aat gtg att                 471
Lys Gly Ile Ser Asp Asp Pro Phe Glu Arg Asn Val Ile
145                 150                 155


<210> 16
<211> 157
<212> PRT
<213> Lactobacillus acidophilus

<400> 16
Met Ala Lys Val Glu Ser Phe Thr Leu Asp His Thr Lys Val Lys Ala
1               5                   10                  15
Pro Tyr Val Arg Leu Ile Thr Val Glu Glu Gly Pro Lys Gly Asp Lys
            20                  25                  30
Ile Ser Asn Tyr Asp Leu Arg Leu Val Gln Pro Asn Glu Asn Ala Ile
        35                  40                  45
Pro Thr Gly Gly Leu His Thr Ile Glu His Leu Leu Ala Ser Leu Leu
    50                  55                  60
Arg Asp Arg Leu Asp Gly Val Ile Asp Cys Ser Pro Phe Gly Cys Arg
65                  70                  75                  80
Thr Gly Phe His Leu Ile Val Trp Gly Glu His Ser Thr Thr Glu Val
                85                  90                  95
Ala Lys Ala Leu Lys Ser Ser Leu Glu Glu Ile Arg Asp Thr Ile Thr
            100                 105                 110
Trp Glu Asp Val Pro Gly Thr Thr Ile Lys Thr Cys Gly Asn Tyr Arg

        115                 120                 125
Asp His Ser Leu Phe Thr Ala Lys Glu Trp Cys Arg Asp Ile Leu Glu
    130                 135                 140
Lys Gly Ile Ser Asp Asp Pro Phe Glu Arg Asn Val Ile
145                 150                 155

<210> 17
<211> 1332
<212> DNA
<213> Lactobacillus acidophilus

<220>
<221> CDS
<222> (1)...(1332)
<223> splA ORF LBA169

<400> 17
atg aag aaa aat tta aga atc gtt agc gct gct gct gct gct tta ctt    48
Met Lys Lys Asn Leu Arg Ile Val Ser Ala Ala Ala Ala Ala Leu Leu
 1               5                  10                  15

gct gtt gct cca gtt gct gct tct gct gta tct act gtt agc gct gct    96
Ala Val Ala Pro Val Ala Ala Ser Ala Val Ser Thr Val Ser Ala Ala
            20                  25                  30

act act att aac gca agt tca tca gca atc aat acc aac act aat gct    144
Thr Thr Ile Asn Ala Ser Ser Ser Ala Ile Asn Thr Asn Thr Asn Ala
        35                  40                  45

aag tac gat gtt gat gta act cct agt gtt tct gca gtt gct gca aat    192
Lys Tyr Asp Val Asp Val Thr Pro Ser Val Ser Ala Val Ala Ala Asn
    50                  55                  60

act gct aac aac act cca gct att gcc ggt aac ctt act ggt act att    240
Thr Ala Asn Asn Thr Pro Ala Ile Ala Gly Asn Leu Thr Gly Thr Ile
65                  70                  75                  80

tca gca agt tac aat ggt aag act tat act gct aac tta aag gca gat    288
Ser Ala Ser Tyr Asn Gly Lys Thr Tyr Thr Ala Asn Leu Lys Ala Asp
                85                  90                  95

act gaa aat gcc act att act gct gct ggt agc act act gcc gtt aaa    336
Thr Glu Asn Ala Thr Ile Thr Ala Ala Gly Ser Thr Thr Ala Val Lys
            100                 105                 110

cct gct gaa tta gct gca ggt gtg gct tac act gta act gtt aac gat    384
Pro Ala Glu Leu Ala Ala Gly Val Ala Tyr Thr Val Thr Val Asn Asp
        115                 120                 125

gtt tca ttt aac ttc ggt tca gaa aat gca ggt aag act gtt acc ctt    432
Val Ser Phe Asn Phe Gly Ser Glu Asn Ala Gly Lys Thr Val Thr Leu
    130                 135                 140

ggt tca gct aac tca aat gta aaa ttc acc ggt aca aac agt gat aat    480
Gly Ser Ala Asn Ser Asn Val Lys Phe Thr Gly Thr Asn Ser Asp Asn
145                 150                 155                 160

caa act gaa act aat gtt tct act ttg aaa gtt aag tta gac caa aac    528
Gln Thr Glu Thr Asn Val Ser Thr Leu Lys Val Lys Leu Asp Gln Asn
                165                 170                 175

ggt gtt gct tca ctt act aat gtt tca att gca aac gta tac gca att    576
Gly Val Ala Ser Leu Thr Asn Val Ser Ile Ala Asn Val Tyr Ala Ile

180                 185                 190

aac act act gat aac agt aac gta aac ttc tac gac gta act agt ggt      624
Asn Thr Thr Asp Asn Ser Asn Val Asn Phe Tyr Asp Val Thr Ser Gly
        195                 200                 205

gct act gta act aac ggt gcc gtt tca gtt aat gct gat aac caa ggt      672
Ala Thr Val Thr Asn Gly Ala Val Ser Val Asn Ala Asp Asn Gln Gly
    210                 215                 220

caa gtt aat gtt gca aac gta gtt gca gca att aat tca aaa tac ttt      720
Gln Val Asn Val Ala Asn Val Val Ala Ala Ile Asn Ser Lys Tyr Phe
225                 230                 235                 240

gca gca caa tac gca gat aag aag tta aat act cgt act gct aat act      768
Ala Ala Gln Tyr Ala Asp Lys Lys Leu Asn Thr Arg Thr Ala Asn Thr
                245                 250                 255

gaa gat gct att aag gca gcc tta aag gac caa aag att gat gta aac      816
Glu Asp Ala Ile Lys Ala Ala Leu Lys Asp Gln Lys Ile Asp Val Asn
            260                 265                 270

tca gta ggt tac ttc aaa gca cct cat act ttc act gtt aac gtt aaa      864
Ser Val Gly Tyr Phe Lys Ala Pro His Thr Phe Thr Val Asn Val Lys
        275                 280                 285

gca act tca aat act aat ggt aag tca gct act ttg cca gta gtt gtt      912
Ala Thr Ser Asn Thr Asn Gly Lys Ser Ala Thr Leu Pro Val Val Val
    290                 295                 300

act gtt cct aat gtt gct gag cca act gta gcc agc gta agc aag aga      960
Thr Val Pro Asn Val Ala Glu Pro Thr Val Ala Ser Val Ser Lys Arg
305                 310                 315                 320

att atg cac aac gca tac tac tac gac aag gac gct aag cgt gtt ggt      1008
Ile Met His Asn Ala Tyr Tyr Tyr Asp Lys Asp Ala Lys Arg Val Gly
                325                 330                 335

act gac agc gtt aag cgt tac aac tca gta agc gta ttg cca aac act      1056
Thr Asp Ser Val Lys Arg Tyr Asn Ser Val Ser Val Leu Pro Asn Thr
            340                 345                 350

act act atc aac ggt aag act tac tac caa gta gtt gaa aac ggt aag      1104
Thr Thr Ile Asn Gly Lys Thr Tyr Tyr Gln Val Val Glu Asn Gly Lys
        355                 360                 365

gct gtt gac aag tac atc aac gct gca aac atc gat ggt act aag cgt      1152
Ala Val Asp Lys Tyr Ile Asn Ala Ala Asn Ile Asp Gly Thr Lys Arg
    370                 375                 380

act ttg aag cac aac gct tac gtt tac gca tca tca aag aag cgt gct      1200
Thr Leu Lys His Asn Ala Tyr Val Tyr Ala Ser Ser Lys Lys Arg Ala
385                 390                 395                 400

aac aag gtt gta ttg aag aag ggt gaa gtt gta act act tac ggt gct      1248
Asn Lys Val Val Leu Lys Lys Gly Glu Val Val Thr Thr Tyr Gly Ala
                405                 410                 415

tca tac aca ttc aag aac ggc caa aag tac tac aag atc ggt gac aac      1296
Ser Tyr Thr Phe Lys Asn Gly Gln Lys Tyr Tyr Lys Ile Gly Asp Asn
            420                 425                 430

act gac aag act tac gtt aag gtt gca aac ttt aga                      1332
Thr Asp Lys Thr Tyr Val Lys Val Ala Asn Phe Arg

435                 440

<210> 18
<211> 444
<212> PRT
<213> Lactobacillus acidophilus

<400> 18
Met Lys Lys Asn Leu Arg Ile Val Ser Ala Ala Ala Ala Ala Leu Leu
1               5                   10                  15
Ala Val Ala Pro Val Ala Ala Ser Ala Val Ser Thr Val Ser Ala Ala
            20                  25                  30
Thr Thr Ile Asn Ala Ser Ser Ser Ala Ile Asn Thr Asn Thr Asn Ala
        35                  40                  45
Lys Tyr Asp Val Asp Val Thr Pro Ser Val Ser Ala Val Ala Ala Asn
    50                  55                  60
Thr Ala Asn Asn Thr Pro Ala Ile Ala Gly Asn Leu Thr Gly Thr Ile
65                  70                  75                  80
Ser Ala Ser Tyr Asn Gly Lys Thr Tyr Thr Ala Asn Leu Lys Ala Asp
                85                  90                  95
Thr Glu Asn Ala Thr Ile Thr Ala Ala Gly Ser Thr Thr Ala Val Lys
            100                 105                 110
Pro Ala Glu Leu Ala Ala Gly Val Ala Tyr Thr Val Thr Val Asn Asp
        115                 120                 125
Val Ser Phe Asn Phe Gly Ser Glu Asn Ala Gly Lys Thr Val Thr Leu
    130                 135                 140
Gly Ser Ala Asn Ser Asn Val Lys Phe Thr Gly Thr Asn Ser Asp Asn
145                 150                 155                 160
Gln Thr Glu Thr Asn Val Ser Thr Leu Lys Val Lys Leu Asp Gln Asn
                165                 170                 175
Gly Val Ala Ser Leu Thr Asn Val Ser Ile Ala Asn Val Tyr Ala Ile
            180                 185                 190
Asn Thr Thr Asp Asn Ser Asn Val Asn Phe Tyr Asp Val Thr Ser Gly
        195                 200                 205
Ala Thr Val Thr Asn Gly Ala Val Ser Val Asn Ala Asp Asn Gln Gly
    210                 215                 220
Gln Val Asn Val Ala Asn Val Val Ala Ala Ile Asn Ser Lys Tyr Phe
225                 230                 235                 240
Ala Ala Gln Tyr Ala Asp Lys Lys Leu Asn Thr Arg Thr Ala Asn Thr
                245                 250                 255
Glu Asp Ala Ile Lys Ala Ala Leu Lys Asp Gln Lys Ile Asp Val Asn
            260                 265                 270
Ser Val Gly Tyr Phe Lys Ala Pro His Thr Phe Thr Val Asn Val Lys
        275                 280                 285
Ala Thr Ser Asn Thr Asn Gly Lys Ser Ala Thr Leu Pro Val Val Val
    290                 295                 300
Thr Val Pro Asn Val Ala Glu Pro Thr Val Ala Ser Val Ser Lys Arg
305                 310                 315                 320
Ile Met His Asn Ala Tyr Tyr Tyr Asp Lys Asp Ala Lys Arg Val Gly
                325                 330                 335
Thr Asp Ser Val Lys Arg Tyr Asn Ser Val Ser Val Leu Pro Asn Thr
            340                 345                 350
Thr Thr Ile Asn Gly Lys Thr Tyr Tyr Gln Val Val Glu Asn Gly Lys
        355                 360                 365
Ala Val Asp Lys Tyr Ile Asn Ala Ala Asn Ile Asp Gly Thr Lys Arg
    370                 375                 380
Thr Leu Lys His Asn Ala Tyr Val Tyr Ala Ser Ser Lys Lys Arg Ala
385                 390                 395                 400
Asn Lys Val Val Leu Lys Lys Gly Glu Val Val Thr Thr Tyr Gly Ala
                405                 410                 415
Ser Tyr Thr Phe Lys Asn Gly Gln Lys Tyr Tyr Lys Ile Gly Asp Asn
            420                 425                 430
Thr Asp Lys Thr Tyr Val Lys Val Ala Asn Phe Arg

435                440

<210> 19
<211> 1689
<212> DNA
<213> Lactobacillus acidophilus

<220>
<221> CDS
<222> (1)...(1689)
<223> fbpA ORF LBA1148

<400> 19
atg gca ttt gac gga tta ttt atc cat agt tta cta caa gat ttg acc     48
Met Ala Phe Asp Gly Leu Phe Ile His Ser Leu Leu Gln Asp Leu Thr
 1               5                  10                  15

cct aca tta gta gat ggt cga tta tca aaa att tac caa cca ttt gaa     96
Pro Thr Leu Val Asp Gly Arg Leu Ser Lys Ile Tyr Gln Pro Phe Glu
            20                  25                  30

caa gat cta ata tta act ttt aga aaa aat aga aag aat tat caa tta    144
Gln Asp Leu Ile Leu Thr Phe Arg Lys Asn Arg Lys Asn Tyr Gln Leu
        35                  40                  45

tta att tca gcc aac gct caa tat cct aga atg tat tta act gag cag    192
Leu Ile Ser Ala Asn Ala Gln Tyr Pro Arg Met Tyr Leu Thr Glu Gln
    50                  55                  60

act att gct aat cca gac aaa gca cct att ttt gtt atg gtt tta aga    240
Thr Ile Ala Asn Pro Asp Lys Ala Pro Ile Phe Val Met Val Leu Arg
65                  70                  75                  80

aag tat ttg gaa ggt tca gtc tta caa tct att gaa caa att ggt gtg    288
Lys Tyr Leu Glu Gly Ser Val Leu Gln Ser Ile Glu Gln Ile Gly Val
                85                  90                  95

aac cga att ata aac ctt cat ttc agt aat cga aac gaa tta ggt gat    336
Asn Arg Ile Ile Asn Leu His Phe Ser Asn Arg Asn Glu Leu Gly Asp
            100                 105                 110

caa gta aaa tta gta tta tcc gtt gaa tta atg gga cga cat agt aat    384
Gln Val Lys Leu Val Leu Ser Val Glu Leu Met Gly Arg His Ser Asn
        115                 120                 125

gta att ctc tat gat caa cag aat ggt cat att att gat cta ttg aag    432
Val Ile Leu Tyr Asp Gln Gln Asn Gly His Ile Ile Asp Leu Leu Lys
    130                 135                 140

cga ata aat cct gat gaa aat aga gct cgt att tta tta cct aag gca    480
Arg Ile Asn Pro Asp Glu Asn Arg Ala Arg Ile Leu Leu Pro Lys Ala
145                 150                 155                 160

aaa tat gag ctt ccc cct ctt aaa cct ggt ata aat ggt tta gta gta    528
Lys Tyr Glu Leu Pro Pro Leu Lys Pro Gly Ile Asn Gly Leu Val Val
                165                 170                 175

act gaa aat caa ttt aaa aag tta agc aat gaa aaa agc att cca gat    576
Thr Glu Asn Gln Phe Lys Lys Leu Ser Asn Glu Lys Ser Ile Pro Asp
            180                 185                 190

tta gtg aaa tca atg gat ggt tta gac aga gac gat cgt gaa gaa tta    624
Leu Val Lys Ser Met Asp Gly Leu Asp Arg Asp Asp Arg Glu Glu Leu

195                 200                 205

act ggt tat ctt gaa gat gat ttt agc tat tct tca ttt aag act ttc     672
Thr Gly Tyr Leu Glu Asp Asp Phe Ser Tyr Ser Ser Phe Lys Thr Phe
    210                 215                 220

ttt gat cag ttt aat aat cca aga gct ttc gtt tta aaa act cca aga     720
Phe Asp Gln Phe Asn Asn Pro Arg Ala Phe Val Leu Lys Thr Pro Arg
225                 230                 235                 240

aat aaa cgt aag att ttc tgt tat ctt ccc tat cat tta gat tta gag     768
Asn Lys Arg Lys Ile Phe Cys Tyr Leu Pro Tyr His Leu Asp Leu Glu
                245                 250                 255

aaa gaa aat tct aat cct gat tta aat aaa ggt tta gat gaa ttt tat     816
Lys Glu Asn Ser Asn Pro Asp Leu Asn Lys Gly Leu Asp Glu Phe Tyr
            260                 265                 270

gaa tac caa gca aca cgt gat tgg gtt aaa caa cgt gct agt cag gtt     864
Glu Tyr Gln Ala Thr Arg Asp Trp Val Lys Gln Arg Ala Ser Gln Val
        275                 280                 285

gaa cgt gtc gtc aaa aac gaa cag aac aaa cta agt aag aag att aag     912
Glu Arg Val Val Lys Asn Glu Gln Asn Lys Leu Ser Lys Lys Ile Lys
    290                 295                 300

aaa tta gaa aag caa tta aac tta gca gaa aat tct gag ggc tat cgt     960
Lys Leu Glu Lys Gln Leu Asn Leu Ala Glu Asn Ser Glu Gly Tyr Arg
305                 310                 315                 320

atc aag ggt gaa ata tta aat gct aat tta gga caa gta aaa cca gga     1008
Ile Lys Gly Glu Ile Leu Asn Ala Asn Leu Gly Gln Val Lys Pro Gly
                325                 330                 335

atg act gag gtt tcc tta cct aac tat tat gaa aat aat aaa cct ata     1056
Met Thr Glu Val Ser Leu Pro Asn Tyr Tyr Glu Asn Asn Lys Pro Ile
            340                 345                 350

agc att aaa ctt gat cca gct tta tct ccg gct cga aat gca caa aaa     1104
Ser Ile Lys Leu Asp Pro Ala Leu Ser Pro Ala Arg Asn Ala Gln Lys
        355                 360                 365

tat ttc act cgc tat aaa aaa tta cgt gat tct atc aaa cac gtt aac     1152
Tyr Phe Thr Arg Tyr Lys Lys Leu Arg Asp Ser Ile Lys His Val Asn
    370                 375                 380

gaa caa att aaa atc gct aaa gaa aat ctt cgc tat ttt gat tct atc     1200
Glu Gln Ile Lys Ile Ala Lys Glu Asn Leu Arg Tyr Phe Asp Ser Ile
385                 390                 395                 400

caa aca gct att gat aat gct gat cca caa gat att gat caa ata act     1248
Gln Thr Ala Ile Asp Asn Ala Asp Pro Gln Asp Ile Asp Gln Ile Thr
                405                 410                 415

gac gaa ttg att aat caa ggt tat att aga aag caa caa aaa aat aga     1296
Asp Glu Leu Ile Asn Gln Gly Tyr Ile Arg Lys Gln Gln Lys Asn Arg
            420                 425                 430

cgt aag aag aaa att act gaa cgc aat cta aat gaa ttc cag ctt tct     1344
Arg Lys Lys Lys Ile Thr Glu Arg Asn Leu Asn Glu Phe Gln Leu Ser
        435                 440                 445

tct gga aaa cat gtc tta gtg ggt aaa aat aat tac caa aat gac tgg     1392
Ser Gly Lys His Val Leu Val Gly Lys Asn Asn Tyr Gln Asn Asp Trp

450                 455                 460

ctt act cta aaa aaa gct aat aaa tta gat tat tgg ttc cac gtt aaa      1440
Leu Thr Leu Lys Lys Ala Asn Lys Leu Asp Tyr Trp Phe His Val Lys
465                 470                 475                 480

aat atg cct ggt tca cat gtt att ttg cga gat gat caa cct acc gat      1488
Asn Met Pro Gly Ser His Val Ile Leu Arg Asp Asp Gln Pro Thr Asp
                485                 490                 495

caa gat att aaa gaa gct gca gag att gct gca ttt ttc tct aaa gcc      1536
Gln Asp Ile Lys Glu Ala Ala Glu Ile Ala Ala Phe Phe Ser Lys Ala
            500                 505                 510

aaa aat tct gcc cac gtt caa gtt gac tac gtt caa gat aag cgt gta      1584
Lys Asn Ser Ala His Val Gln Val Asp Tyr Val Gln Asp Lys Arg Val
        515                 520                 525

aag aag cct aat ggg gct aag cca gga ttc gta att tac aca gga cag      1632
Lys Lys Pro Asn Gly Ala Lys Pro Gly Phe Val Ile Tyr Thr Gly Gln
    530                 535                 540

aat tca atc gaa gtt aca cct aaa gaa gac gaa att atg gct aaa aaa      1680
Asn Ser Ile Glu Val Thr Pro Lys Glu Asp Glu Ile Met Ala Lys Lys
545                 550                 555                 560

atc aat aaa                                                          1689
Ile Asn Lys

<210> 20
<211> 563
<212> PRT
<213> Lactobacillus acidophilus

<400> 20
Met Ala Phe Asp Gly Leu Phe Ile His Ser Leu Leu Gln Asp Leu Thr
1               5                   10                  15
Pro Thr Leu Val Asp Gly Arg Leu Ser Lys Ile Tyr Gln Pro Phe Glu
            20                  25                  30
Gln Asp Leu Ile Leu Thr Phe Arg Lys Asn Arg Lys Asn Tyr Gln Leu
        35                  40                  45
Leu Ile Ser Ala Asn Ala Gln Tyr Pro Arg Met Tyr Leu Thr Glu Gln
    50                  55                  60
Thr Ile Ala Asn Pro Asp Lys Ala Pro Ile Phe Val Met Val Leu Arg
65                  70                  75                  80
Lys Tyr Leu Glu Gly Ser Val Leu Gln Ser Ile Glu Gln Ile Gly Val
                85                  90                  95
Asn Arg Ile Ile Asn Leu His Phe Ser Asn Arg Asn Glu Leu Gly Asp
            100                 105                 110
Gln Val Lys Leu Val Leu Ser Val Glu Leu Met Gly Arg His Ser Asn
        115                 120                 125
Val Ile Leu Tyr Asp Gln Gln Asn Gly His Ile Ile Asp Leu Leu Lys
    130                 135                 140
Arg Ile Asn Pro Asp Glu Asn Arg Ala Arg Ile Leu Leu Pro Lys Ala
145                 150                 155                 160
Lys Tyr Glu Leu Pro Pro Leu Lys Pro Gly Ile Asn Gly Leu Val Val
                165                 170                 175
Thr Glu Asn Gln Phe Lys Lys Leu Ser Asn Glu Lys Ser Ile Pro Asp
            180                 185                 190
Leu Val Lys Ser Met Asp Gly Leu Asp Arg Asp Asp Arg Glu Glu Leu
        195                 200                 205
Thr Gly Tyr Leu Glu Asp Asp Phe Ser Tyr Ser Ser Phe Lys Thr Phe

210                 215                 220
Phe Asp Gln Phe Asn Asn Pro Arg Ala Phe Val Leu Lys Thr Pro Arg
225                 230                 235                 240
Asn Lys Arg Lys Ile Phe Cys Tyr Leu Pro Tyr His Leu Asp Leu Glu
                245                 250                 255
Lys Glu Asn Ser Asn Pro Asp Leu Asn Lys Gly Leu Asp Glu Phe Tyr
            260                 265                 270
Glu Tyr Gln Ala Thr Arg Asp Trp Val Lys Gln Arg Ala Ser Gln Val
        275                 280                 285
Glu Arg Val Val Lys Asn Glu Gln Asn Lys Leu Ser Lys Lys Ile Lys
    290                 295                 300
Lys Leu Glu Lys Gln Leu Asn Leu Ala Glu Asn Ser Glu Gly Tyr Arg
305                 310                 315                 320
Ile Lys Gly Glu Ile Leu Asn Ala Asn Leu Gly Gln Val Lys Pro Gly
                325                 330                 335
Met Thr Glu Val Ser Leu Pro Asn Tyr Tyr Glu Asn Asn Lys Pro Ile
            340                 345                 350
Ser Ile Lys Leu Asp Pro Ala Leu Ser Pro Ala Arg Asn Ala Gln Lys
        355                 360                 365
Tyr Phe Thr Arg Tyr Lys Lys Leu Arg Asp Ser Ile Lys His Val Asn
    370                 375                 380
Glu Gln Ile Lys Ile Ala Lys Glu Asn Leu Arg Tyr Phe Asp Ser Ile
385                 390                 395                 400
Gln Thr Ala Ile Asp Asn Ala Asp Pro Gln Asp Ile Asp Gln Ile Thr
                405                 410                 415
Asp Glu Leu Ile Asn Gln Gly Tyr Ile Arg Lys Gln Gln Lys Asn Arg
            420                 425                 430
Arg Lys Lys Lys Ile Thr Glu Arg Asn Leu Asn Glu Phe Gln Leu Ser
        435                 440                 445
Ser Gly Lys His Val Leu Val Gly Lys Asn Asn Tyr Gln Asn Asp Trp
    450                 455                 460
Leu Thr Leu Lys Lys Ala Asn Lys Leu Asp Tyr Trp Phe His Val Lys
465                 470                 475                 480
Asn Met Pro Gly Ser His Val Ile Leu Arg Asp Asp Gln Pro Thr Asp
                485                 490                 495
Gln Asp Ile Lys Glu Ala Ala Glu Ile Ala Ala Phe Phe Ser Lys Ala
            500                 505                 510
Lys Asn Ser Ala His Val Gln Val Asp Tyr Val Gln Asp Lys Arg Val
        515                 520                 525
Lys Lys Pro Asn Gly Ala Lys Pro Gly Phe Val Ile Tyr Thr Gly Gln
    530                 535                 540
Asn Ser Ile Glu Val Thr Pro Lys Glu Asp Glu Ile Met Ala Lys Lys
545                 550                 555                 560
Ile Asn Lys

<210> 21
<211> 1212
<212> DNA
<213> Lactobacillus acidophilus

<220>
<221> CDS
<222> (13)...(1212)
<223> metK ORF LBA1622

<400> 21
aggggacta at atg gaa aaa cgt tta ttt act tca gaa tca gtt tct gaa 51
             Met Glu Lys Arg Leu Phe Thr Ser Glu Ser Val Ser Glu
              1               5                  10

gga cat cca gat aaa gtt gct gac caa att tca gat gca att tta gat    99
Gly His Pro Asp Lys Val Ala Asp Gln Ile Ser Asp Ala Ile Leu Asp

15                  20                  25

gcg atg ttg aaa aaa gat cct aat tca cac gta gct tgt gaa acg att     147
Ala Met Leu Lys Lys Asp Pro Asn Ser His Val Ala Cys Glu Thr Ile
 30                  35                  40                  45

gta act aca gga atg gtt ttt gta ttt ggt gaa att tca aca agt gca     195
Val Thr Thr Gly Met Val Phe Val Phe Gly Glu Ile Ser Thr Ser Ala
                 50                  55                  60

tat gtt gat att caa gat gta gta cga aaa acc att tta aaa ata ggc     243
Tyr Val Asp Ile Gln Asp Val Val Arg Lys Thr Ile Leu Lys Ile Gly
             65                  70                  75

tat gat cgt cca gag tta ggt ttc gat ggt aat aac tgt gct gta atg     291
Tyr Asp Arg Pro Glu Leu Gly Phe Asp Gly Asn Asn Cys Ala Val Met
         80                  85                  90

gtg gat att gat gaa caa tca cct gat atc gct gat ggt gtt gat cac     339
Val Asp Ile Asp Glu Gln Ser Pro Asp Ile Ala Asp Gly Val Asp His
     95                 100                 105

tca ctt gaa act aga gaa aat aag tct gat aac gat gaa ttg gac caa     387
Ser Leu Glu Thr Arg Glu Asn Lys Ser Asp Asn Asp Glu Leu Asp Gln
110                 115                 120                 125

att ggt gca ggt gac caa ggt tta atg ttt gga ttt gct att aag gaa     435
Ile Gly Ala Gly Asp Gln Gly Leu Met Phe Gly Phe Ala Ile Lys Glu
                130                 135                 140

act cca gaa ctt atg ccg cta cca att tca ctt tcg cat cgt tta atg     483
Thr Pro Glu Leu Met Pro Leu Pro Ile Ser Leu Ser His Arg Leu Met
            145                 150                 155

agg cgt gtg gct tca ctt aga aaa gac cat act tta gaa tgg ctt cgt     531
Arg Arg Val Ala Ser Leu Arg Lys Asp His Thr Leu Glu Trp Leu Arg
        160                 165                 170

cca gat gct aaa gct caa gtt act gta gaa tac gac gaa aat ggt aaa     579
Pro Asp Ala Lys Ala Gln Val Thr Val Glu Tyr Asp Glu Asn Gly Lys
    175                 180                 185

cca ctt tgt gtt gat act gtg gtt atc tct act caa act gat gca gaa     627
Pro Leu Cys Val Asp Thr Val Val Ile Ser Thr Gln Thr Asp Ala Glu
190                 195                 200                 205

gta tca aat gaa gaa atc tgt cgt gca atg atc gac cta gta att aaa     675
Val Ser Asn Glu Glu Ile Cys Arg Ala Met Ile Asp Leu Val Ile Lys
                210                 215                 220

gaa gtt att cca gct aag tat ttg gac gaa aat act aag ttc ttg atc     723
Glu Val Ile Pro Ala Lys Tyr Leu Asp Glu Asn Thr Lys Phe Leu Ile
            225                 230                 235

aac cca tct ggt cgt ttc gtt atc ggt gga cca aag ggt gat tct ggt     771
Asn Pro Ser Gly Arg Phe Val Ile Gly Gly Pro Lys Gly Asp Ser Gly
        240                 245                 250

tta acg ggt cgt aag att att gtt gat act tat ggt gga tac gca cgc     819
Leu Thr Gly Arg Lys Ile Ile Val Asp Thr Tyr Gly Gly Tyr Ala Arg
    255                 260                 265

cat ggt ggt ggt gca ttt tca ggt aaa gat cct aca aag gtt gac cgt     867
His Gly Gly Gly Ala Phe Ser Gly Lys Asp Pro Thr Lys Val Asp Arg

270                 275                 280                 285

agt gct agt tat gct gca aga tat gta gca aag aat att gta gct gca     915
Ser Ala Ser Tyr Ala Ala Arg Tyr Val Ala Lys Asn Ile Val Ala Ala
                290                 295                 300

ggt tta gcg tat cgt tgt gaa gtt caa tta gca tat gcc att ggt gtt     963
Gly Leu Ala Tyr Arg Cys Glu Val Gln Leu Ala Tyr Ala Ile Gly Val
            305                 310                 315

gct cac cct gta tcg att atg att gac aca gca ggt act ggt aca gtt     1011
Ala His Pro Val Ser Ile Met Ile Asp Thr Ala Gly Thr Gly Thr Val
        320                 325                 330

gat gat gaa ctt tta act gaa gca gtt cgt aat gta ttt gat ctt cgt     1059
Asp Asp Glu Leu Leu Thr Glu Ala Val Arg Asn Val Phe Asp Leu Arg
    335                 340                 345

cca gct ggc att att aag atg cta gac ttg cgt cgt cct att tat gag     1107
Pro Ala Gly Ile Ile Lys Met Leu Asp Leu Arg Arg Pro Ile Tyr Glu
350                 355                 360                 365

caa act gct gca tat ggt cac ttc ggt aga aca gac gtt gat tta cca     1155
Gln Thr Ala Ala Tyr Gly His Phe Gly Arg Thr Asp Val Asp Leu Pro
                370                 375                 380

tgg gaa aag acc gat aaa act caa gct ttg ctt gat tac atc aaa aat     1203
Trp Glu Lys Thr Asp Lys Thr Gln Ala Leu Leu Asp Tyr Ile Lys Asn
            385                 390                 395

aat caa taa                                                         1212
Asn Gln  *

<210> 22
<211> 399
<212> PRT
<213> Lactobacillus acidophilus

<400> 22
Met Glu Lys Arg Leu Phe Thr Ser Glu Ser Val Ser Glu Gly His Pro
 1               5                  10                  15
Asp Lys Val Ala Asp Gln Ile Ser Asp Ala Ile Leu Asp Ala Met Leu
            20                  25                  30
Lys Lys Asp Pro Asn Ser His Val Ala Cys Glu Thr Ile Val Thr Thr
        35                  40                  45
Gly Met Val Phe Val Phe Gly Glu Ile Ser Thr Ser Ala Tyr Val Asp
    50                  55                  60
Ile Gln Asp Val Val Arg Lys Thr Ile Leu Lys Ile Gly Tyr Asp Arg
65                  70                  75                  80
Pro Glu Leu Gly Phe Asp Gly Asn Asn Cys Ala Val Met Val Asp Ile
                85                  90                  95
Asp Glu Gln Ser Pro Asp Ile Ala Asp Gly Val Asp His Ser Leu Glu
            100                 105                 110
Thr Arg Glu Asn Lys Ser Asp Asn Asp Glu Leu Asp Gln Ile Gly Ala
        115                 120                 125
Gly Asp Gln Gly Leu Met Phe Gly Phe Ala Ile Lys Glu Thr Pro Glu
    130                 135                 140
Leu Met Pro Leu Pro Ile Ser Leu Ser His Arg Leu Met Arg Arg Val
145                 150                 155                 160
Ala Ser Leu Arg Lys Asp His Thr Leu Glu Trp Leu Arg Pro Asp Ala
                165                 170                 175
Lys Ala Gln Val Thr Val Glu Tyr Asp Glu Asn Gly Lys Pro Leu Cys

180                 185                 190
Val Asp Thr Val Val Ile Ser Thr Gln Thr Asp Ala Glu Val Ser Asn
        195                 200                 205
Glu Glu Ile Cys Arg Ala Met Ile Asp Leu Val Ile Lys Glu Val Ile
    210                 215                 220
Pro Ala Lys Tyr Leu Asp Glu Asn Thr Lys Phe Leu Ile Asn Pro Ser
225                 230                 235                 240
Gly Arg Phe Val Ile Gly Gly Pro Lys Gly Asp Ser Gly Leu Thr Gly
                245                 250                 255
Arg Lys Ile Ile Val Asp Thr Tyr Gly Gly Tyr Ala Arg His Gly Gly
            260                 265                 270
Gly Ala Phe Ser Gly Lys Asp Pro Thr Lys Val Asp Arg Ser Ala Ser
        275                 280                 285
Tyr Ala Ala Arg Tyr Val Ala Lys Asn Ile Val Ala Ala Gly Leu Ala
    290                 295                 300
Tyr Arg Cys Glu Val Gln Leu Ala Tyr Ala Ile Gly Val Ala His Pro
305                 310                 315                 320
Val Ser Ile Met Ile Asp Thr Ala Gly Thr Gly Thr Val Asp Asp Glu
                325                 330                 335
Leu Leu Thr Glu Ala Val Arg Asn Val Phe Asp Leu Arg Pro Ala Gly
            340                 345                 350
Ile Ile Lys Met Leu Asp Leu Arg Arg Pro Ile Tyr Glu Gln Thr Ala
        355                 360                 365
Ala Tyr Gly His Phe Gly Arg Thr Asp Val Asp Leu Pro Trp Glu Lys
    370                 375                 380
Thr Asp Lys Thr Gln Ala Leu Leu Asp Tyr Ile Lys Asn Asn Gln
385                 390                 395

<210> 23
<211> 28
<212> DNA
<213> Artificial Sequence

<220>
<223> Oligonucleotide

<400> 23
gatcagatct aagttaaggc accttacg                                        28

<210> 24
<211> 28
<212> DNA
<213> Artificial Sequence

<220>
<223> Oligonucleotide

<400> 24
gatctctaga tttcgaatgg gtcatcac                                        28

<210> 25
<211> 29
<212> DNA
<213> Artificial Sequence

<220>
<223> Oligonucleotide

<400> 25
gatctctaga tgacagaaga cgatgaatg                                       29

<210> 26
<211> 29

<212> DNA
<213> Artificial Sequence

<220>
<223> Oligonucleotide

<400> 26
gatcagatct attgcgacta agttcagac                                          29

<210> 27
<211> 36
<212> DNA
<213> Artificial Sequence

<220>
<223> Oligonucleotide

<400> 27
gatcgaattc tcgttcggtt gaactaaacg taagtc                                  36

<210> 28
<211> 36
<212> DNA
<213> Artificial Sequence

<220>
<223> Oligonucleotide

<400> 28
gatcgaattc cgaacaggat tccacctaat cgtttg                                  36

<210> 29
<211> 22
<212> DNA
<213> Artificial Sequence

<220>
<223> Oligonucleotide

<400> 29
gccaacttag ccttaagcac tc                                                 22

<210> 30
<211> 22
<212> DNA
<213> Artificial Sequence

<220>
<223> Oligonucleotide

<400> 30
ttgttcctgc tcctcagcct tc                                                 22

<210> 31
<211> 20
<212> DNA
<213> Artificial Sequence

<220>
<223> Oligonucleotide

<400> 31
tgctttagca acttcagtag                                                    20

<210> 32
<211> 20
<212> DNA
<213> Artificial Sequence

<220>
<223> Oligonucleotide

<400> 32
taaagttaag gcaccttacg                                                20

<210> 33
<211> 794
<212> DNA
<213> Lactobacillus acidophilus

<220>
<221> CDS
<222> (12)...(794)
<223> ORF LBA1735

<400> 33
aggaggacaa a atg cca tta ttt aag aaa aag cgt ggt aca gat gaa act   50
             Met Pro Leu Phe Lys Lys Lys Arg Gly Thr Asp Glu Thr
              1               5                  10

att aaa cat ggt gct aag tta atc act gta gct aag cca aag agt cca   98
Ile Lys His Gly Ala Lys Leu Ile Thr Val Ala Lys Pro Lys Ser Pro
     15                  20                  25

ata gct gaa caa ttc cgt act gtt cgt act aat att aac ttt atg gca   146
Ile Ala Glu Gln Phe Arg Thr Val Arg Thr Asn Ile Asn Phe Met Ala
 30                  35                  40                  45

gtg gat cat gac att aag tct tta gca ttt act tct gct aat att agt   194
Val Asp His Asp Ile Lys Ser Leu Ala Phe Thr Ser Ala Asn Ile Ser
                 50                  55                  60

gaa ggt aaa tct act gtg gct gct aac gtt gct gtt act tat gct caa   242
Glu Gly Lys Ser Thr Val Ala Ala Asn Val Ala Val Thr Tyr Ala Gln
             65                  70                  75

gca ggt cgt aaa gtc tta ttg gtc gat gcc gac tta cgt aga cct aca   290
Ala Gly Arg Lys Val Leu Leu Val Asp Ala Asp Leu Arg Arg Pro Thr
         80                  85                  90

gtg cac tca acc ttt aac tta agt aat cat gtt ggt ctt agt acg gtt   338
Val His Ser Thr Phe Asn Leu Ser Asn His Val Gly Leu Ser Thr Val
     95                 100                 105

atc tct tca act gct aag gaa gtt gat ctt gat agc gta gta caa gag   386
Ile Ser Ser Thr Ala Lys Glu Val Asp Leu Asp Ser Val Val Gln Glu
110                 115                 120                 125

agc ggc gta gat aat ctt tac gtg tta acg gct ggt ccg atg cca cct   434
Ser Gly Val Asp Asn Leu Tyr Val Leu Thr Ala Gly Pro Met Pro Pro
                130                 135                 140

aac ccg gca gaa ctg ata ggt tct aag cgt atg cgt gac ttt gtt aaa   482
Asn Pro Ala Glu Leu Ile Gly Ser Lys Arg Met Arg Asp Phe Val Lys
            145                 150                 155

ctt act gaa gaa cac tac gat tta gtt att atc gac tta gct cct gtt   530
Leu Thr Glu Glu His Tyr Asp Leu Val Ile Ile Asp Leu Ala Pro Val

160                 165                 170

ctt gaa gta tct gat aca caa gaa ctt gct agt cac tta gat ggg gtt      578
Leu Glu Val Ser Asp Thr Gln Glu Leu Ala Ser His Leu Asp Gly Val
    175                 180                 185

gtc tta gta gtt cgc caa ggg aag acg caa aag atg gcc att aag cgt      626
Val Leu Val Val Arg Gln Gly Lys Thr Gln Lys Met Ala Ile Lys Arg
190                 195                 200                 205

gct gtt gaa atg ctt gaa ttt gca aag gca cgt atc ttg ggt tac atc      674
Ala Val Glu Met Leu Glu Phe Ala Lys Ala Arg Ile Leu Gly Tyr Ile
                210                 215                 220

atg aat gat gta agt tct gat aat gcg ggt tat ggc tat ggt tac ggc      722
Met Asn Asp Val Ser Ser Asp Asn Ala Gly Tyr Gly Tyr Gly Tyr Gly
            225                 230                 235

tac ggc tat ggt tat ggc tac gga gaa gaa gat aca aag aaa aaa gga      770
Tyr Gly Tyr Gly Tyr Gly Tyr Gly Glu Glu Asp Thr Lys Lys Lys Gly
        240                 245                 250

ttg ttc tct aaa ttt agg aag taa                                      794
Leu Phe Ser Lys Phe Arg Lys  *
    255                 260


<210> 34
<211> 260
<212> PRT
<213> Lactobacillus acidophilus

<400> 34
Met Pro Leu Phe Lys Lys Lys Arg Gly Thr Asp Glu Thr Ile Lys His
 1               5                  10                  15
Gly Ala Lys Leu Ile Thr Val Ala Lys Pro Lys Ser Pro Ile Ala Glu
            20                  25                  30
Gln Phe Arg Thr Val Arg Thr Asn Ile Asn Phe Met Ala Val Asp His
        35                  40                  45
Asp Ile Lys Ser Leu Ala Phe Thr Ser Ala Asn Ile Ser Glu Gly Lys
    50                  55                  60
Ser Thr Val Ala Ala Asn Val Ala Val Thr Tyr Ala Gln Ala Gly Arg
65                  70                  75                  80
Lys Val Leu Leu Val Asp Ala Asp Leu Arg Arg Pro Thr Val His Ser
                85                  90                  95
Thr Phe Asn Leu Ser Asn His Val Gly Leu Ser Thr Val Ile Ser Ser
            100                 105                 110
Thr Ala Lys Glu Val Asp Leu Asp Ser Val Val Gln Glu Ser Gly Val
        115                 120                 125
Asp Asn Leu Tyr Val Leu Thr Ala Gly Pro Met Pro Pro Asn Pro Ala
    130                 135                 140
Glu Leu Ile Gly Ser Lys Arg Met Arg Asp Phe Val Lys Leu Thr Glu
145                 150                 155                 160
Glu His Tyr Asp Leu Val Ile Ile Asp Leu Ala Pro Val Leu Glu Val
                165                 170                 175
Ser Asp Thr Gln Glu Leu Ala Ser His Leu Asp Gly Val Val Leu Val
            180                 185                 190
Val Arg Gln Gly Lys Thr Gln Lys Met Ala Ile Lys Arg Ala Val Glu
        195                 200                 205
Met Leu Glu Phe Ala Lys Ala Arg Ile Leu Gly Tyr Ile Met Asn Asp
    210                 215                 220
Val Ser Ser Asp Asn Ala Gly Tyr Gly Tyr Gly Tyr Gly Tyr Gly Tyr
225                 230                 235                 240
Gly Tyr Gly Tyr Gly Glu Glu Asp Thr Lys Lys Lys Gly Leu Phe Ser

245                 250                 255
Lys Phe Arg Lys
            260


<210> 35
<211> 887
<212> DNA
<213> Lactobacillus acidophilus

<220>
<221> CDS
<222> (12)...(887)
<223> ORF LBA1736

<400> 35
aggaaagaga t atg gaa caa aaa caa gaa caa gaa aat aca atc gat ctt   50
             Met Glu Gln Lys Gln Glu Gln Glu Asn Thr Ile Asp Leu
              1               5                  10

acc caa tta tta cgc att tgc cgt aag cat atc tgg gca ttg att ctt   98
Thr Gln Leu Leu Arg Ile Cys Arg Lys His Ile Trp Ala Leu Ile Leu
     15                  20                  25

tgg agt gta ggt ctt gcc tta gtg ggc tgg gga gtt tca gaa ttt gta   146
Trp Ser Val Gly Leu Ala Leu Val Gly Trp Gly Val Ser Glu Phe Val
 30                  35                  40                  45

att tct cct aaa tat act tca act gct caa tta tta gtt aac caa aag   194
Ile Ser Pro Lys Tyr Thr Ser Thr Ala Gln Leu Leu Val Asn Gln Lys
                 50                  55                  60

agt cgt aac aat gat cca aac gca gct tat gcg act caa caa gct aac   242
Ser Arg Asn Asn Asp Pro Asn Ala Ala Tyr Ala Thr Gln Gln Ala Asn
             65                  70                  75

atg cag atg gtt act act tat aaa gac att gta aca agt aac aag atc   290
Met Gln Met Val Thr Thr Tyr Lys Asp Ile Val Thr Ser Asn Lys Ile
         80                  85                  90

tta aca gaa gct tct aat cgt ttg gcc aat cca act gtt gtt gtg aaa   338
Leu Thr Glu Ala Ser Asn Arg Leu Ala Asn Pro Thr Val Val Val Lys
     95                 100                 105

aaa gca caa aaa gca gtg tac aga act gat gaa aat ggt aga aga aga   386
Lys Ala Gln Lys Ala Val Tyr Arg Thr Asp Glu Asn Gly Arg Arg Arg
110                 115                 120                 125

tta gta aga aaa gct caa cca gct gta att gaa cga agc ggt aag agt   434
Leu Val Arg Lys Ala Gln Pro Ala Val Ile Glu Arg Ser Gly Lys Ser
                130                 135                 140

tat tca gtt tct gca agt gaa ctt gct aag agt atc tca gta ggt acc   482
Tyr Ser Val Ser Ala Ser Glu Leu Ala Lys Ser Ile Ser Val Gly Thr
            145                 150                 155

caa caa caa tca caa gtc ttc tca att tca gcg gaa gcc gat aca cca   530
Gln Gln Gln Ser Gln Val Phe Ser Ile Ser Ala Glu Ala Asp Thr Pro
        160                 165                 170

gct aag gcg aag gca gaa gta aat gca gta gct gaa acg ttc cgt aaa   578
Ala Lys Ala Lys Ala Glu Val Asn Ala Val Ala Glu Thr Phe Arg Lys
    175                 180                 185

gaa att cct act att atg agc gtt aat aac gta acg att gtg gca aat     626
Glu Ile Pro Thr Ile Met Ser Val Asn Asn Val Thr Ile Val Ala Asn
190                 195                 200                 205

ggt act aat ggt gtt caa tcc tca cct aac gtt aag ttg ttc act cta     674
Gly Thr Asn Gly Val Gln Ser Ser Pro Asn Val Lys Leu Phe Thr Leu
                210                 215                 220

gct gga ttt gta gtt ggg tta gta ttg agc ttt gct gta gtt att att     722
Ala Gly Phe Val Val Gly Leu Val Leu Ser Phe Ala Val Val Ile Ile
            225                 230                 235

cgt gaa atg agt aat act act gtt cgt gac gat gaa ttc tta act cgt     770
Arg Glu Met Ser Asn Thr Thr Val Arg Asp Asp Glu Phe Leu Thr Arg
        240                 245                 250

gaa tta ggc tta act aac tta ggt caa att gct cac ttc cac ttg tca     818
Glu Leu Gly Leu Thr Asn Leu Gly Gln Ile Ala His Phe His Leu Ser
    255                 260                 265

tct tca ttt act att aaa aag agt gca aat atg act aat cgt gga caa     866
Ser Ser Phe Thr Ile Lys Lys Ser Ala Asn Met Thr Asn Arg Gly Gln
270                 275                 280                 285

gct aag aga cgt aga gta taa                                         887
Ala Lys Arg Arg Arg Val  *
                290

<210> 36
<211> 291
<212> PRT
<213> Lactobacillus acidophilus

<400> 36
Met Glu Gln Lys Gln Glu Gln Glu Asn Thr Ile Asp Leu Thr Gln Leu
1               5                   10                  15
Leu Arg Ile Cys Arg Lys His Ile Trp Ala Leu Ile Leu Trp Ser Val
            20                  25                  30
Gly Leu Ala Leu Val Gly Trp Gly Val Ser Glu Phe Val Ile Ser Pro
        35                  40                  45
Lys Tyr Thr Ser Thr Ala Gln Leu Leu Val Asn Gln Lys Ser Arg Asn
    50                  55                  60
Asn Asp Pro Asn Ala Ala Tyr Ala Thr Gln Gln Ala Asn Met Gln Met
65                  70                  75                  80
Val Thr Thr Tyr Lys Asp Ile Val Thr Ser Asn Lys Ile Leu Thr Glu
                85                  90                  95
Ala Ser Asn Arg Leu Ala Asn Pro Thr Val Val Val Lys Lys Ala Gln
            100                 105                 110
Lys Ala Val Tyr Arg Thr Asp Glu Asn Gly Arg Arg Arg Leu Val Arg
        115                 120                 125
Lys Ala Gln Pro Ala Val Ile Glu Arg Ser Gly Lys Ser Tyr Ser Val
    130                 135                 140
Ser Ala Ser Glu Leu Ala Lys Ser Ile Ser Val Gly Thr Gln Gln Gln
145                 150                 155                 160
Ser Gln Val Phe Ser Ile Ser Ala Glu Ala Asp Thr Pro Ala Lys Ala
                165                 170                 175
Lys Ala Glu Val Asn Ala Val Ala Glu Thr Phe Arg Lys Glu Ile Pro
            180                 185                 190
Thr Ile Met Ser Val Asn Asn Val Thr Ile Val Ala Asn Gly Thr Asn
        195                 200                 205
Gly Val Gln Ser Ser Pro Asn Val Lys Leu Phe Thr Leu Ala Gly Phe
    210                 215                 220
Val Val Gly Leu Val Leu Ser Phe Ala Val Val Ile Ile Arg Glu Met

225                 230                 235                 240
Ser Asn Thr Thr Val Arg Asp Asp Glu Phe Leu Thr Arg Glu Leu Gly
                245                 250                 255
Leu Thr Asn Leu Gly Gln Ile Ala His Phe His Leu Ser Ser Ser Phe
            260                 265                 270
Thr Ile Lys Lys Ser Ala Asn Met Thr Asn Arg Gly Gln Ala Lys Arg
        275                 280                 285
Arg Arg Val
    290

<210> 37
<211> 1287
<212> DNA
<213> Lactobacillus acidophilus

<220>
<221> CDS
<222> (1)...(1287)
<223> ORF LBA1923

<400> 37
atg agt aat aaa cgc cgg ctg tgg caa att ttt ggc cca gtt ctt tgc      48
Met Ser Asn Lys Arg Arg Leu Trp Gln Ile Phe Gly Pro Val Leu Cys
 1               5                  10                  15

gct ttt atc ctt tta tta gtt gta ttt ctt att ccc tgg gaa aga act      96
Ala Phe Ile Leu Leu Leu Val Val Phe Leu Ile Pro Trp Glu Arg Thr
            20                  25                  30

ttt tct aag caa act atc tat gaa gca gct gcc tca caa aat agt act     144
Phe Ser Lys Gln Thr Ile Tyr Glu Ala Ala Ala Ser Gln Asn Ser Thr
        35                  40                  45

gta ttt aag ggc agt aca atg aag caa gaa gct tat aaa gat ggt tat     192
Val Phe Lys Gly Ser Thr Met Lys Gln Glu Ala Tyr Lys Asp Gly Tyr
    50                  55                  60

gta cca ttc tat ggt tca agt gaa ttg tct aga ttt gat cca ctt cac     240
Val Pro Phe Tyr Gly Ser Ser Glu Leu Ser Arg Phe Asp Pro Leu His
65                  70                  75                  80

cct agt gtt att gct gaa aag tat cac aga aat tac cgt cca ttt ctt     288
Pro Ser Val Ile Ala Glu Lys Tyr His Arg Asn Tyr Arg Pro Phe Leu
                85                  90                  95

cta ggt gga cca ggt agt caa tct ttg gct caa ttc ttg ggg atg cag     336
Leu Gly Gly Pro Gly Ser Gln Ser Leu Ala Gln Phe Leu Gly Met Gln
            100                 105                 110

ggt aca act aaa cag ctt aaa aac aaa aag gct gta gtg att att tca     384
Gly Thr Thr Lys Gln Leu Lys Asn Lys Lys Ala Val Val Ile Ile Ser
        115                 120                 125

cca caa tgg ttt acc aag aaa ggc caa gat cct aat gca ttt gct tta     432
Pro Gln Trp Phe Thr Lys Lys Gly Gln Asp Pro Asn Ala Phe Ala Leu
    130                 135                 140

tat tat tca cca ctt caa gca tgt aac ttc ttg tta agt gct aag aat     480
Tyr Tyr Ser Pro Leu Gln Ala Cys Asn Phe Leu Leu Ser Ala Lys Asn
145                 150                 155                 160

aat aag act gat cgt tat gct gct aag cgt ctg ctt gat atg cca gat     528
Asn Lys Thr Asp Arg Tyr Ala Ala Lys Arg Leu Leu Asp Met Pro Asp

165                 170                 175

gta aag ggt gaa att aga aac agt ctt aag caa att gct gca ggt aaa     576
Val Lys Gly Glu Ile Arg Asn Ser Leu Lys Gln Ile Ala Ala Gly Lys
        180                 185                 190

aag cta act act ttt gaa aga ttt tat tta gaa aat cgt cgt aga atg     624
Lys Leu Thr Thr Phe Glu Arg Phe Tyr Leu Glu Asn Arg Arg Arg Met
        195                 200                 205

tta cgt aac gaa gat aac ttc ttt agt tca ttc caa tta cgc gat cgt     672
Leu Arg Asn Glu Asp Asn Phe Phe Ser Ser Phe Gln Leu Arg Asp Arg
    210                 215                 220

gta aat aag att caa aat aga gct aaa gta tta cct aat act tat tct     720
Val Asn Lys Ile Gln Asn Arg Ala Lys Val Leu Pro Asn Thr Tyr Ser
225                 230                 235                 240

gta gct gct ttg aac aag gtg gct gaa gaa cag gct gca gca cat act     768
Val Ala Ala Leu Asn Lys Val Ala Glu Glu Gln Ala Ala Ala His Thr
                245                 250                 255

act tca aat aac ttg gga att gac aat act ttc tat aga act cgt ttg     816
Thr Ser Asn Asn Leu Gly Ile Asp Asn Thr Phe Tyr Arg Thr Arg Leu
            260                 265                 270

cct aga aag gta tta aag aga ctc aag ggt agt caa cgt cac ttt gat     864
Pro Arg Lys Val Leu Lys Arg Leu Lys Gly Ser Gln Arg His Phe Asp
        275                 280                 285

tac gtt aga tct gtt gaa tat ggc gac ttc cag tta atg ctg gaa caa     912
Tyr Val Arg Ser Val Glu Tyr Gly Asp Phe Gln Leu Met Leu Glu Gln
    290                 295                 300

ttt gcc aag caa cat act aat gtg ttg ttc att att cca cca att aat     960
Phe Ala Lys Gln His Thr Asn Val Leu Phe Ile Ile Pro Pro Ile Asn
305                 310                 315                 320

ggt aag tgg atg aag tat act ggt tta tca caa aaa atg tat caa gaa     1008
Gly Lys Trp Met Lys Tyr Thr Gly Leu Ser Gln Lys Met Tyr Gln Glu
                325                 330                 335

tca gtt gct aaa att gaa caa caa ttg act agt caa ggt ttt gaa aat     1056
Ser Val Ala Lys Ile Glu Gln Gln Leu Thr Ser Gln Gly Phe Glu Asn
            340                 345                 350

att gca gat ctt tct aaa cgt ggt aat gaa aag tac ttc atg caa gat     1104
Ile Ala Asp Leu Ser Lys Arg Gly Asn Glu Lys Tyr Phe Met Gln Asp
        355                 360                 365

act att cac ctt ggt tgg aaa ggc tgg gta gct gtt gat caa gct gtt     1152
Thr Ile His Leu Gly Trp Lys Gly Trp Val Ala Val Asp Gln Ala Val
    370                 375                 380

aga cca ttt atg aag ttg cct aac gaa cgt tac aac tat gat atg tct     1200
Arg Pro Phe Met Lys Leu Pro Asn Glu Arg Tyr Asn Tyr Asp Met Ser
385                 390                 395                 400

aac tac tac ttc tca aag aag tgg cag aat aaa gat aac gtt aaa cgt     1248
Asn Tyr Tyr Phe Ser Lys Lys Trp Gln Asn Lys Asp Asn Val Lys Arg
                405                 410                 415

gta aat tta aat aat aaa gat cgt tta aaa gtg aag tag                 1287
Val Asn Leu Asn Asn Lys Asp Arg Leu Lys Val Lys  *

420                 425

<210> 38
<211> 428
<212> PRT
<213> Lactobacillus acidophilus

<400> 38
Met Ser Asn Lys Arg Arg Leu Trp Gln Ile Phe Gly Pro Val Leu Cys
1               5                   10                  15
Ala Phe Ile Leu Leu Leu Val Val Phe Leu Ile Pro Trp Glu Arg Thr
            20                  25                  30
Phe Ser Lys Gln Thr Ile Tyr Glu Ala Ala Ala Ser Gln Asn Ser Thr
        35                  40                  45
Val Phe Lys Gly Ser Thr Met Lys Gln Glu Ala Tyr Lys Asp Gly Tyr
    50                  55                  60
Val Pro Phe Tyr Gly Ser Ser Glu Leu Ser Arg Phe Asp Pro Leu His
65                  70                  75                  80
Pro Ser Val Ile Ala Glu Lys Tyr His Arg Asn Tyr Arg Pro Phe Leu
                85                  90                  95
Leu Gly Gly Pro Gly Ser Gln Ser Leu Ala Gln Phe Leu Gly Met Gln
            100                 105                 110
Gly Thr Thr Lys Gln Leu Lys Asn Lys Lys Ala Val Val Ile Ile Ser
        115                 120                 125
Pro Gln Trp Phe Thr Lys Lys Gly Gln Asp Pro Asn Ala Phe Ala Leu
    130                 135                 140
Tyr Tyr Ser Pro Leu Gln Ala Cys Asn Phe Leu Leu Ser Ala Lys Asn
145                 150                 155                 160
Asn Lys Thr Asp Arg Tyr Ala Ala Lys Arg Leu Leu Asp Met Pro Asp
                165                 170                 175
Val Lys Gly Glu Ile Arg Asn Ser Leu Lys Gln Ile Ala Ala Gly Lys
            180                 185                 190
Lys Leu Thr Thr Phe Glu Arg Phe Tyr Leu Glu Asn Arg Arg Arg Met
        195                 200                 205
Leu Arg Asn Glu Asp Asn Phe Phe Ser Ser Phe Gln Leu Arg Asp Arg
    210                 215                 220
Val Asn Lys Ile Gln Asn Arg Ala Lys Val Leu Pro Asn Thr Tyr Ser
225                 230                 235                 240
Val Ala Ala Leu Asn Lys Val Ala Glu Glu Gln Ala Ala Ala His Thr
                245                 250                 255
Thr Ser Asn Asn Leu Gly Ile Asp Asn Thr Phe Tyr Arg Thr Arg Leu
            260                 265                 270
Pro Arg Lys Val Leu Lys Arg Leu Lys Gly Ser Gln Arg His Phe Asp
        275                 280                 285
Tyr Val Arg Ser Val Glu Tyr Gly Asp Phe Gln Leu Met Leu Glu Gln
    290                 295                 300
Phe Ala Lys Gln His Thr Asn Val Leu Phe Ile Ile Pro Pro Ile Asn
305                 310                 315                 320
Gly Lys Trp Met Lys Tyr Thr Gly Leu Ser Gln Lys Met Tyr Gln Glu
                325                 330                 335
Ser Val Ala Lys Ile Glu Gln Gln Leu Thr Ser Gln Gly Phe Glu Asn
            340                 345                 350
Ile Ala Asp Leu Ser Lys Arg Gly Asn Glu Lys Tyr Phe Met Gln Asp
        355                 360                 365
Thr Ile His Leu Gly Trp Lys Gly Trp Val Ala Val Asp Gln Ala Val
    370                 375                 380
Arg Pro Phe Met Lys Leu Pro Asn Glu Arg Tyr Asn Tyr Asp Met Ser
385                 390                 395                 400
Asn Tyr Tyr Phe Ser Lys Lys Trp Gln Asn Lys Asp Asn Val Lys Arg
                405                 410                 415
Val Asn Leu Asn Asn Lys Asp Arg Leu Lys Val Lys
            420                 425

## Description

### FIELD OF THE INVENTION

The present invention concerns bacteria, particularly probiotic and lactic acid bacteria, and methods and constructs for the control of cell signaling therein.

### BACKGROUND OF THE INVENTION

In the small intestine, lactobacilli represent a major and important component of the commensal microflora. Various *Lactobacillus* species have been used as probiotic cultures selected for use in foods or dietary adjuncts based on specific benefits exacted from that organism and bioprocessing characteristics desired by the manufacturer. Not all probiotic cultures exhibit the same characteristics, making selection of these strains and clarification of their benefits paramount Lactic acid bacteria, and lactobacilli specifically, are a common genus included in food products for a range of prospective health-promoting factors.

Lactic acid bacteria (LAB) are naturally found in a variety of environmental niches where they exist as members of complex microbial communities. Survival in each niche depends on the ability of the organism to sense and respond to varying conditions such as temperature, pH, nutrient availability, and cell population density. One major niche that LAB frequently occupy is that of the mammalian gastrointestinal tract (GIT). Multiple signal transduction pathways likely control the expression of adhesion factors, stress response genes, and other genetic determinants that promote survival and competition of LAB within the various compartments of the GIT. Some intestinal lactobacilli, along with Gram-positive and Gram-negative pathogenic bacteria, have the ability to associate with the intestinal epithelium and mucosal layers therein. This association is thought to be important for the realization of certain probiotic properties including competitive exclusion, immunomodulation, and the delivery of biotherapeutics, among others. Although the molecular mechanisms involved with this association are not understood, it is clear that the process is complex, involving host-specific, bacterial-specific, and environmental factors.

Some intestinal lactobacilli, along with Gram-positive and Gram-negative pathogenic bacteria, have the ability to associate with the intestinal epithelium and mucosal layers therein. The ability to colonize these surfaces may afford probiotics a distinct advantage over other indigenous and pathogenic microflora. Of particular interest is the mechanism through which these bacteria survive and potentially colonize the kinetic environment of the intestinal tract. Certain environmental factors have been studied that influence adhesion including pH, growth phase, and the presence of other microorganisms.

Lactobacilli must maintain a balance between meeting their own growth requirements and surviving the hostile conditions, including gastric-acid shock, presented by the host defenses and competing microflora (Tannock (2005) Appl. Environ. Microbiol. 71:8419-8425). However, little is known about how these stressors influence the ability of lactobacilli to associate with the varied substrates in the intestinal environment.

### BRIEF SUMMARY OF THE INVENTION

Methods and compositions are provided which improve the adhesion of a bacteria to target substrates and/or improve the stress tolerance of a bacteria. Methods comprise exposing bacteria to adhesion adaptive conditions and thereby increasing the adhesion and/or stress tolerance of the bacteria. Further provided are bacteria having a modulated level of autoinducer-2 (AI-2). Compositions include recombinant bacteria expressing nucleic acid molecules involved in the pathway of autoinducer-2 production. Further provided are autoinducer-2-related fusion proteins; antigenic peptides, antibodies, and vectors. Methods of screening compounds or environmental conditions which will stimulate the production of autoinducer-2 and/or produce an adhesion adaptive response are further provided, as are various methods of use for the bacteria having increased adhesion and/or stress tolerance.

The present invention is explained in further detail in the drawings herein and the specification set forth below.

### BRIEF DESCRIPTION OF THE FIGURES

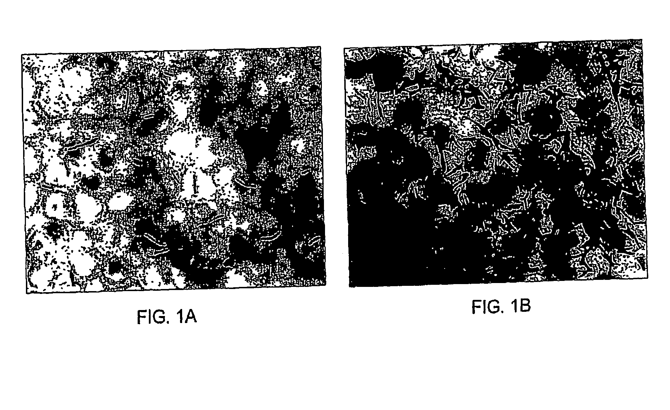

Figure 1 demonstrates the adhesion *of L. acidophilus* NCFM to Caco-2 cells *in vitro* as observed microscopically. Bacterial cells were either (a) incubated for one hour at 37°C in non-concentrated condition (1 x 10⁸ CFU/ml) or (b) pelleted and incubated in a 10 X concentrated condition (1 x 10⁹ CFU/ml) (AAR conditions) for 1 hour at 37°C.

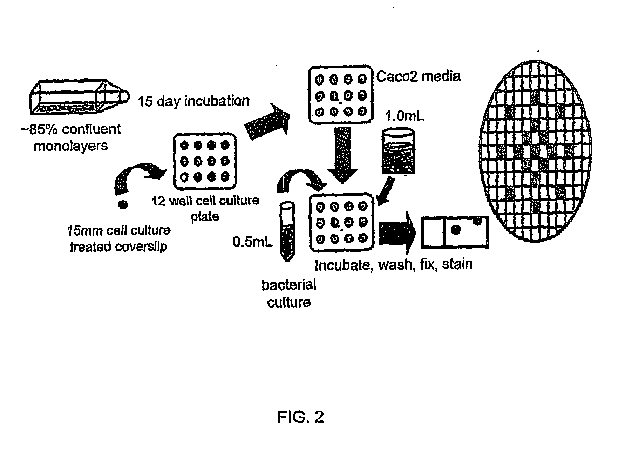

Figure 2 depicts the adhesion protocol.

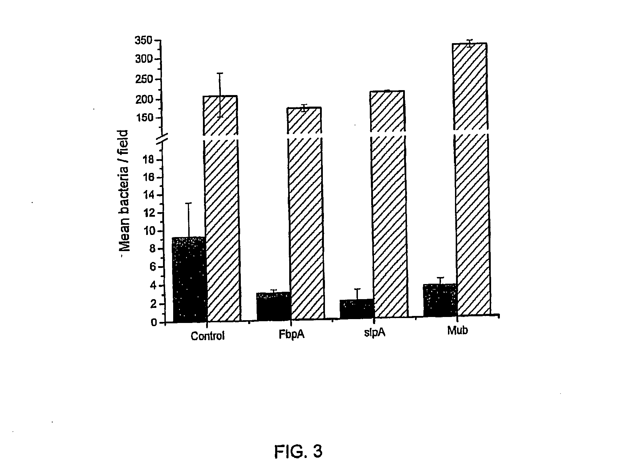

Figure 3 demonstrates adhesion properties of mutant strains and *NCFM::lacL* control strain to Caco-2 cells in standard incubation conditions (solid bars) and adhesion adaptive conditions (striped bars). Counts are expressed as mean bacteria adhering per microscopic field and error bars represent one standard deviation from the mean.

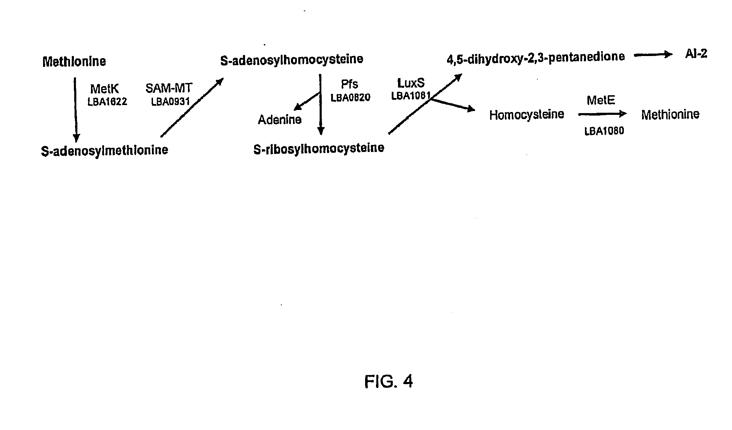

Figure 4 shows the pathway for the production of AI-2 from methionine in *L*. *acidophilus* NCFM. Essential intermediates for the production of AI-2 are represented in bold, and ORFs encoding each enzyme are listed under the enzyme name. The SAM-dependent methyltransferase is abbreviated SAM-MT. The final step involves the nonenzymatic circularization of 4,5-dihydroxy-2,3-pentanedione into AI-2.

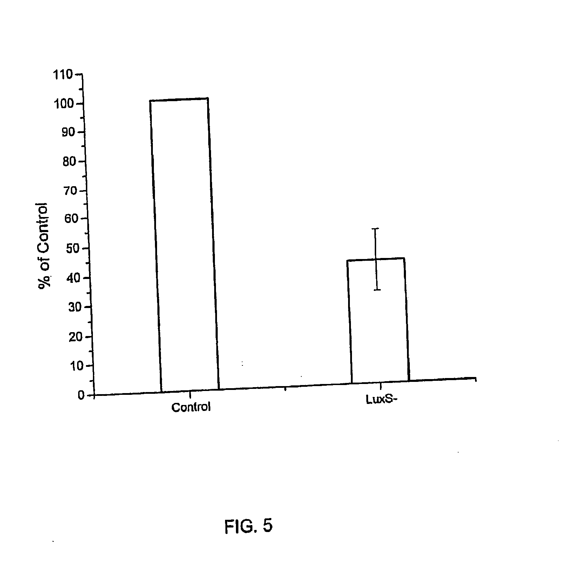

Figure 5 shows the adhesion ability of a LuxS⁻ mutant strain *of L. acidophilus* NCFM to Caco-2 cells compared to the NCFM::*lacL* control strain.

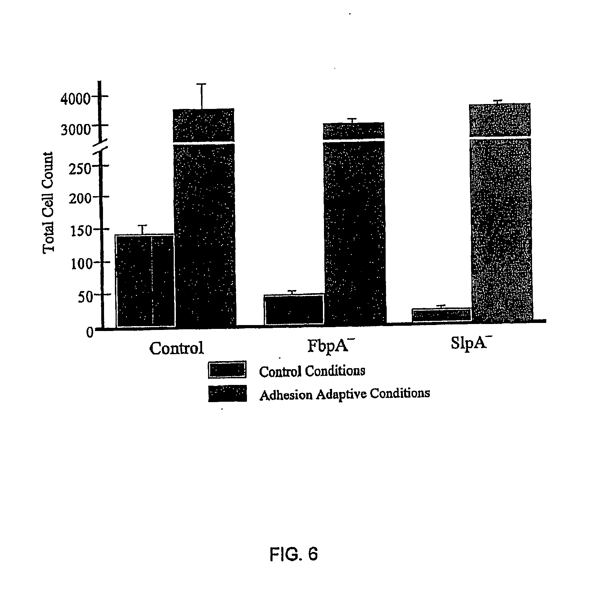

Figure 6 shows AI-2 produced during the growth phase *of L. acidophilus* NCFM, as detected from sterile neutralized supernatant (striped bars) and expressed as mean luminescence of the parent strain / mean luminescence of the LuxS- mutant strain. Growth *of L. acidophilus* NCFM, represented by OD₆₀₀ (solid line with circles) and the drop in pH (dashed line with triangles), coincides with rapid AI-2 production during logarithmic phase of growth. Each value represents the mean of triplicate experiments and error bars are one standard deviation from the mean.

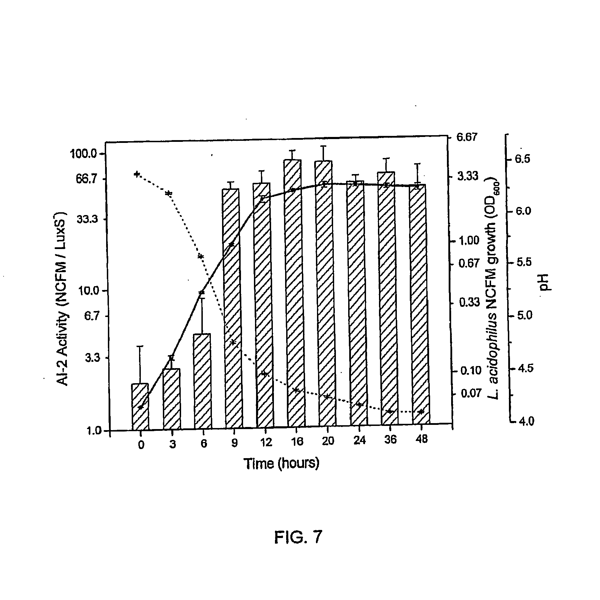

Figure 7 demonstrates the adhesion properties of control strain and selected mutant strains *of L. acidophilus* NCFM with and without exposure to the adhesion adaptive conditions.

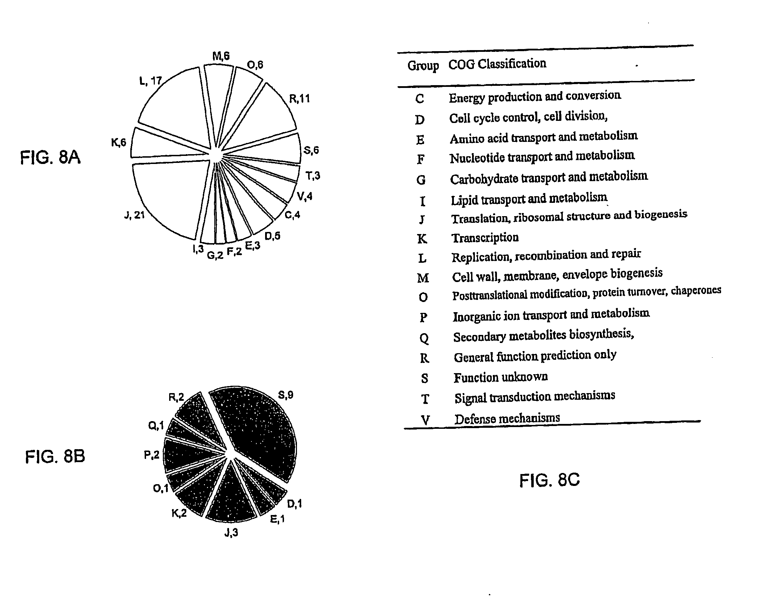

Figure 8 charts the COG classification of total ORFs overexpressed in the wild-type (Panel A) or LuxS- mutant strain (Panel B). COG groups (Panel C) are listed on pie charts with the number of overexpressed ORFs in that group (COG, # of ORFs).

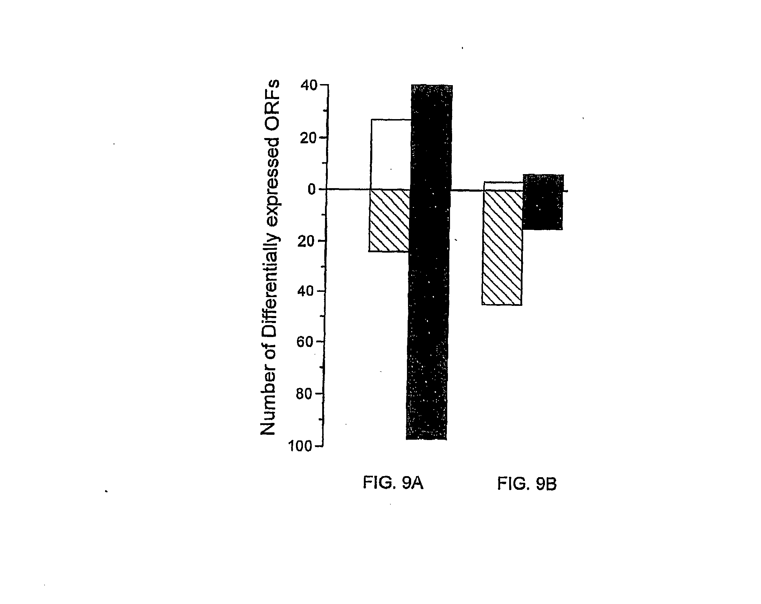

Figure 9 shows the number of ORFs whose expression increased (no slashes) or decreased (slashes) from (A) early to middle-log phase or (B) from middle to late-log phase. The wild type is represented by white bars and the LuxS- mutant strain represented by gray bars.

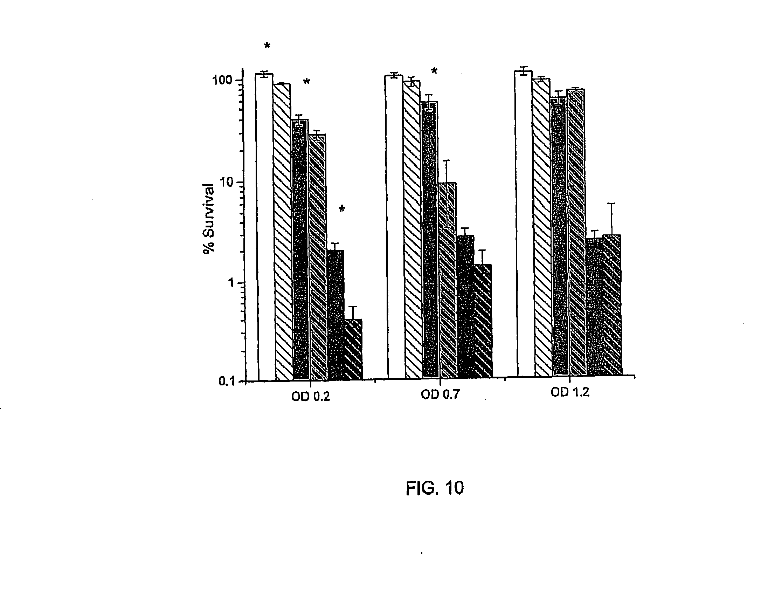

Figure 10 demonstrates bile tolerance of L. acidophilus NCFM (no slashes) and the LuxS- mutant strain (slashes) harvested at early-log (OD₆₀₀ 0.2), middle-log (OD₆₀₀ 0.7), and late-log (OD₆₀₀ 1.2) growth phase. Bacterial populations were diluted and plated on MRS supplemented with either 0.75 % (white bars), 1.0 % (gray bars), or 2.0 % (black bars) Oxgall. Percent survival was calculated by comparison to CFU/ml grown on non-supplemented MRS. Significant differences detected by the Student's t test (P < 0.05) are represented by an asterisk (*). Error bars represent one standard deviation from the mean.

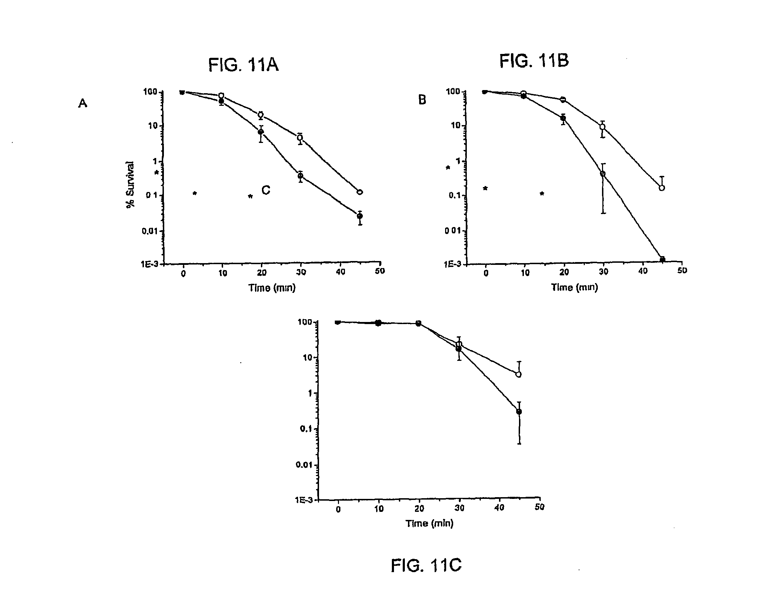

Figure 11 demonstrates survival of wild type (open circles) and LuxS- (closed circles) populations after heat stress at 55°C, when harvested at (Panel A) early-log, OD₆₀₀ 0.2, (Panel B) middle-log, OD₆₀₀ 0.7, or (Panel C) late-log, OD₆₀₀ 1.2, growth phase. An asterisk (*) indicates a statistically significant difference (P < 0.05) has been detected for that time point. Error bars represent one standard deviation from the mean.

### DETAILED DESCRIPTION OF THE INVENTION

The present inventions now will be described more fully hereinafter with reference to the accompanying drawings, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

As used herein, "a," "an" and "the" can be plural or singular as used throughout the specification and claims. For example "a" cell can mean a single cell or a multiplicity of cells.

Also as used herein, "and/or" refers to and encompasses any and all possible combinations of one or more of the associated listed items, as well as the lack of combinations when interpreted in the alternative ("or").

### I. Overview

Methods and compositions of the invention are provided which improve the adhesion of a bacteria to target substrates and/or improve stress tolerance of a bacteria. Increased adhesion of a commensal organism, such as a probiotic, to cells of the gastrointestinal tract or the urogenital tract find use in reducing the risk of infection of the gut, urogential tract, and wound sites. Similarly, improving the stress tolerance of a probiotic organism, such that the survival rate of the probiotic is increased in the harsh environment of the intestinal tract, will further aid in reducing the risk of infection of the gut, urogential tract, and wound sites.

In one embodiment, the present invention has identified "adhesion adaptive conditions." As used herein "adhesion adaptive conditions" comprise any physical, chemical, biological, or similar condition that improves the adhesion of bacteria to a substrate. The present invention has demonstrated that preconditioning bacterial cells to these adhesion adaptive conditions results in an increase in adhesion and/or an increase in stress tolerance. As used herein, the improved adhesion properties of the bacteria is referred to as the "adhesion adaptive response" or "AAR" Polypeptides and polynucleotides whose levels and/or activity are modulated under adhesion adaptive conditions are referenced to herein as "adhesion adaptive response-related" or "AAR-related" polypeptides or polynucleotides. Various AAR-related polynucleotide and polypeptides are disclosed herein. See Tables 8 and 9 below. Additional AAR-related polynucleotides, and their encoded polypeptides, are set forth in SEQ ID NO: 1, 2, 3, 4, 5, 6, 7, 8, 9, 10, 11, 12, 13,14, 15, 16, 17, 18, 19, 20, 21, 22, 33, 34, 35, 36, 37, and 38.

Methods and compositions are further provided which modulate the autoinducer-2 pathway of a bacteria, and thereby modulate the adhesion and/or the stress tolerance of the bacteria. More specifically, polynucleotides and polypeptides of the autoinducer-2 (AI-2) production pathway in bacteria are provided. Bacterial cells frequently communicate via quorum sensing mechanisms, which involve the density-dependant recognition of an autoinducer followed by changes in gene expression. One important quorum sensing system that can be used to communicate among and between species is based on a furanosyl borate diester called autoinducer-2 (AI-2), produced in four enzymatic steps from methionine. AI-2 regulates the expression of various phenotypes including virulence factors, DNA processing, cell morphology, motility, biofilm formation, toxin production, light production, and cell division in numerous species (Xavier et al. (2003) Curr. Opin. Microbiol. 6:191-197). Accordingly, methods and compositions are provided which allow for the modulation of the level and/or activity of the polypeptides in the AI-2 pathway and thereby the ability to modulate various bacterial phenotypes is provided. The polynucleotide and polypeptide sequences of the AI-2 pathway are set for in SEQ ID NO:1, 2, 3, 4, 13, 14, 15, 16, 21 or 22. Such sequences are referred to herein as "AI-2-related sequences".

### II. AI-2-related and AAR-Related Polypeptides and Polynucleotides

Compositions are provided which employ the various AAR-related and AI-2 related polynucleotides and polypeptides of the invention. The invention further provides fragments and variants of these AI-2-related or AAR-related sequences, which can also be used to practice the methods of the present invention. As used herein, the terms "gene" and "recombinant gene" refer to nucleic acid molecules comprising an open reading frame, particularly those encoding proteins involved in the production of AI-2 or in the AAR. Isolated nucleic acid molecules of the present invention comprise nucleic acid sequences encoding AI-2-related or AAR-related proteins set forth in SEQ ID NOS:2, 4, 6, 8, 10, 12, 14, 16, 18, 20, 22, 34, 36, or 38 the nucleic acid sequences set forth in SEQ ID NOS:1, 3, 5, 7, 9, 11, 13, 15, 17, 19, 21, 33, 35, or 37 and variants and fragments thereof. The present invention also encompasses antisense nucleic acid molecules, as described below.

Isolated polypeptides and proteins involved in the production of AI-2 or in the AAR, and variants and fragments thereof, are encompassed, as well as methods for producing those polypeptides. For purposes of the present invention, the terms "protein" and "polypeptide" are used interchangeably. Exemplary AI-2-related polypeptides include SEQ ID NOS:2, 4, 14, 16, 22. Exemplary AAR-related polypeptides include SEQ ID NO: 2, 4, 6, 8, 10, 12, 14, 16, 18, 20, 22, 34, 36, and 38.

The "production of AI-2" or the "adhesion adapted response (AAR)" refers to a biological or functional activity as determined *in vivo* or *in vitro* according to standard assay techniques. For example, the production of AI-2 can be measured using a reporter strain *Vibrio harveyi* and an autoinducer bioassay (DeKeersmaecker et al. (2003) Microbiology 149:1953-6). Additional assays involve, for example, measuring bacterial survival or growth under the adhesion adaptive conditions described elsewhere herein.

Bacteria and methods of the invention are useful for bacterial fermentations, for example where it is desired to stimulate the production of AI-2, or to promote adhesion or biofilm formation of the bacteria on a substrate for carrying out the fermentative process.

Bacteria of the invention are useful as probiotic bacteria, for example where it is desired to stimulate the production of AI-2, or to promote adhesion or biofilm formation of the bacteria on the gut or intestinal wall to competitively exclude other less desired bacteria therefrom.

Methods of inducing cell signaling or an adhesion adaptive response in bacteria are useful in stimulating the production of AI-2, or in promoting adhesion or biofilm formation of bacteria, including both recombinant and non-recombinant wild type bacteria, to stimulate the production of AI-2, or to promote adhesion or biofilm formation by probiotic bacteria.

The nucleic acid and polypeptide compositions encompassed by the present invention are isolated or substantially purified. By "isolated" or "substantially purified" is intended that the nucleic acid or polypeptide molecules, or biologically active fragments or variants, are substantially or essentially free from components normally found in association with the nucleic acid molecule or protein in its natural state. Such components include other cellular material, culture media from recombinant production, and various chemicals used in chemically synthesizing the proteins or nucleic acids. Preferably, an "isolated" nucleic acid of the present invention is free of nucleic acid sequences that flank the nucleic acid of interest in the genomic DNA of the organism from which the nucleic acid was derived (such as coding sequences present at the 5' or 3' ends). However, the molecule may include some additional bases or moieties that do not deleteriously affect the basic characteristics of the composition. For example, in various embodiments, the isolated nucleic acid molecule contains less than 5 kb, 4 kb, 3 kb, 2 kb, 1 kb, 0.5 kb, or 0.1 kb of nucleic acid sequence normally associated with the genomic DNA in the cells from which it was derived. Similarly, a substantially purified protein has less than about 30%, 20%, 10%, 5%, or 1% (by dry weight) of contaminating protein, or non-AI-2-related or non-AAR-related protein. When the protein is recombinantly produced, preferably culture medium represents less than 30%, 20%, 10%, or 5% of the volume of the protein preparation, and when the protein is produced chemically, preferably the preparations have less than about 30%, 20%, 10%, or 5% (by dry weight) of chemical precursors, or chemicals not related to the production of AI-2 or to the adhesion adaptive conditions.

### i. Fragments and Variants

The invention provides isolated nucleic acid molecules comprising nucleotide sequences encoding AI-2-related or AAR-related proteins, as well as the AI-2-related or AAR-related proteins encoded thereby. Fragments and variants of these nucleotide sequences and encoded proteins are also provided. By "fragment" of a nucleotide sequence or protein is intended a portion of the nucleotide or amino acid sequence.

Fragments of the nucleic acid molecules disclosed herein can be used as hybridization probes to identify AI-2-related or AAR-related protein-encoding nucleic acids, or can be used as primers in PCR amplification or mutation of AI-2-related or AAR-related nucleic acid molecules. Fragments of nucleic acids can also be bound to a physical substrate to comprise what may be considered a macro- or microarray (for example, U.S. Patent No. 5,837,832; U.S. Patent No. 5,861,242). Such arrays of nucleic acids may be used to study gene expression or to identify nucleic acid molecules with sufficient identity to the target sequences.

The present invention further provides a nucleic acid array or chip, i.e., a multitude of nucleic acids (e.g., DNA) as molecular probes precisely organized or arrayed on a solid support, which allow for the sequencing of genes, the study of mutations contained therein and/or the analysis of the expression of genes, as such arrays and chips are currently of interest given their very small size and their high capacity in terms of number of analyses.

The function of these nucleic acid arrays/chips is based on molecular probes, mainly oligonucleotides, which are attached to a carrier having a size of generally a few square centimeters or more, as desired. For an analysis, the carrier, such as in a DNA array/chip, is coated with DNA probes (e.g., oligonucleotides) that are arranged at a predetermined location or position on the carrier. A sample containing a target nucleic acid and/or fragments thereof to be analyzed, for example DNA or RNA or cDNA, that has been labeled beforehand, is contacted with the DNA array/chip leading to the formation, through hybridization, of a duplex. After a washing step, analysis of the surface of the chip allows any hybridizations to be located by means of the signals emitted by the labeled target. A hybridization fingerprint results, which, by computer processing, allows retrieval of information such as the expression of genes, the presence of specific fragments in the sample, the determination of sequences and/or the identification of mutations.

In one embodiment of this invention, hybridization between target nucleic acids and nucleic acids of the invention, used in the form of probes and deposited or synthesized *in situ* on a DNA chip/array, can be determined by means of fluorescence, radioactivity, electronic detection or the like, as are well known in the art.

In another embodiment, the nucleotide sequences of the invention can be used in the form of a DNA array/chip to carry out analyses of the expression of genes involved in the production of AI-2 or in the AAR. This analysis is based on DNA array/chips on which probes, chosen for their specificity to characterize a given gene or nucleotide sequence, are present The target sequences to be analyzed are labeled before being hybridized onto the chip. After washing, the labeled complexes are detected and quantified, with the hybridizations being carried out at least in duplicate. Comparative analyses of the signal intensities obtained with respect to the same probe for different samples and/or for different probes with the same sample, allows, for example, for differential transcription of RNA derived from the sample.

In yet another embodiment, arrays/chips containing nucleotide sequences of the invention can comprise nucleotide sequences specific for other microorganisms, which allows for serial testing and rapid identification of the presence of a microorganism in a sample.

In a further embodiment, the principle of the DNA array/chip can also be used to produce protein arrays/chips on which the support has been coated with a polypeptide and/or an antibody of this invention, or arrays thereof, in place of the nucleic acid. These protein arrays/chips make it possible, for example, to analyze the biomolecular interactions induced by the affinity capture of targets onto a support coated, e.g., with proteins, by surface plasma resonance (SPR). The polypeptides or antibodies of this invention, capable of specifically binding antibodies or polypeptides derived from the sample to be analyzed, can be used in protein arrays/chips for the detection and/or identification of proteins and/or peptides in a sample.

Thus, the present invention provides a microarray or microchip comprising various nucleic acids of this invention in any combination, including repeats, as well as a microarray comprising various polypeptides of this invention in any combination, including repeats. Also provided is a microarray comprising one or more antibodies that specifically react with various polypeptides of this invention, in any combination, including repeats.

By "nucleic acid molecule" is intended DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA. A fragment of a nucleic acid molecule encoding an AI-2-related or AAR-related protein may encode a protein fragment that is biologically active, or it may be used as a hybridization probe or PCR primer as described below. A biologically active fragment of a polypeptide disclosed herein can be prepared by isolating a portion of one of the nucleotide sequences of the invention, expressing the encoded portion of the AI-2-related or AAR-related protein (e.g., by recombinant expression *in vitro*)*,* and assessing the activity of the encoded portion of the AI-2-related or AAR-related protein. Fragments of nucleic acid molecules encoding AI-2-related or AAR-related proteins comprise at least about 15, 20, 50, 75, 100, 200, 250, 300, 350, 400, 450, 500, 550, 600, 650, 700, 750, 800, 850, 900, 950, 1000, 1100, 1200, 1300, 1400, 1500, 1600 nucleotides or up to the total number of nucleotides present in a full-length AI-2-related or AAR-related nucleotide sequence as disclosed herein (for example, 693 for SEQ ID NO:1, 942 for SEQ ID NO:3, 1116 for SEQ ID NO:13, 471 for SEQ ID NO:15, etc.).

Fragments of amino acid sequences include polypeptide fragments suitable for use as immunogens to raise antibodies against polypeptides involved in the production of AI-2 or in the AAR. Fragments include peptides comprising amino acid sequences sufficiently identical to or derived from the amino acid sequence of an AI-2-related or AAR-related protein, or partial-length protein, of the invention and exhibiting at least one activity of an AI-2-related or AAR-related protein, but which include fewer amino acids than the full-length AI-2-related or AAR-related proteins disclosed herein. In one embodiment, the fragments may include polypeptides that are biologically active. A biologically active portion of a polypeptide is one that exhibits at least one activity (e.g., an activity associated with the production of AI-2 or the AAR) of the full-length protein of the invention. A biologically active portion of an AI-2-related or AAR-related protein can be a polypeptide that is, for example, 10, 25, 50, 100, 150, 200, 250, 300, 400, 500 contiguous amino acids in length, or up to the total number of amino acids present in a full-length AI-2-related or AAR-related protein of the current invention (for example, 231 for SEQ ID NO:2, 314 for SEQ ID NO:4, 372 for SEQ ID NO: 14, 157 for SEQ ID NO:16, etc.). Such biologically active portions can be prepared by recombinant techniques and evaluated for one or more of the functional activities of a native AI-2-related or AAR-related protein. As used here, a fragment comprises at least 5 contiguous amino acids of even SEQ ID NOS:2-22, 34, 36, or 38. The invention encompasses other fragments, however, such as any fragment in the protein greater than 6, 7, 8, 9, 10, 20, 50, 100, 200, 300, 400 or greater than 500 amino acids.

AAR-related sequences of the invention comprise SEQ ID NO:19 and 20 which encode a fibronectin binding protein; SEQ ID NO:33, 34, 35 and 36 which encode proteins involved in the production of exopolysaccharides; and SEQ ID NO:37 and 38 which encode a protein involved in D-alanine esterification of lipoteichoic acid and wall teichoic acid. In specific embodiments, a biologically active variant or fragment of SEQ ID NO:19 or 20 will continue to have binding activity to the intestinal mucosa, including binding to fibronectin, mucin or extracellular matrix molecules. Methods to assay for this activity are known. For example, fibronectin binding activity of the variant can be assayed using fluorescent microspheres coated with FN-120, which is a chymotryptic cell-binding fragment of plasma fibronectin. See, for example, Schultz and Armant (1995) J. Biol. Chem. 270(19):11522-31. A biologically active variant or fragment of SEQ ID NO:33, 34, 35, or 36 will continue to be involved in the production of exopolysaccharides. Exopolysaccharide production can be estimated by the phenol/sulphuric acid quantitative method described in Mozi, et al. (2001) J. Appl. Microbiol. 91(1):160-167. A biologically active variant or fragment of SEQ ID NO:37 or 38 will continue to be involved in the D-alanation of lipotechoic acid in bacteria. D-alanine uptake can be measured according to the methods described by O'Brien, et al. (1995) Microbios 83(335):119-137.

Other AAR-related sequences of the invention comprise enzymes involved in the production of AI-2, including SEQ ID NOS:1 and 2, which encode a nucleoside phosphorylase (*pfs*); SEQ ID NOS:3 and 4, which encode a SAM-dependent methyl transferase (SAM-MT); SEQ ID NOS:13 and 14, which encode a methionine adenosyltransferase (*metE*); SEQ ID NOS:15 and 16, which encode an S-ribosylhomocysteinase (luxS); and, SEQ ID NOS:21 and 22, which encode a methionine adenosyltransferase (*metK*). In specific embodiments, a biologically active variant or fragment of SEQ ID NOS:1, 2, 3, 4, 13,14, 15, 16, 21, or 22 will continue to have the enzymatic activity cited above, respectively. Methods to assay for activity for each of these enzymes are known. For example, metK activity can be measured by quantitating the production of S-adenosyltransferase using high performance liquid chromatography according to the methods described in Posnick and Samson (1999) J. Bacteriol. 181(21):6756-6762. The methyl donor activity of SAM-MT can be assayed according to the methods of Borchardt et al. (1974) J. Med. Chem. 19(9):1104-1110. MTA/SAH nucleoside activity can be measured by following the conversion of ¹⁴C-methylthioadenosine to ¹⁴C-methylthioribose, as described by Della-Ragione et al. (1995) Biochem. J. 232:335-341 and Cornell et al. (1996) Biochem. J. 317:285-290. MetE activity can be measured according to the assay described by Hondorp and Matthews (2004) PLoS Biol. 2(11):e336. LuxS activity can be measured using the *Vibrio harveyi* reporter assay as described elsewhere herein.

Variants of the nucleotide and amino acid sequences are encompassed in the present invention. By "variant" is intended a sufficiently identical sequence. Accordingly, the invention encompasses isolated nucleic acid molecules that are sufficiently identical to the nucleotide sequences encoding AI-2-related or AAR-related proteins in even SEQ ID NOS:2-22, 34, 36, or 38 or nucleic acid molecules that hybridize to a nucleic acid molecule of odd SEQ ID NOS:1-21, 33, 35, or 37, or a complement thereof, under stringent conditions. Variants also include variant polypeptides encoded by the nucleotide sequences of the present invention. In addition, polypeptides of the current invention have an amino acid sequence that is sufficiently identical to an amino acid sequence put forth in even SEQ ID NOS:2-22, 34, 36, or 38. By "sufficiently identical" is intended that one amino acid sequence or nucleotide sequence contains a sufficient or minimal number of equivalent or identical amino acid residues or nucleotides as compared to a second amino acid sequence or nucleotide sequence, thus providing a common structural domain and/or indicating a common functional activity. Conservative variants include those nucleotide sequences that differ due to the degeneracy of the genetic code.

In general, amino acid sequences or nucleotide sequences that have at least about 45%, 55%, or 65% identity, at least about 70% or 75% identity, at least about 80%, 85% or 95%, at least about 91%, 92%, 93%, 94%, 95%, 96%, 97%, 98%, 99%, or 99.5% sequence identity to any of the amino acid sequences of even SEQ ID NOS:2-22, 34, 36, or 38, or any of the nucleotide sequences of odd SEQ ID NOS:1-21, 33, 35, or 37, respectively, are defined herein as sufficiently identical. Variant proteins encompassed by the present invention are biologically active, that is they retain the desired biological activity of the native protein. A biologically active variant of a protein of the invention may differ from that protein by as few as 1-15 amino acid residues, as few as 1-10, such as 6-10, as few as 5, as few as 4, 3, 2, or even 1 amino acid residue.

Naturally occurring variants may exist within a population (e.g., the lactic acid bacteria population). Such variants can be identified by using well-known molecular biology techniques, such as the polymerase chain reaction (PCR), and hybridization as described below. Synthetically derived nucleotide sequences, for example, sequences generated by site-directed mutagenesis or PCR-mediated mutagenesis that still encode an AI-2-related or AAR-related protein, are also included as variants. One or more nucleotide or amino acid substitutions, additions, or deletions can be introduced into a nucleotide or amino acid sequence disclosed herein, such that the substitutions, additions, or deletions are introduced into the encoded protein. The additions (insertions) or deletions (truncations) may be made at the N-terminal or C-terminal end of the native protein, or at one or more sites in the native protein. Similarly, a substitution of one or more nucleotides or amino acids may be made at one or more sites in the native protein.

For example, conservative amino acid substitutions may be made at one or more predicted, preferably nonessential amino acid residues. A "nonessential" amino acid residue is a residue that can be altered from the wild-type sequence of a protein without altering the biological activity, whereas an "essential" amino acid is required for biological activity. A "conservative amino acid substitution" is one in which the amino acid residue is replaced with an amino acid residue with a similar side chain. Families of amino acid residues having similar side chains are known in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e,g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Such substitutions would not be made for conserved amino acid residues, or for amino acid residues residing within a conserved motif, where such residues are essential for protein activity.

Alternatively, mutations can be made randomly along all or part of the length of the AI-2-related or AAR-related coding sequence, such as by saturation mutagenesis. The mutants can be expressed recombinantly, and screened for those that retain biological activity by assaying for AI-2-related or AAR-related protective activity using standard assay techniques. Methods for mutagenesis and nucleotide sequence alterations are known in the art. See, for example, Kunkel (1985) Proc. Natl. Acad. Sci. USA 82:488-492; Kunkel et al. (1987) Methods in Enzymol. Molecular Biology (MacMillan Publishing Company, New York) and the references sited therein. The mutations made in the DNA encoding the variant should not disrupt the reading frame and preferably will not create complementary regions that could produce secondary mRNA structure. See, EP Patent Application Publication No. 75,444. Guidance as to appropriate amino acid substitutions that do not affect biological activity of the protein of interest may be found in the model of Dayhoff et al. (1978) Atlas of protein Sequence and Structure (Natl. Biomed. Res. Found., Washington, D.C.), herein incorporated by reference.

The deletions, insertions, and substitutions of the protein sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays. That is, the activity can be evaluated by comparing the activity of the modified sequence with the activity of the original sequence. For example, the activity of variants and fragments of polypeptides involved in the production of AI-2 or in the AAR can be measured by assaying for the production of AI-2 using methods disclosed elsewhere herein.

Variant nucleotide and amino acid sequences of the present invention also encompass sequences derived from mutagenic and recombinogenic procedures such as DNA shuffling. With such a procedure, one or more different AI-2-related or AAR-related protein coding regions can be used to create a new AI-2-related or AAR related protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between the AI-2-related or AAR-related gene of the invention and other known AI-2-related or AAR-related genes to obtain a new gene coding for a protein with an improved property of interest, such as an increased Kₘ in the case of an enzyme. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

Variants of an AI-2-related or AAR-related protein of the invention can be identified by screening combinatorial libraries of mutants, e.g., truncation mutants, of an AI-2-related or AAR-related protein. In one embodiment, a variegated library of AI-2-related or AAR-related variants is generated by combinatorial mutagenesis at the nucleic acid level and is encoded by a variegated gene library. A variegated library of AI-2-related or AAR-related variants can be produced by, for example, enzymatically ligating a mixture of synthetic oligonucleotides into gene sequences such that a degenerate set of potential AI-2-related or AAR-related sequences is expressible as individual polypeptides, or alternatively, as a set of larger fusion proteins (e.g., for phage display) containing the set of AI-2-related or AAR-related sequences therein. There are a variety of methods that can be used to produce libraries of potential AI-2-related or AAR-related variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential AI-2-related or AAR-related sequences. Methods for synthesizing degenerate oligonucleotides are known in the art (see, e.g., Narang (1983) Tetrahedron 39:3; Itakura et al. (1984) Annu. Rev. Biochem. 53:323; Itakura et al. (1984) Science 198:1056; Ike et al. (1983) Nucleic Acids Res. 11:477).

In addition, libraries of fragments of an AI-2-related or AAR-related protein coding sequence can be used to generate a variegated population of AI-2-related or AAR-related fragments for screening and subsequent selection of variants of an AI-2-related or AAR-related protein. In one embodiment, a library of coding sequence fragments can be generated by treating a double-stranded PCR fragment of an AI-2-related or AAR-related coding sequence with a nuclease under conditions wherein nicking occurs only about once per molecule, denaturing the double-stranded DNA, renaturing the DNA to form double-stranded DNA which can include sense/antisense pairs from different nicked products, removing single-stranded portions from reformed duplexes by treatment with S1 nuclease, and ligating the resulting fragment library into an expression vector. By this method, one can derive an expression library that encodes N-terminal and internal fragments of various sizes of the AI-2-related or AAR-related protein.

Several techniques are known in the art for screening gene products of combinatorial libraries made by point mutations or truncation and for screening cDNA libraries for gene products having a selected property. Such techniques are adaptable for rapid screening of the gene libraries generated by the combinatorial mutagenesis of AI-2-related or AAR-related proteins. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique that enhances the frequency of functional mutants in the libraries, can be used in combination with the screening assays to identify AI-2-related or AAR-related variants (Arkin and Yourvan (1992) Proc. Natl. Acad. Sci. USA 89:7811-7815; Delgrave et al. (1993) Protein Engineering 6(3):327-331).

### ii. Sequence Identity

The AI-2-related or AAR-related sequences are members of a family of molecules with conserved functional features. By "family" is intended two or more proteins or nucleic acid molecules having sufficient nucleotide sequence or amino acid sequence identity. A family that contains deeply divergent groups may be divided into subfamilies. A clan is a group of families that are thought to have common ancestry. Members of a clan often have a similar tertiary structure. By "sequence identity" is intended the nucleotide or amino acid residues that are the same when aligning two sequences for maximum correspondence over at least one specified comparison window. By "comparison window" is intended a contiguous segment of the two nucleotide sequences or amino acid sequences for optimal alignment, wherein the second sequence may contain additions or deletions (i.e., gaps) as compared to the first sequence. Generally, for nucleic acid alignments, the comparison window is at least 20 contiguous nucleotides in length, and optionally can be 30, 40, 50, 100, or longer. For amino acid sequence alignments, the comparison window is at least 6 contiguous amino acids in length, and optionally can be 10, 15, 20, 30, or longer. Those of skill in the art understand that to avoid a high similarity due to inclusion of gaps, a gap penalty is typically introduced and is subtracted from the number of matches.

Family members may be from the same or different species, and can include homologues as well as distinct proteins. Often, members of a family display common functional characteristics. Homologues can be isolated based on their identity to the AI-2-related or AAR-related nucleic acid sequences disclosed herein using the cDNA, or a portion thereof, as a hybridization probe according to standard hybridization techniques under stringent hybridization conditions as disclosed below.

To determine the percent identity of two amino acid or nucleotide sequences, an alignment is performed. Percent identity of the two sequences is a function of the number of identical residues shared by the two sequences in the comparison window (i.e., percent identity = number of identical residues/total number of residues x 100). In one embodiment, the sequences are the same length. Methods similar to those mentioned below can be used to determine the percent identity between two sequences. The methods can be used with or without allowing gaps. Alignment may also be performed manually by inspection.

When amino acid sequences differ in conservative substitutions, the percent identity may be adjusted upward to correct for the conservative nature of the substitution. Means for making this adjustment are known in the art. Typically the conservative substitution is scored as a partial, rather than a full mismatch, thereby increasing the percentage sequence identity.

Mathematical algorithms can be used to determine the percent identity of two sequences. Non-limiting examples of mathematical algorithms are the algorithm of Karlin and Altschul (1990) Proc. Natl. Acad. Sci. USA 87:2264, modified as in Karlin and Altschul (1993) Proc. Natl. Acad. Sci. USA 90:5873-5877; the algorithm of Myers and Miller (1988) CABIOS 4:11-17; the local alignment algorithm of Smith et al. (1981) Adv. Appl. Math. 2:482; the global alignment algorithm of Needleman and Wunsch (1970) J. Mol. Biol. 48:443-453; and the search-for-local alignment-method of Pearson and Lipman (1988) Proc. Natl. Acad. Sci. USA 85:2444-2448.

Various computer implementations based on these mathematical algorithms have been designed to enable the determination of sequence identity. The BLAST programs of Altschul et al. (1990) J. Mol. Biol. 215:403 are based on the algorithm of Karlin and Altschul (1990) *supra.* Searches to obtain nucleotide sequences that are homologous to nucleotide sequences of the present invention can be performed with the BLASTN program, score = 100, wordlength = 12. To obtain amino acid sequences homologous to sequences encoding a protein or polypeptide of the current invention, the BLASTX program may be used, score = 50, wordlength = 3. Gapped alignments may be obtained by using Gapped BLAST (in BLAST 2.0) as described in Altschul et al. (1997) Nucleic Acids Res. 25:3389. To detect distant relationships between molecules, PSI-BLAST can be used. See Altschul *et al.* (1997) *supra.* For all of the BLAST programs, the default parameters of the respective programs can be used. See www.ncbi.nlm.nih.gov. Alignment may also be performed manually by inspection.

Another program that can be used to determine percent sequence identity is the ALIGN program (version 2.0), which uses the mathematical algorithm of Myers and Miller (1988) *supra.* A PAM120 weight residue table, a gap length penalty of 12, and a gap penalty of 4 can be used with this program when comparing amino acid sequences.

In addition to the ALIGN and BLAST programs, the BESTFIT, GAP, FASTA and TFASTA programs are part of the GCG Wisconsin Genetics Software Package, Version 10 (available from Accelrys Inc., 9685 Scranton Rd., San Diego, California, USA), and can be used for performing sequence alignments. The preferred program is GAP version 10, which used the algorithm of Needleman and Wunsch (1970) *supra.* Unless otherwise stated the sequence identity similarity values provided herein refer to the value obtained using GAP Version 10 with the following parameters: % identity and % similarity for a nucleotide sequence using GAP Weight of 50 and Length Weight of 3 and the nwsgapdna.cmp scoring matrix; % identity and % similarity for an amino acid sequence using GAP Weight of 8 and Length Weight of 2, and the BLOSUM62 scoring matrix; or any equivalent program. By "equivalent program" is intended any sequence comparison program that, for any two sequences in question, generates an alignment having identical nucleotide or amino acid residue matches and an identical percent sequence identity when compared to the corresponding alignment generated by GAP Version 10.

Alignment of a sequence in a database to a queried sequence produced by BLASTN, FASTA, BLASTP or like algorithm is commonly described as a "hit." Hits to one or more database sequences by a queried sequence produced by BLASTN, FASTA, BLASTP or a similar algorithm, align and identify similar portions of a sequence. A hit to a database sequence generally represents an overlap over a fraction of the sequence length of the queried sequence, i.e., a portion or fragment of the queried sequence. However, the overlap can represent the entire length of the queried sequence. The hits in an alignment to a queried sequence produced by BLASTN, FASTA, or BLASTP algorithms to sequences in a database are commonly arranged in order of the degree of similarity and the length of sequence overlap.

Polynucleotide and polypeptide hits aligned by BLASTN, FASTA, or BLASTP algorithms to a queried sequence produce "Expect" values. The Expect value (E value) indicates the number of hits one can "expect" to see over a certain number of contiguous sequences at random when searching a database of a certain size. The Expect value is used as a significance threshold for determining whether the hit to a database, such as the GENBANK® or the EMBL database, indicates actual similarity. For example, an E value of 0.1 assigned to a polynucleotide hit is interpreted as meaning that in a database of the size of the GENBANK® database, one might expect to see 0.1 matches over the aligned portion of the sequence with a similar score randomly. By this criterion, the aligned and matched portions of the polynucleotide sequences then have a probability of 95% of being the same. For sequences having an E value of 0.01 or less over aligned and matched portions, the probability of finding a match randomly in the GENBANK® database is 1% or less, using the BLASTN or FASTA algorithm.

According to an embodiment of this invention, "variant" polynucleotides and polypeptides of this invention, comprise sequences producing an E value of about 0.01 or less when compared to the polynucleotide or polypeptide sequences of the present invention. That is, a variant polynucleotide or polypeptide is any sequence that has at least a 99% probability of being the same as the polynucleotide or polypeptide of the present invention, measured as having an E value of 0.01 or less using the BLASTN, FASTA, or BLASTP algorithms set at parameters described herein. In other embodiments, a variant polynucleotide is a sequence having the same number of, or fewer nucleic acids than a polynucleotide of the present invention that has at least a 99% probability of being the same as the polynucleotide of the present invention, measured as having an E value of 0.01 or less using the BLASTN or FASTA algorithms set at parameters described herein. Similarly, a variant polypeptide is a sequence having the same number of, or fewer amino acids than a polypeptide of the present invention that has at least a 99% probability of being the same as a polypeptide of the present invention, measured as having an E value of 0.01 or less using the BLASTP algorithm set at the parameters described herein.

As noted above, the percentage identity is determined by aligning sequences using one of the BLASTN, FASTA, or BLASTP algorithms, set at the running parameters described herein, and identifying the number of identical nucleic acids or amino acids over the aligned portions; dividing the number of identical nucleic acids or amino acids by the total number of nucleic acids or amino acids of the polynucleotide or polypeptide sequence of the present invention; and then multiplying by 100 to determine the percent identity. For example, a polynucleotide of the present invention having 220 nucleic acids has a hit to a polynucleotide sequence in the GENBANK® database having 520 nucleic acids over a stretch of 23 nucleotides in the alignment produced by the BLASTN algorithm using the parameters described herein. The 23 nucleotide hit includes 21 identical nucleotides, one gap and one different nucleotide. The percent identity of the polynucleotide of the present invention to the hit in the GENBANK® library is thus 21/220 times 100, or 9.5%. The polynucleotide sequence in the GENBANK® database is thus not a variant of a polynucleotide of the present invention.

### iii. Identification and Isolation of Homologous Sequences

AI-2-related or AAR-related nucleotide sequences identified based on their sequence identity to the AI-2-related or AAR-related nucleotide sequences set forth herein or to fragments and variants thereof are encompassed by the present invention. Methods such as PCR or hybridization can be used to identify sequences from a cDNA or genomic library, for example, which are substantially identical to a sequence of this invention. *See,* for example, Sambrook et al. (1989) Molecular Cloning: Laboratory Manual (2d ed., Cold Spring Harbor Laboratory Press, Cold Spring Harbor, New York) and Innis, et al. (1990) PCR Protocols: A Guide to Methods and Applications (Academic Press, New York). Methods for construction of such cDNA and genomic libraries are generally known in the art and are also disclosed in the above reference.

In hybridization techniques, the hybridization probes may be genomic DNA fragments, cDNA fragments, RNA fragments, or other oligonucleotides, and may consist of all or part of a known nucleotide sequence disclosed herein. In addition, they may be labeled with a detectable group such as ³²P, or any other detectable marker, such as other radioisotopes, a fluorescent compound, an enzyme, or an enzyme co-factor. Probes for hybridization can be made by labeling synthetic oligonucleotides, based on the known AI-2-related or AAR-related nucleotide sequences disclosed herein. Degenerate primers designed on the basis of conserved nucleotides or amino acid residues in a known AI-2-related or AAR-related nucleotide sequence or encoded amino acid sequence can additionally be used. The hybridization probe typically comprises a region of nucleotide sequence that hybridizes under stringent conditions to at least about 10, preferably about 20, more preferably about 50, 75, 100, 125, 150, 175, 200, 250, 300, 350, or 400 consecutive nucleotides of an AI-2-related or AAR-related nucleotide sequence of the invention or a fragment or variant thereof. To achieve specific hybridization under a variety of conditions, such probes include sequences that are unique among AI-2-related or AAR-related protein sequences. Methods for the preparation of probes for hybridization are generally known in the art and are disclosed in Sambrook et al. (1989) Molecular Cloning: A Laboratory Manual (2d ed., Cold Spring Harbor Laboratory Press, Cold Spring Harbor, New York), herein incorporated by reference.

In one embodiment, the entire nucleotide sequence encoding an AI-2-related or AAR-related protein is used as a probe to identify novel AI-2-related or AAR-related sequences and messenger RNAs. In another embodiment, the probe is a fragment of a nucleotide sequence disclosed herein. In some embodiments, the nucleotide sequence that hybridizes under stringent conditions to the probe can be at least about 300, 325, 350, 375, 400, 425, 450, 500, 550, 600, 650, 700, 800, 900, 1000, 1500 or more nucleotides in length.

Accordingly, in addition to the AI-2-related or AAR-related nucleotide sequences disclosed herein, and fragments and variants thereof, the isolated nucleic acid molecules of the current invention also encompass homologous DNA sequences identified and isolated from other organisms or cells by hybridization with entire or partial sequences obtained from the AI-2-related or AAR-related nucleotide sequences disclosed herein, or variants and fragments thereof.

Substantially identical sequences will hybridize to each other under stringent conditions. By "stringent conditions" is intended conditions under which a probe will hybridize to its target sequence to a detectably greater degree than to other sequences (e.g., at least 2-fold over background). Generally, stringent conditions encompass those, conditions for hybridization and washing under which nucleotides having at least about 60%, 65%, 70%, or at least about 75% sequence identity typically remain hybridized to each other. Stringent conditions are known in the art and can be found in Ausubel et al., eds. (1989) Current Protocols in Molecular Biology (John Wiley & Sons, New York). Hybridization typically occurs for less than about 24 hours, usually about 4 to about 12 hours.

Stringent conditions are sequence dependent and will differ in different circumstances. Full-length or partial nucleic acid sequences may be used to obtain homologues and orthologs encompassed by the present invention. By "orthologs" is intended genes derived from a common ancestral gene and which are found in different species as a result of speciation. Genes found in different species are considered orthologs when their nucleotide sequences and/or their encoded protein sequences share substantial identity as defined elsewhere herein. Functions of orthologs are often highly conserved among species.

When using probes, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides).

The post-hybridization washes are instrumental in controlling specificity. The two critical factors are ionic strength and temperature of the final wash solution. For the detection of sequences that hybridize to a full-length or approximately full-length target sequence, the temperature under stringent conditions is selected to be about 5°C lower than the thermal melting point (Tₘ) for the specific sequence at a defined ionic strength and pH. However, stringent conditions would encompass temperatures in the range of 1°C to 20°C lower than the Tₘ, depending on the desired degree of stringency as otherwise qualified herein. For DNA-DNA hybrids, the Tₘ can be determined using the equation of Meinkoth and Wahl (1984) Anal. Biochem. 138:267-284: Tₘ =81.5°C + 16.6 (logM) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe.

The ability to detect sequences with varying degrees of homology can be obtained by varying the stringency of the hybridization and/or washing conditions. To target sequences that are 100% identical (homologous probing), stringency conditions must be obtained that do not allow mismatching. By allowing mismatching of nucleotide residues to occur, sequences with a lower degree of similarity can be detected (heterologous probing). For every 1% of mismatching, the Tₘ is reduced about 1°C; therefore, hybridization and/or wash conditions can be manipulated to allow hybridization of sequences of a target percentage identity. For example, if sequences with ≥95% sequence identity are preferred, the Tₘ can be decreased by 10°C. Two nucleotide sequences could be substantially identical, but fail to hybridize to each other under stringent conditions, if the polypeptides they encode are substantially identical. This situation could arise, for example, if the maximum codon degeneracy of the genetic code is used to create a copy of a nucleic acid.

Exemplary low stringency conditions include hybridization with a buffer solution of 30-35% formamide, 1 M NaCl, 1% SDS (sodium dodecyl sulfate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl,1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours. An extensive guide to the hybridization of nucleic acids is found in Tij ssen (1993) Laboratory Techniques in Biochemistry and Molecular Biology - Hybridization with Nucleic Acid Probes, Part I, Chapter 2 (Elsevier, New York); and Ausubel et al., eds. (1995) Current Protocols in Molecular- Biology, Chapter 2 (Greene Publishing and Wiley-Interscience, New York). See Sambrook et al. (1989) Molecular Cloning: A Laboratory Manual (2d ed.; Cold Spring Harbor Laboratory Press, Cold Spring Harbor, New York).

In a PCR approach, oligonucleotide primers can be designed for use in PCR reactions to amplify corresponding DNA sequences from cDNA or genomic DNA extracted from any organism of interest. PCR primers are preferably at least about 10 nucleotides in length, and most preferably at least about 20 nucleotides in length. Methods for designing PCR primers and PCR cloning are generally known in the art and are disclosed in Sambrook et al. (1989) Molecular Cloning: A Laboratory Manual (2d ed., Cold Spring Harbor Laboratory Press, Cold Spring Harbor, New York). See also Innis et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

### iv. Antisense Nucleotide Sequences

The present invention also encompasses antisense nucleic acid molecules, i.e., molecules that are complementary to a sense nucleic acid encoding a protein, e.g., complementary to the coding strand of a double-stranded cDNA molecule, or complementary to an mRNA sequence. Accordingly, an antisense nucleic acid can hydrogen bond to a sense nucleic acid. The antisense nucleic acid can be complementary to an entire AI-2-related or AAR-related coding strand, or to only a portion thereof, e.g., all or part of the protein coding region (or open reading frame). An antisense nucleic acid molecule can be antisense to a noncoding region of the coding strand of a nucleotide sequence encoding an AI-2-related or AAR-related protein. The noncoding regions are the 5' and 3' sequences that flank the coding region and are not translated into amino acids. Antisense nucleotide sequences are useful in disrupting the expression of the target gene. Antisense constructions having 70%, 80%, or 85% sequence identity to the corresponding sequence may be used.

Given the coding-strand sequence encoding an AI-2-related or AAR-related protein disclosed herein (e.g., odd SEQ ID NOS:1-21, 33, 35, or 37), antisense nucleic acids of the invention can be designed according to the rules of Watson and Crick base pairing. The antisense nucleic acid molecule can be complementary to the entire coding region of AI-2-related or AAR-related mRNA, but more preferably is an oligonucleotide that is antisense to only a portion of the coding or noncoding region of AI-2-related or AAR-related mRNA. An antisense oligonucleotide can be, for example, about 5, 10, 15, 20, 25, 30, 35, 40, 45, or 50 nucleotides in length, or it can be 100, 200 nucleotides, or greater in length. An antisense nucleic acid of the invention can be constructed using chemical synthesis and enzymatic ligation procedures known in the art.

An antisense nucleic acid molecule of the invention can be an α-anomeric nucleic acid molecule (Gaultier et al. (1987) Nucleic Acids Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330). The invention also encompasses ribozymes, which are catalytic RNA molecules with ribonuclease activity that are capable of cleaving a single-stranded nucleic acid, such as an mRNA, to which they have a complementary region. The invention also encompasses nucleic acid molecules that form triple helical structures. See generally Helene (1991) Anticancer Drug Des. 6(6):569; Helene (1992) Ann. N.Y. Acad. Sci. 660:27; and Maher (1992) Bioassays 14(12):807.

In some embodiments, the nucleic acid molecules of the invention can be modified at the base moiety, sugar moiety, or phosphate backbone to improve, e.g., the stability, hybridization, or solubility of the molecule. As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid-phase peptide synthesis protocols as described, for example, in Hyrup *et al.* (1996) *supra*; Perry-O'Keefe et al. (1996) Proc. Natl. Acad. Sci. USA 93:14670.

In another embodiment, PNAs of an AI-2-related or AAR-related molecule can be modified, e.g., to enhance their stability, specificity, or cellular uptake, by attaching lipophilic or other helper groups to PNA, by the formation of PNA-DNA chimeras, or by the use of liposomes or other techniques of drug delivery known in the art. The synthesis of PNA-DNA chimeras can be performed as described in Hyrup (1996) *supra*; Finn et al. (1996) Nucleic Acids Res. 24(17):3357-63; Mag et al. (1989) Nucleic Acids Res. 17:5973; and Peterson et al. (1975) Bioorganic Med. Chem. Lett. 5:1119.

### v. Fusion Proteins

The invention also includes AI-2-related or AAR related chimeric or fusion proteins. An AI-2-related or AAR-related "chimeric protein" or "fusion protein" comprises an AI-2-related or AAR-related polypeptide operably linked to a non-AI-2-related or AAR-related polypeptide. A "AI-2-related" or "AAR-related polypeptide" refers to a polypeptide having an amino acid sequence corresponding to a polypeptide that is involved in the production of AI-2 or in the AAR, whereas a "non-AI-2-related" or "non-AAR-related polypeptide" refers to a polypeptide having an amino acid sequence corresponding to a protein that is not substantially identical to a polypeptide that is involved in the production of AI-2 or in the AAR, and which is derived from the same or a different organism. Within an AI-2-related or AAR-related fusion protein, the AI-2-related or AAR-related polypeptide can correspond to all or a portion of an AI-2-related or AAR-related protein, preferably including at least one biologically active portion of an AI-2-related or AAR-related protein. Within the fusion protein, the term "operably linked" is intended to indicate that the AI-2-related or AAR-related polypeptide and the non-AI-2-related or AAR-related polypeptide are fused in-frame to each other. The non-AI-2-related or AAR-related polypeptide can be fused to the N-terminus or C-terminus of the AI-2-related or AAR-related polypeptide.

Expression of the linked coding sequences results in two linked heterologous amino acid sequences that form the fusion protein. The carrier sequence (the non-AI-2-related or AAR-related polypeptide) can encode a carrier polypeptide that potentiates or increases expression of the fusion protein in the bacterial host. The portion of the fusion protein encoded by the carrier sequence, i.e., the carrier polypeptide, may be a protein fragment, an entire functional moiety, or an entire protein sequence. The carrier region or polypeptide may additionally be designed to be used in purifying the fusion protein, either with antibodies or with affinity purification specific for that carrier polypeptide. Likewise, physical properties of the carrier polypeptide can be exploited to allow selective purification of the fusion protein.

Particular carrier polypeptides of interest include superoxide dismutase (SOD), maltose-binding protein (MBP), glutathione-S-transferase (GST), an N-terminal histidine (His) tag, and the like. This list is not intended to be limiting, as any carrier polypeptide that potentiates expression of the AI-2-related or AAR-related protein as a fusion protein can be used in the methods of the invention.

In one embodiment, the fusion protein is a GST-AI-2-related or AAR-related fusion protein in which the AI-2-related or AAR-related sequences are fused to the C-terminus of the GST sequences. In another embodiment, the fusion protein is an AI-2-related or AAR-related-immunoglobulin fusion protein in which all or part of an AI-2-related or AAR-related protein is fused to sequences derived from a member of the immunoglobulin protein family. The AI-2-related or AAR-related-immunoglobulin fusion proteins of the invention can be used as immunogens to produce anti-AI-2-related or AAR-related antibodies in a subject, to purify AI-2-related or AAR-related ligands, and in screening assays to identify molecules that inhibit the interaction of an AI-2-related or AAR-related protein with an AI-2-related or AAR-related ligand.

One of skill in the art will recognize that the particular carrier polypeptide is chosen with the purification scheme in mind. For example, His tags, GST, and maltose-binding protein represent carrier polypeptides that have readily available affinity columns to which they can be bound and eluted Thus, where the carrier polypeptide is an N-terminal His tag such as hexahistidine (His₆ tag), the AI-2-related or AAR-related fusion protein can be purified using a matrix comprising a metal-chelating resin, for example, nickel nitrilotriacetic acid (Ni-NTA), nickel iminodiacetic acid (Ni-IDA), and cobalt-containing resin (Co-resin). See, for example, Steinert et al. (1997) QIAGEN News 4:11-15, herein incorporated by reference in its entirety. Where the carrier polypeptide is GST, the AI-2-related or AAR-related fusion protein can be purified using a matrix comprising glutathione-agarose beads (Sigma or Pharmacia Biotech); where the carrier polypeptide is a maltose-binding protein (MBP), the AI-2-related or AAR-related fusion protein can be purified using a matrix comprising an agarose resin derivatized with amylose.

Preferably, a chimeric or fusion protein of the invention is produced by standard recombinant DNA techniques. For example, DNA fragments coding for the different polypeptide sequences may be ligated together in-frame, or the fusion gene can be synthesized, such as with automated DNA synthesizers. Alternatively, PCR amplification of gene fragments can be carried out using anchor primers that give rise to complementary overhangs between two consecutive gene fragments, which can subsequently be annealed and re-amplified to generate a chimeric gene sequence (see, e.g., Ausubel et al., eds. (1995) Current Protocols in Molecular Biology (Greene Publishing and Wiley-Interscience, New York). Moreover, an AI-2-related or AAR-related-encoding nucleic acid can be cloned into a commercially available expression vector such that it is linked in-frame to an existing fusion moiety.

The fusion protein expression vector is typically designed for ease of removing the carrier polypeptide to allow the AI-2-related or AAR-related protein to retain the native biological activity associated with it. Methods for cleavage of fusion proteins are known in the art. See, for example, Ausubel et al., eds. (1998) Current Protocols in Molecular Biology (John Wiley & Sons, Inc.). Chemical cleavage of the fusion protein can be accomplished with reagents such as cyanogen bromide, 2-(2-nitrophenylsulfenyl)-3-methyl-3'-bromoindolenine, hydroxylamine, or low pH. Chemical cleavage is often accomplished under denaturing conditions to cleave otherwise insoluble fusion proteins.

Where separation of the AI-2-related or AAR-related polypeptide from the carrier polypeptide is desired and a cleavage site at the junction between these fused polypeptides is not naturally occurring, the fusion construct can be designed to contain a specific protease cleavage site to facilitate enzymatic cleavage and removal of the carrier polypeptide. In this manner, a linker sequence comprising a coding sequence for a peptide that has a cleavage site specific for an enzyme of interest can be fused in-frame between the coding sequence for the carrier polypeptide (for example, MBP, GST, SOD, or an N-terminal His tag) and the coding sequence for the AI-2-related or AAR-related polypeptide. Suitable enzymes having specificity for cleavage sites include, but are not limited to, factor Xa, thrombin, enterokinase, remin, collagenase, and tobacco etch virus (TEV) protease. Cleavage sites for these enzymes are well known in the art. Thus, for example, where factor Xa is to be used to cleave the carrier polypeptide from the AI-2-related or AAR-related polypeptide, the fusion construct can be designed to comprise a linker sequence encoding a factor Xa-sensitive cleavage site, for example, the sequence IEGR (see, for example, Nagai and Thøgersen (1984) Nature 309:810-812, Nagai and Thøgersen (1987) Meth. Enzymol. 153:461-481, and Pryor and Leiting (1997) Protein Expr. Purif. 10(3):309-319, herein incorporated by reference). Where thrombin is to be used to cleave the carrier polypeptide from the AI-2-related or AAR-related polypeptide, the fusion construct can be designed to comprise a linker sequence encoding a thrombin-sensitive cleavage site, for example the sequence LVPRGS or VIAGR (see, for example, Pryor and Leiting (1997) Protein Expr. Purif. 10(3):309-319, and Hong et al. (1997) Chin. Med. Sci. J. 12(3):143-147, respectively, herein incorporated by reference). Cleavage sites for TEV protease are known in the art. See, for example, the cleavage sites described in U.S. Patent No. 5,532,142, herein incorporated by reference in its entirety. See also the discussion in Ausubel et al., eds. (1998) Current Protocols in Molecular Biology (John Wiley & Sons, Inc.), Chapter 16.

### vi. Antibodies

An isolated polypeptide of the present invention can be used as an immunogen to generate antibodies that specifically bind AI-2-related or AAR-related proteins, or stimulate production of antibodies against AI-2 related or AAR-related polypeptides *in vivo.* The full-length AI-2-related or AAR-related protein can be used as an immunogen or, alternatively, antigenic peptide fragments of AI-2-related or AAR-related proteins as described herein can be used. The antigenic peptide of an AI-2-related or AAR-related protein comprises at least 8, preferably 10, 15, 20, or 30 amino acid residues of the amino acid sequence shown in even SEQ ID NOS:2-22, 34; 36 or 38 and encompasses an epitope of an AI-2-related or AAR-related protein such that an antibody raised against the peptide forms a specific immune complex with the AI-2-related or AAR-related protein. Preferred epitopes encompassed by the antigenic peptide are regions of an AI-2-related or AAR-related protein that are located on the surface of the protein, e.g., hydrophilic regions.

### vii. Assays

Diagnostic assays to detect expression of the disclosed polypeptides and/or nucleic acid molecules as well as their disclosed activity in a sample are disclosed. An exemplary method for detecting the presence or absence of a disclosed nucleic acid or protein comprising the disclosed polypeptide in a sample involves obtaining a sample from a food/dairy/feed product, starter culture (mother, seed, bulk/set, concentrated, dried, lyophilized, frozen), cultured food/dairy/feed product, dietary supplement, bioprocessing fermentate, or a subject that has ingested a probiotic material, and contacting the sample with a compound or an agent capable of detecting the disclosed polypeptides or nucleic acids (e.g., an mRNA or genomic DNA comprising the disclosed nucleic acid or fragment thereof) such that the presence of the disclosed sequence is detected in the sample. Results obtained with a sample from the food, supplement, culture, product, or subject may be compared to results obtained with a sample from a control culture, product, or subject.

One agent for detecting the mRNA or genomic DNA comprising a disclosed nucleotide sequence is a labeled nucleic acid probe capable of hybridizing to the disclosed nucleotide sequence of the mRNA or genomic DNA. The nucleic acid probe can be, for example, a disclosed nucleic acid molecule, such as the nucleic acid of odd SEQ ID NOS:1-21, 33, 35, 37, or a portion thereof, such as a nucleic acid molecule of at least 15, 30, 50, 100, 200, 300, 400, 500, 600, 700, 800, 900, 1000, 1500 or more nucleotides in length and sufficient to specifically hybridize under stringent conditions to the mRNA or genomic DNA comprising the disclosed nucleic acid sequence. Other suitable probes for use in the diagnostic assays of the invention are described herein.

One agent for detecting a protein comprising a disclosed polypeptide sequence is an antibody capable of binding to the disclosed polypeptide, preferably an antibody with a detectable label. Antibodies can be polyclonal, or more preferably, monoclonal. An intact antibody, or a fragment thereof (e.g., Fab or F(abN)₂) can be used. The term "labeled," with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

The term "sample" is intended to include tissues, cells, and biological fluids present in or isolated from a subject, as well as cells from starter cultures or food products carrying such cultures, or derived from the use of such cultures. That is, the detection method of the invention can be used to detect mRNA, protein, or genomic DNA comprising a disclosed sequence in a sample both *in vitro* and *in vivo. In vitro* techniques for detection of mRNA comprising a disclosed sequence include Northern hybridizations *and in situ* hybridizations. *In vitro* techniques for detection of a protein comprising a disclosed polypeptide include enzyme linked immunosorbent assays (ELISAs), Western blots, immunoprecipitations, and immunofluorescence. *In vitro* techniques for detection of genomic DNA comprising the disclosed nucleotide sequences include Southern hybridizations. Furthermore, *in vivo* techniques for detection of a protein comprising a disclosed polypeptide include introducing into a subject a labeled antibody against the disclosed polypeptide. For example, the antibody can be labeled with a radioactive marker whose presence and location in a subject can be detected by standard imaging techniques.

In one embodiment, the sample contains protein molecules from a test subject that has consumed a probiotic material. Alternatively, the sample can contain mRNA or genomic DNA from a starter culture.

The invention also encompasses kits for detecting the presence of disclosed nucleic acids or proteins comprising disclosed polypeptides in a sample. Such kits can be used to determine if a microbe expressing a specific polypeptide of the invention is present in a food product or starter culture, or in a subject that has consumed a probiotic material. For example, the kit can comprise a labeled compound or agent capable of detecting a disclosed polypeptide or mRNA in a sample and means for determining the amount of the disclosed polypeptide in the sample (e.g., an antibody that recognizes the disclosed polypeptide or an oligonucleotide probe that binds to DNA encoding a disclosed polypeptide, e.g., odd SEQ ID NOS:1-21, 33, 35, or 37). Kits can also include instructions detailing the use of such compounds.

For antibody-based kits, the kit can comprise, for example: (1) a first antibody (e.g., attached to a solid support) that binds to a disclosed polypeptide; and, optionally, (2) a second, different antibody that binds to the disclosed polypeptide or the first antibody and is conjugated to a detectable agent. For oligonucleotide-based kits, the kit can comprise, for example: (1) an oligonucleotide, e.g., a detectably labeled oligonucleotide, that hybridizes to a disclosed nucleic acid sequence or (2) a pair of primers useful for amplifying a disclosed nucleic acid molecule.

The kit can also comprise, e.g., a buffering agent, a preservative, or a protein stabilizing agent. The kit can also comprise components necessary for detecting the detectable agent (e.g., an enzyme or a substrate). The kit can also contain a control sample or a series of control samples that can be assayed and compared to the test sample contained. Each component of the kit is usually enclosed within an individual container, and all of the various containers are within a single package along with instructions for use.

In one embodiment, the kit comprises multiple probes in an array format, such as those described, for example, in U.S. Patent Nos. 5,412,087 and 5,545,531, and International Publication No. WO 95/00530, herein incorporated by reference. Probes for use in the array may be synthesized either directly onto the surface of the array, as disclosed in International Publication No. WO 95/00530, or prior to immobilization onto the array surface (Gait, ed. (1984), Oligonucleotide Synthesis a Practical Approach IRL Press Oxford, England). The probes may be immobilized onto the surface using techniques well known to one of skill in the art, such as those described in U.S. Patent No. 5,412,087. Probes may be a nucleic acid or peptide sequence, preferably purified, or an antibody.

The arrays may be used to screen organisms, samples, or products for differences in their genomic, cDNA, polypeptide, or antibody content, including the presence or absence of specific sequences or proteins, as well as the concentration of those materials. Binding to a capture probe is detected, for example, by signal generated from a label attached to the nucleic acid molecule comprising the disclosed nucleic acid sequence, a polypeptide comprising the disclosed amino acid sequence, or an antibody. The method can include contacting the molecule comprising the disclosed nucleic acid, polypeptide, or antibody with a first array having a plurality of capture probes and a second array having a different plurality of capture probes. The results of each hybridization can be compared to analyze differences in expression between a first and second sample. The first plurality of capture probes can be from a control sample, e.g., a wild type lactic acid bacteria, or control subject, e.g., a food, dietary supplement, starter culture sample or a biological fluid. The second plurality of capture probes can be from an experimental sample, e.g., a mutant type lactic acid bacteria, or subject that has consumed a probiotic material, e.g., a starter culture sample, or a biological fluid.

These assays may be especially useful in microbial selection and quality control procedures where the detection of unwanted materials is essential. The detection of particular nucleotide sequences or polypeptides may also be useful in determining the genetic composition of food, fermentation products, or industrial microbes, or microbes present in the digestive system of animals or humans that have consumed probiotics.

Assays to detect expression of the disclosed polypeptides and/or nucleic acid molecules can also include the detection and/or quantitation of AI-2. Methods for the detection of AI-2 are described elsewhere herein. Assays to measure adherence, for example, in response to adhesion adaptive conditions, can also be measured to evaluate the expression of the polypeptides of the present invention. Such methods are also described elsewhere herein.

### III. Recombinant Expression Vectors and Host Cells

The nucleic acid molecules of the present invention may be included in vectors, preferably expression vectors. "Vector" refers to a nucleic acid molecule capable of transporting another nucleic acid to which it has been linked. Expression vectors include one or more regulatory sequences and direct the expression of genes to which they are operably linked. By "operably linked" is intended that the nucleotide sequence of interest is linked to the regulatory sequence(s) such that expression of the nucleotide sequence is allowed (e.g., in an in vitro transcription/translation system or in a host cell when the vector is introduced into the host cell). The term "regulatory sequence" is intended to include controllable transcriptional promoters, operators, enhancers, transcriptional terminators, and other expression control elements such as translational control sequences (e.g., Shine-Dalgamo consensus sequence, initiation and termination codons). These regulatory sequences will differ, for example, depending on the host cell being used.

The vectors can be autonomously replicated in a host cell (episomal vectors), or may be integrated into the genome of a host cell, and replicated along with the host genome (non-episomal mammalian vectors). Integrating vectors typically contain at least one sequence homologous to the bacterial chromosome that allows for recombination to occur between homologous DNA in the vector and the bacterial chromosome. Integrating vectors may also comprise bacteriophage or transposon sequences. Episomal vectors, or plasmids are circular double-stranded DNA loops into which additional DNA segments can be ligated. Plasmids capable of stable maintenance in a host are generally the preferred form of expression vectors when using recombinant DNA techniques.

The expression constructs or vectors encompassed in the present invention comprise a nucleic acid construct of the invention in a form suitable for expression of the nucleic acid in a host cell. Expression in prokaryotic host cells is encompassed in the present invention. It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (e.g., AI-2-related or AAR-related proteins, mutant forms of AI-2-related or AAR-related proteins, fusion proteins, etc.).

Regulatory sequences include those that direct constitutive expression of a nucleotide sequence as well as those that direct inducible expression of the nucleotide sequence only under certain environmental conditions. A bacterial promoter is any DNA sequence capable of binding bacterial RNA polymerase and initiating the downstream (3') transcription of a coding sequence (e.g., structural gene) into mRNA. A promoter will have a transcription initiation region, which is usually placed proximal to the 5' end of the coding sequence. This transcription initiation region typically includes an RNA polymerase binding site and a transcription initiation site. A bacterial promoter may also have a second domain called an operator, which may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence.

An example of a gene activator protein is the catabolite activator protein (CAP), which helps initiate transcription of the lac operon in *Escherichia coli* (Raibaud et al. (1984) Annu. Rev. Gercet. 18:173). Regulated expression may therefore be either positive or negative, thereby either enhancing or reducing transcription. Other examples of positive and negative regulatory elements are well known in the art. Various promoters that can be included in the protein expression system include, but are not limited to, a T7/LacO hybrid promoter, a trp promoter, a T7 promoter, a lac promoter, and a bacteriophage lambda promoter. Any suitable promoter can be used to carry out the present invention, including the native promoter or a heterologous promoter. Heterologous promoters may be constitutively active or inducible. A non-limiting example of a heterologous promoter is given in US Patent No. 6,242,194 to Kullen and Klaenhammer.

Sequences encoding metabolic pathway enzymes provide particularly useful promoter sequences. Examples include promoter sequences derived from sugar metabolizing enzymes, such as galactose, lactose (lac) (Chang et al. (1987) Nature 198:1056), and maltose. Additional examples include promoter sequences derived from biosynthetic enzymes such as tryptophan (trp) (Goeddel et al. (1980) Nucleic Acids Res. 8:4057; Yelverton et al. (1981) Nucleic Acids Res. 9:731; U.S. Patent No. 4,738,921; EPO Publication Nos. 36,776 and 121,775). The beta-lactamase (bla) promoter system (Weissmann, (1981) "The Cloning of Interferon and Other Mistakes," in Interferon 3 (ed. I. Gresser); bacteriophage lambda PL (Shimatake et al. (1981) Nature 292:128); the arabinose-inducible araB promoter (U.S. Patent No. 5,028,530); and T5 (U.S. Pat. No. 4,689,406) promoter systems also provide useful promoter sequences. See also Balbas (2001) Mol. Biotech. 19:251-267, where E. *coli* expression systems are discussed.

In addition, synthetic promoters that do not occur in nature also function as bacterial promoters. For example, transcription activation sequences of one bacterial or bacteriophage promoter may be joined with the operon sequences of another bacterial or bacteriophage promoter, creating a synthetic hybrid promoter (U.S. Patent No. 4,551,433). For example, the tac (Amann et al. (1983) Gene 25:167; de Boer et al. (1983) Proc. Natl. Acad Sci. 80:21) and trc (Brosius et al. (1985) J. Biol. Chem. 260:3539-3541) promoters are hybrid trp-lac promoters comprised of both trp promoter and lac operon sequences that are regulated by the lac repressor. The tac promoter has the additional feature of being an inducible regulatory sequence. Thus, for example, expression of a coding sequence operably linked to the tac promoter can be induced in a cell culture by adding isopropyl-1-thio-β-D-galactoside (IPTG). Furthermore, a bacterial promoter can include naturally occurring promoters of non-) bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system (Studier et al. (1986) J. Mol. Biol. 189:113; Tabor et al. (1985) Proc. Natl. Acad. Sci. 82:1074). In addition, a hybrid promoter can also be comprised of a bacteriophage promoter and an E. *coli* operator region (EPO Publication No. 267,851).

The vector may additionally contain a gene encoding the repressor (or inducer) for that promoter. For example, an inducible vector of the present invention may regulate transcription from the Lac operator (LacO) by expressing the gene encoding the LacI repressor protein. Other examples include the use of the lexA gene to regulate expression of pRecA, and the use of trpO to regulate ptrp. Alleles of such genes that increase the extent of repression (e.g., lacIq) or that modify the manner of induction (e.g., lambda CI857, rendering lambda pL thermo-inducible, or lambda CI+, rendering lambda pL chemo-inducible) may be employed.

In addition to a functioning promoter sequence, an efficient ribosome-binding site is also useful for the expression of the fusion construct. In prokaryotes, the ribosome binding site is called the Shine-Dalgarno (SD) sequence and includes an initiation codon (ATG) and a sequence 3-9 nucleotides in length located 3-11 nucleotides upstream of the initiation codon (Shine et al. (1975) Nature 254:34). The SD sequence is thought to promote binding of mRNA to the ribosome by the pairing of bases between the SD sequence and the 3' end of bacterial 16S rRNA (Steitz et al. (1979) "Genetic Signals and Nucleotide Sequences in Messenger RNA," in Biological Regulation and Development: Gene Expression (ed. R. F. Goldberger, Plenum Press, NY).

AI-2-related or AAR-related proteins can also be secreted from the cell by creating chimeric DNA molecules that encode a protein comprising a signal peptide sequence fragment that provides for secretion of the AI-2-related or AAR-related polypeptides in bacteria (U.S. Patent No. 4,336,336). The signal sequence fragment typically encodes a signal peptide comprised of hydrophobic amino acids that direct the secretion of the protein from the celL The protein is either secreted into the growth media (Gram-positive bacteria) or into the periplasmic space, located between the inner and outer membrane of the cell (Gram-negative bacteria). Preferably there are processing sites, which can be cleaved either *in vivo* or *in vitro,* encoded between the signal peptide fragment and the AI-2-related or AAR-related protein.

DNA encoding suitable signal sequences can be derived from genes for secreted bacterial proteins, such as the *E. coli* outer membrane protein gene (ompA) (Masui et al. (1983) FEBS Lett. 151(1):159-164; Ghrayeb et al. (1984) EMBO J. 3:2437-2442) and the *E. coli* alkaline phosphatase signal sequence (phoA) (Oka et al. (1985) Proc. Natl. Acad. Sci. 82:7212). Other prokaryotic signals include, for example, the signal sequence from penicillinase, Ipp, or heat stable enterotoxin II leaders.

Bacteria such as *L. acidophilus* generally utilize the start codon ATG, which specifies the amino acid methionine (which is modified to N-formylmethionine in prokaryotic organisms). Bacteria also recognize alternative start codons, such as the codons GTG and TTG, which code for valine and leucine, respectively. When they are used as the initiation codon, however, these codons direct the incorporation of methionine rather than of the amino acid they normally encode. *Lactobacillus acidophilus* NCFM recognizes these alternative start sites and incorporates methionine as the first amino acid.

Typically, transcription termination sequences recognized by bacteria are regulatory regions located 3' to the translation stop codon and thus, together with the promoter, flank the coding sequence. These sequences direct the transcription of an mRNA that can be translated into the polypeptide encoded by the DNA. Transcription termination sequences frequently include DNA sequences (of about 50 nucleotides) that are capable of forming stem loop structures that aid in terminating transcription. Examples include transcription termination sequences derived from genes with strong promoters, such as the trp gene in *E*. *coli* as well as other biosynthetic genes.

The expression vectors will have a plurality of restriction sites for insertion of the AI-2-related or AAR-related sequence so that it is under transcriptional regulation of the regulatory regions. Selectable marker genes that ensure maintenance of the vector in the cell can also be included in the expression vector. Preferred selectable markers include those that confer resistance to drugs such as ampicillin, chloramphenicol, erythromycin, kanamycin (neomycin), and tetracycline (Davies et al. (1978) Annu. Rev. Microbiol. 32:469). Selectable markers may also allow a cell to grow on minimal medium, or in the presence of toxic metabolite and may include biosynthetic genes, such as those in the histidine, tryptophan, and leucine biosynthetic pathways.

As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous polynucleotide is from a species different from the species from which the polynucleotide was derived, or, if from the same/analogous species, one or both are substantially modified from their original form and/or genomic locus, or the promoter is not the native promoter for the operably linked polynucleotide.

The regulatory regions may be native (homologous), or may be foreign (heterologous) to the host cell and/or the nucleotide sequence of the invention. The regulatory regions may also be natural or synthetic. Where the region is "foreign" or "heterologous" to the host cell, it is intended that the region is not found in the native cell into which the region is introduced. Where the region is "foreign" or "heterologous" to the AI-2-related or AAR-related nucleotide sequence of the invention, it is intended that the region is not the native or naturally occurring region for the operably linked AI-2-related or AAR-related nucleotide sequence of the invention. For example, the region may be derived from phage. While it may be preferable to express the sequences using heterologous regulatory regions, native regions may be used. Such constructs would be expected in some cases to alter expression levels of AI-2-related or AAR-related proteins in the host cell. Thus, the phenotype of the host cell could be altered.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

The invention further provides a recombinant expression vector comprising a DNA molecule of the invention cloned into the expression vector in an antisense orientation. That is, the DNA molecule is operably linked to a regulatory sequence in a manner that allows for expression (by transcription of the DNA molecule) of an RNA molecule that is antisense to AI-2-related or AAR-related mRNA. Regulatory sequences operably linked to a nucleic acid cloned in the antisense orientation can be chosen to direct the continuous or inducible expression of the antisense RNA molecule. The antisense expression vector can be in the form of a recombinant plasmid or phagemid in which antisense nucleic acids are produced under the control of a high efficiency regulatory region, the activity of which can be determined by the cell type into which the vector is introduced. For a discussion of the regulation of gene expression using antisense genes *see* Weintraub et al. (1986) Reviews - Trends in Genetics, Vol. 1(1).

Alternatively, some of the above-described components can be put together in transformation vectors. Transformation vectors are typically comprised of a selectable market that is either maintained in a replicon or developed into an integrating vector, as described above.

### IV. Microbial or Bacterial Host Cells

Any bacteria of interest can be used in the methods and compositions of the invention. In specific embodiments of the invention, the bacteria employed in the methods is a probiotic bacteria. By "probiotic" is intended a live microorganism that survives passage through the gastrointestinal tract and has a beneficial effect on the subject. As used herein, "probiotic properties" comprises enhanced gut function and stability; improved protection against infectious and non-infectious diseases; immune system modulation; alleviated lactose intolerance; improved digestion and nutrient absorption; reduced blood cholesterol; reduced allergy risk; and reduced risk of urinary tract infections. In some embodiments, an increase in adhesion, stress tolerance, or AI-2 production in a bacterium results in improvement of at least one probiotic property of the bacterium.

In other embodiments of the. invention, the bacteria is a lactic acid bacteria. As used herein, "lactic acid bacteria" is intended bacteria from a genus selected from the following: *Aerococcus,* Carnobacterium, *Enterococcus, Lactococcus, Lactobacillus,* Leuconostoc, *Oenococcus, Pediococcus,* Streptococcus, *Melissococcus, Alloiococcus, Dolosigranulum, Lactosphaera, Tetragenococcus, Vagococcus, and Weissella* (Holzapfel et al. (2001) Am. J. Clin. Nutr. 73:365S-373S; Sneath, ed. (1986) Bergey's Manual of Systematic Bacteriology Vol 2, Lippincott, Williams and Wilkins, Hagerstown, MD).

In still other embodiments, *Lactobacillus* is used. By *"Lactobacillus"* is meant any bacteria from the genus *Lactobacillus,* including but not limited to *L. casei, L. rhamnosus, L. johnsonni, L. gasseri, L. acidophilus, L. plantarum, L. fermentum*, *L. salivarius, L. bulgaricus,* and numerous other species outlined by Wood et al. (Holzapfel and Wood, eds. (1995) The Genera of Lactic Acid Bacteria, Vol. 2., Springer, New York)

The production of bacteria containing heterologous genes, the preparation of starter cultures of such bacteria, and methods of fermenting substrates, particularly food substrates such as milk, may be carried out in accordance with known techniques, including but not limited to those described in Mäyrä-Mäkinen and Bigret (1993) Lactic Acid Bacteria. Salminen and vonWright eds. Marcel Dekker, Inc. New York. 65-96.; Sandine (1996) Dairy Starter Cultures Cogan and Accolas eds. VCH Publishers, New York. 191-206; Gilliland (1985) Bacterial Starter Cultures for Food. CRC Press, Boca Raton, Florida.

By "fermenting" is intended the energy-yielding, metabolic breakdown of organic compounds by microorganisms that generally proceeds under anaerobic conditions and with the evolution of gas.

Nucleic acid molecules or amino acid sequences of the invention may be introduced into host cells by methods known in the art. By "introducing" is intended introduction into prokaryotic cells via conventional transformation or transfection techniques, or by phage-mediated infection. As used herein, the terms "transformation," "transduction," "conjugation," and "protoplast fusion" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation. Suitable methods for transforming or transfecting host cells can be found in Sambrook et al. (1989) Molecular Cloning: A Laboratory Manual (2d ed., Cold Spring Harbor Laboratory Press, Cold Spring Harbor, New York) and other laboratory manuals.

Bacterial cells used to produce the AI-2-related or AAR-related polypeptides of this invention are cultured in suitable media, as described generally in Sambrook et al. (1989) Molecular Cloning, A Laboratory Manual (2d ed., Cold Spring Harbor Laboratory Press, Cold Spring Harbor, New York).

Bacterial strains encompassed by the present invention include those that are biologically pure cultures of a bacterium comprising at least one nucleotide or amino acid sequence of the present invention. These strains may include but are not limited to: *Lactobacillus acidophilus, L. gasseri, L. johnsonii,* or *L. plantarum.*

A heterologous expression control sequence or promoter can be operatively associated with a desired nucleotide sequence in accordance with known techniques, such as by targeted insertion or "gene activation" by homologous recombination. *See, e.g*., US Patent Nos. 6,391,633 and 6,569,681.

A "subject bacteria or cell" is one in which genetic alteration, such as transformation, has been effected as to a gene of interest, a cell which is descended from a cell so altered and which comprises the alteration, or is a bacteria that has been subjected to adhesion adaptive conditions. A "control" or "control cell" or "control bacteria" provides a reference point for measuring changes in phenotype of the subject bacteria.

A control bacteria may comprise, for example: (a) a wild-type bacteria, i.e., of the same genotype as the starting material for the genetic alteration which resulted in the subject bacteria; (b) a bacteria of the same genotype as the starting material but which has been transformed with a null construct (i.e. with a construct which has no known effect on the trait of interest, such as a construct comprising a marker gene); (c) bacteria genetically identical to the subject bacteria but which is not exposed to adhesion adaptive conditions or conditions or stimuli that would modulate AI-2 production; or (d) the subject bacteria itself, under conditions in which the gene of interest is not expressed.

### V. Methods

### i. Modulating the Adhesion Adaptive Response

In one embodiment, the present invention has identified "adhesion adaptive conditions." As used herein "adhesion adaptive conditions" comprise any physical, chemical, biological, or similar condition that improves the adhesion of bacteria to a substrate. Adhesion can be modulated, for example, by culturing a bacteria to a desired cell density, and then incubating the bacteria under conditions that will enhance the adhesion response of the bacteria. For the purposes of the present invention, "culturing" or a "cell culture" is intended to describe cells that are grown in a synthetic environment. Culture conditions (for example growth media, pH, temperature) vary widely for each cell type, and variation of conditions for a particular cell type can result in different phenotypes being expressed. The cells are typically cultured in favorable conditions to promote cell growth. The cells can be cultured in minimal media (containing the exact nutrients, including any growth factors, needed by the bacteria for growth) or complex media (usually containing the full range of growth factors that may be required for the growth of the bacteria). One can also adjust the physical conditions of a culture medium, such as pH and temperature, to prevent the growth of some organisms while enhancing the growth of others.

In specific embodiments, the bacterial culture conditions comprise culturing anaerobically at 37°C or 42°C in Mann-Rogosa-Sharpe (MRS) media. The media can be modified to substitute galactose or other suitable carbohydrates for glucose as the source of sugar in the media. The cells can be harvested at early-log growth phase (defined as having an optical density at 600 nm (OD₆₀₀) up to 0.4), mid-log growth phase (OD₆₀₀ around 0.4, 0.5, 0.6, 0.7, 0.8, or 0.9), or late-log growth phase (OD₆₀₀ greater than 0.9) prior to exposure to adhesive adaption conditions. In specific embodiments, the cells are harvested an OD₆₀₀ of about 0.6. Bacterial cells are harvested, for example, by sedimentation in a centrifuge or other appropriate device, and resuspended in suitable media.

The harvested bacterial cells can then be preconditioned by incubation under adhesion adaptive conditions (i.e., by incubating the bacterial cells under adhesion adaptive conditions) which results in an increase in adhesion to a target substrate. "Incubating" or "incubation" refers to maintaining a population of bacteria under specific conditions (i.e., adhesion adaptive conditions) in order to promote a particular reaction (for example, an adhesion adaptive response). The adhesion adaptive conditions can include, for example, incubation of bacteria for a time sufficient to increase the adhesion of the bacteria. In some embodiments, the bacteria of the present invention are incubated for at least about 30, 35, 40, 45, 50, 55, 60, 65, 70, 75, 80, 85, 90, 100, or 120 minutes. In other embodiments, the bacteria are incubated under conditions in which the cells have been concentrated to a density of about 1.5 x 10⁸ to about 1 x 10¹⁰ colony forming units per milliliter (cfu/ml) of diluent (e.g., MRS media), including about 2.0 x 10⁸, 3.0 x 10⁸, 4.0 x 10⁸, 5.0 x 10⁸, 6.0 x 10⁸, 7.0 x 10⁸, 8.0 x 10⁸, 9.0 x 10⁸, 1.0 x 10⁹ or greater cfu/ml. In specific embodiments, the concentration is greater than 1.0 x 10⁸ cfu/ml. Further embodiments include incubation in a media that has been adjusted to a pH of less than 5, including 4.5, 4.0, 3.5, and 3.0. In specific embodiments, the pH is allowed to naturally fall through a range of about 7.0 to about 4.5. Incubation of bacterial cells under these or other suitable conditions prior to contact of the bacteria to a target substrate ("preconditioning") can either enhance or diminish, depending on the incubation conditions, the adhesion of the preconditioned bacteria to the target substrate. Any substrate of interest can be employed including, for example, a cell of the gastrointestinal tract or the urogenital tract.

An increase in adhesion comprises any statistically significant increase in adhesion to a target substrate when compared to an appropriate control (for example, bacteria that have not been exposed to adhesion adaptive conditions). In specific embodiments, the increase can comprise, but is not limited to, at least around 90%, 100%, 120%, 130%, 140%, 150%, 200%, 250% or greater. An increase in adhesion can be assayed *in vitro* by, for example, contacting the preconditioned bacteria to a suitable target (i.e., epithelial or mucosal cells) and counting the number of bacteria that adhere to the target substrate. An increase in adhesion can be assayed *in vivo* by monitoring host response to exposure to preconditioned bacteria. For example, adhesion of a probiotic bacterium that has been preconditioned under adhesion adaptive conditions can be assessed by monitoring improvement of probiotic properties such as enhanced gut function and stability; improved protection against infectious and non-infectious diseases; immune system modulation; alleviated lactose intolerance; improved digestion and nutrient absorption; reduced blood cholesterol; reduced allergy risk; and reduced risk of urinary tract infections. Additional methods for measuring bacterial adhesion *in vivo* are described in Leffler, et al. (1995) Methods Enzymol. 253:206-220.

In some embodiments, an increase in adhesion comprises modulation of expression of at least one of SEQ ID NO:19, 20, 33, 34, 35, 36, 37, or 38, or variants and fragments thereof. Further embodiments comprise modulation of expression of 2, 3, 4 or more of these sequences. A modulation in adhesion can be measured by comparing the expression of the polynucleotides of the invention in a bacterium in which the polynucleotides have been introduced to the expression of the same polynucleotide(s) in a control bacterium as defined elsewhere herein. Methods for measuring expression of these sequences are known in the art and are discussed elsewhere herein.

### ii. Modulating AI-2 Production

The compositions and methods of the present invention can be used to modulate the production of AI-2 in bacteria. By "modulate," "alter," or "modify" is intended the up- or down-regulation of a target biological activity, particularly the up-regulation of activity. Nucleic acid molecules and polypeptides of the invention are useful in modifying the biological activities of lactic acid bacteria, especially lactic acid bacteria that are used to ferment foods with nutritional or health-promoting characteristics. Up- or down-regulation of expression from a polynucleotide of the present invention is encompassed. Up-regulation may be accomplished by providing multiple gene copies, modulating expression by modifying regulatory elements, promoting transcriptional or translational mechanisms, or other means. Down-regulation may be accomplished by using known antisense and gene silencing techniques.

In specific embodiments, the invention provides a bacterium comprising at least one heterologous AI-2 related nucleic acid molecule (i.e., SEQ ID NO: 1, 3, 13, 15, or 21) or biologically active variants or fragments thereof. Further embodiments include a bacterium comprising at least two, at least three, at least four, or at least five AI-2 related nucleic acid molecules as described above. Expression of these heterologous sequences in the bacterium produces an increased level of autoinducer-2 than when compared to an appropriate control bacteria. As used herein, an increase in autoinducer-2 comprises any significant increase in AI-2 over the control. In specific embodiments, the increase can comprise, but is not limited to, at least about 90%, 100%, 120%, 130%, 140%, 150%, 200%, 250% or greater increase in AI-2 production when compared to an appropriate control. Methods to assay for this increase in AI-2 production are discussed in detail elsewhere herein.

The bacteria of the invention having the increase in AI-2 production can display increased adhesion to a target substrate and/or also display improved stress tolerance.

Microbes expressing the polypeptides of the present invention are useful as additives in dairy and fermentation processing. The polynucleotide sequences, encoded polypeptides, and microorganisms expressing them are useful in the manufacture of milk-derived products, such as cheeses, yogurt, fermented milk products, sour milks, and buttermilk. Microorganisms that express polypeptides of the invention may be probiotic organisms, a lactic acid bacteria, or any other bacterial host of interest.

### iii. Improving Stress Tolerance and/or Adhesion

As discussed above, the present invention provides bacteria having improved stress tolerance and/or adhesion activity. As used herein, an improvement in adhesion to a target substrate comprises any significant increase in adhesion to the target substrate including, but not limited to, a 90%, 100%, 120%, 130%, 140%, 150%, 200%, 250% or greater increase in adhesion when compared to an appropriate control. Any substrate of interest can be employed including, for example, a cell of the gastrointestinal tract or the urogenital tract. In specific embodiments, the cell comprises an epithelial cell or a mucosal cell. It if further recognized that the cell can be contacted with the bacteria either *in vitro* or *in vivo.*

As used herein, an improved stress-tolerance of a bacteria comprises any significant increase in survival of the bacteria under stress conditions, including, but not limited to, around a 90%, 100%, 120%, 130%, 140%, 150%, 200%, 250% or greater increase in survival when compared to an appropriate control.

A probiotic bacteria of the invention having improved adhesion and/or improved stress tolerance finds use in promoting the heath of a subject. While not intending to be limited by any mechanism of action, probiotic bacteria having these enhanced characteristics upon administration to a subject can block adhesion and compete with pathogens; stimulate host-cell immune defenses; and/or trigger cell-signaling events that "silence" the production of virulence factors from the pathogens. Thus improving the stress tolerance and/or the adhesion activity of a probiotic organism will further aid in reducing the risk of infection of the gut, urogenital tract, and wound sites.

The probiotic bacteria having the increased stress-tolerance and/or increased adhesion can be administered to any subject in need thereof. For example, the subject can comprise a mammal, a human, a domestic animal or an agricultural animal. Administration may be nasal, oral, vaginal or anal. In contexts where mucosal administration is not preferred, the bacterium may be administered by an other suitable means within the capacity of those skilled in the art, i.e., parental routs (i.v., i.p., s.c., i.m.).

It is recognized that the bacteria of the invention having the improved stress-tolerance and/or improved adhesion can further express at least one polynucleotide and/or polypeptide that has therapeutic activity. By "therapeutic activity" is intended that the biological effect when the polypeptide is delivered to a subject is beneficial to that subject. Administration is preferably in a "therapeutically effective amount," this being sufficient to show benefit to the subject. Such benefit may be at least amelioration of at least one symptom. In a prophylactic context, the amount may be sufficient to reduce the deleterious effect on the individual of a subsequent pathogenic challenge, for instance, by enhancing the immune response. The actual amount administered, rate and time-course of administration, will depend on the aim of the administration, e.g., the biological effect sought in view of the nature and severity of the challenge, and is the subject of routine optimization. Prescription of treatment, including prophylactic vaccination, for example, decisions on dosage, etc., is within the responsibility of general practitioners and other medical doctors.

Polynucleotides and/or polypeptides that have therapeutic activity and can be expressed in the probiotic bacteria having improved adhesion activity or improved stress tolerance can include, for example, insulin, growth hormone, prolactin, calcitonin, luteinizing hormone, parathyroid hormone, somatostatin, thyroid-stimulating hormone, vasoactive intestinal polypeptide, a structural group 1 cytokine adopting an antiparallel 4.alpha. helical bundle structure such as IL-2, IL-3, IL-4, IL-5, IL-6, IL-7, IL-9, IL-10, IL-11, IL-12, IL-13, GM-CSF, M-CSF, SCF, IFN-γ, EPO, G-CSF, LIF, OSM, CNTF, GH, PRL or IFN-α/β, the TNF family of cytokines, e.g., TNFα, TNFβ, CD40, CD27 or FAS ligands, the IL-1 family of cytokines, the fibroblast growth factor family, the platelet-derived growth factors, transforming growth factor .beta. and nerve growth factors, a structural group 3 cytokine comprising short chain α/β molecules, which are produced as large transmembrane precursor molecules which each contain at least one EGF domain in the extracellular region, e.g., the epidermal growth factor family of cytokines, the chemokines characterized by their possession of amino acid sequences grouped around conserved cysteine residues (the C--C or C--X--C chemokine subgroups) or the insulin-related cytokines, a structural group 4 cytokine which exhibits mosaic structures such as the heregulins or neuregulins composed of different domains, e.g., EGF, immunoglobulin-like and kringle domains. Alternatively, the biologically active polypeptide can be a receptor or antagonist for biologically active polypeptides as defined above. In specific embodiments, the polypeptide having therapeutic activity comprises an antigen. For additional therapeutic polypeptide that can be expressed in non-pathogenic bacteria see, for example, U.S. Application 20030202991; Vandenbroucke et al. (2004) Gastroenterology 127:667-8; Huyghebaert et al. (2005) Eur J Pharm Biopharm 59:9-15; Steidler et al. (2000) Science 289:1352-1355; Steidler et al. (2001) The Scientific World 1:215-217; U.S. Application No. 20020019043; each of which are herein incorporated by reference in their entirety. It is recognized that in particular applications, the therapeutic polypeptide may be engineered to be secreted from the probiotic bacteria.

### iv. Method of Screening

Method of screening for chemicals or environmental conditions that increase adhesion of a bacterium to a substrate are provided. The method comprises subjecting the bacterium to an environmental condition suspected of increasing adhesion and/or suspected of increasing Al-2 production; and, contacting said bacterium to the substrate. An increase in adhesion of the bacterium subjected to the environmental condition to the substrate compared to adhesion of the same bacterium that has not been subjected to the environmental conditions indicates that the environmental condition is effective in increasing adhesion of the bacterium.

A variety of candidate chemicals or environmental conditions can be employed in this screening method. Such conditions may include, but are not limited to, inducers/suppressors of AI-2 or any gene involved in the production of AI-2, microbiological agents (e.g., prokaryotic and eukaryotic organisms), temperature, concentration, time of incubation in adhesion adaptive conditions, composition of incubation media (e.g., presence, abundance, and/or type of growth factors, carbohydrates, amino acids, salt, minerals, and the like), cell density, or pH. A candidate agent may be a chemical compound, a mixture of chemical compounds, or a biological macromolecule. Such candidate agents can be contained in various agent banks including, for example, compound libraries, peptide libraries, and the like.

The conditions or compounds identified by this process will stimulate bacteria, including both recombinant and non-recombinant wild type bacteria, to produce AI-2, or to promote adhesion or biofilm formation for use in fermentation or probiotic bacteria, or in any other method described herein.

The present invention is explained in greater detail in the examples set forth below.

### EXPERIMENTAL

### Example 1

### Gapped BlastP Analysis of Amino Acid Sequences

Sequences of the invention were aligned using the Gapped BlastP alignment method and parameters disclosed herein. Table 1 summarizes the top Blast results for these sequences.

A Gapped BlastP amino acid sequence alignment showed that SEQ ID NO:14 (372 amino acids, ORF LBA1080) has about 55% identity from amino acids 11-371 with a protein from *Leuconostoc mesenteroides* subsp. *mesenteroides* that is a methionine synthase II (cobalamin-independent) protein (Accession No. ZP_00064070.1), about 47% identity from amino acids 5-372 with a protein from *Lactobacillus gasseri* that is a methionine synthase II (cobalamin-independent) protein (Accession No. ZP_00046311.1), about 46% identity from amino acids 7-372 with a hypothetical protein from *Chlamydophila pneumoniae* (Accession No. NP_2243 51.1), 44% identity from amino acids 4-372 with a hypothetical protein from *Lactobacillus johnsonii* (Accession No. NP_965623.1), and 45% identity from amino acids 9-372 with a protein from *Oenococcus oeni* that is a methionine synthase II (cobalamin-independent) protein (Accession No. ZP_00069898.1).

A Gapped BlastP amino acid sequence alignment showed that SEQ ID NO:16 (157 amino acids ORF LBA1081) has about 87% identity from amino acids 1-157 with a protein from *Lactobacillus johnsonii* that is an autoinducer-2 production protein LuxS (Accession No. NP_965624.1), about 87% identity from amino acids 1-157 with a protein from *Lactobacillus gasseri* that is a LuxS protein involved in autoinducer AI2 synthesis (Accession No. ZP_00046310.1), about 76% identity from amino acids 4-157 with a protein from *Streptococcus bovis* that is a LuxS autoinducer 2 synthase (Accession No. dbj|BAD06876.1), 77% identity from amino acids 1-157 with a protein from *Lactobacillus plantarum* that is an autoinducer production protein (Accession No. NP_784522.1), and 73% identity from amino acids 4-157 with a protein from *Streptococcus pyogenes* that is an autoinducer-2 production protein (Accession No. NP_269689.1).

A Gapped BlastP amino acid sequence alignment showed that SEQ ID NO:18 (444 amino acids, ORF LBA169) has about 95% identity from amino acids 49-444 with a protein from *Lactobacillus acidophilus* that is an S-layer protein precursor (Accession No. sp|P35829|SLAP_LACAC), about 67% identity from amino acids 49-443 with a protein from *Lactobacillus helveticus* that is a surface layer protein (Accession No. emb|CAA62606.1), about 67% identity from amino acids 49-443 with a protein from *Lactobacillus helveticus* that is a surface layer protein (Accession Nos. emb|CAB46984.1; AJ388558), 66% identity from amino acids 49-443 with a protein from *Lactobacillus helveticus* that is a surface layer protein (Accession No. emb|CAB46985.1), and 66% identity from amino acids 49-443 with a protein from *Lactobacillus helveticus* that is a surface layer protein (Accession No. emb|CAB46986.1).

A Gapped BlastP amino acid sequence alignment showed that SEQ ID NO:20 (566 amino acids, ORF LBA1148) has about 69% identity from amino acids 4-564 with a hypothetical protein from Lactobacillus johnsonii (Accession No. NP_965038.1), about 66% identity from amino acids 4-564 with a protein from *Lactobacillus gasseri* that is a predicted RNA-binding protein homologous to a eukaryotic snRNP (Accession No. ZP_00045959.1), about 41% identity from amino acids 4-566 with a protein from *Enterococcus faecium* that is a predicted RNA-binding protein homologous to a eukaryotic snRNP (Accession No. ZP_00037499.1), about 41 % identity from amino acids 4-566 with a protein from *Enterococcus faecalis* that is homologous to a fibronectin/fibrinogen-binding protein (Accession No. NP_814975.1), and about 41% identity from amino acids 4-557 with a protein from *Lactobacillus* plantarum that is an adherence protein (Accession No. NP_785358.1).

**Table 1. Top Blast result for protein sequences of the invention**

| **SEQ ID NO:** | **ORF** | **Percent Identity** | **Amino Acid Range** | **Organism** | **Description** | **Accession No.** |
|---|---|---|---|---|---|---|
| 2 | 820 | 69 | 1 to 229 | *Lactobacillus gasseri* | COG0775: Nucleoside phosphorylase | ref\|ZP_00046270.1 |
| 4 | 931 | 67 | 1 to 312 | *Lactobacillus gasseri* | COG2264: Ribosomal protein L11 methylase | ref\|ZP_00046385.1 |
| 6 | 1042 | 49 | 1 to 219 | *Streptococcus pneumoniae R6* | ABC transporter membrane-spanning permease - glutamine transport | ref\|NP_786178.1 |
| 8 | 1044 | 49 | 1 to 219 | *Streptococcus pneumoniae R6* | ABC transporter membrane-spanning permease - glutamine transport | ref\|ZP_00046137.1 |
| 10 | 1045 | 55 | 1 to 246 | *Leuconostoc mesenteroides subsp. mesenteroides ATCC 8293* | COG1126: ABC-type polar amino acid transport system, ATPase | ref\|NP_861002.1 |
| 12 | 1046 | 59 | 14 to 285 | *Leuconostoc lactis* | orf J; putative ATP-binding cassette transport system; similar to substrate binding protein and to components of ABC transport systems | ref\|NP_786170.1 |
| 14 | 1080 | 55 | 11 to 371 | *Leuconostoc mesenteroides subsp. mesenteroides* | methionine synthase II (cobalamin-independent) protein, MetE | ref\|ZP_00064070.1 |
| 16 | 1081 | 87 | 1 to 157 | *Lactobacillus johnsonii* | autoinducer-2 production protein LuxS | ref\|NP_965624.1 |
| 18 | 169 | 90 | 49 to 444 | *Lactobacillus acidophilus* | S-layer protein precursor | ref\|sp\|P35829 |
| 20 | 1148 | 69 | 4 to 564 | *Lactobacillus* johnsonii | Fibronectin binding protein, FbpA | ref\|NP_965038.1 |

### PFAM Analysis of Amino Acid Sequences

SEQ ID NO:2 (231 amino acids, ORF LBA820) contains a predicted PNP_UDP_1 domain from about amino acids 2 to 222, and is a member of the 5'-methyladenosine phosphorylase (MTA phosphorylase) family (PFAM Accession PF01048).

SEQ ID NO:4 (314 amino acids, ORF LBA931) contains a PrmA domain from about amino acids 6 to 312, and is a member of the Ribosomal protein L11 methyltransferase (PrmA) family (PFAM Accession PF06325).

SEQ ID NO:14 (372 amino acids, ORF LBA1080) contains a predicted Methionine_synt domain from about amino acids 11 to 369, and is a member of the vitamin-B12 independent methionine synthase protein family (PFAM Accession PF01717).

SEQ ID NO:16 (157 amino acids ORF LBA1081) contains a predicted LuxS domain located from about amino acids 2 to 155, and is a member of the LuxS protein family (LuxS) (PFAM Accession PF02664).

SEQ ID NO:20 (566 amino acids, ORF LBA1148) contains a predicted FbpA domain from about amino acids 1 to 425, and is a member of the Fibronectin-binding protein A (FbpA) family (PFAM Accession PF05833).

SEQ ID NO:22 (399 amino acids, ORF LBA1622) contains a predicted S-adenosylmethionine synthetase N-terminal domain from about amino acids 2-102 (PFAM Accession 00438), an S-adenosylmethionine synthetase central domain from about amino acids 124-243 (PFAM Accession 02772), and an S-adenosylmethionine synthetase C-terminal domain from about amino acids 245-384 (PFAM Accession 02773).

### Bacterial strains and growth conditions

*Lactobacillus* strains were cultivated anaerobically at 37°C or 42°C in MRS broth (Difco Laboratories Inc., Detroit, MI) or, when appropriate, in MRS supplemented with 1.5% agar. *Escherichia coli* was propagated aerobically in Luria-Bertani (LB, Difco) medium or on LB medium supplemented with 1.5% agar at 37°C. Brain Heart Infusion (BHI, Difco) medium supplemented with 1.5 % agar and 150 µg/ml erythromycin (Em) was used for selection of E. *coli* transformants. Autoinducer Bioassay (AB) media (Bassler et al. (1993) Mol. Microbiol. 9:773-786) was used for the propagation of all *Vibrio* harveyi strains. When appropriate, chloramphenicol (Cm, 5.0 µg/ml) and Em (5.0 µg/ml or 150 µg/ml) were used for selection. Colony forming units (CPU) per ml were determined with appropriate dilutions using a Whitley Automatic Spiral Plater (Don Whitley Scientific Limited, West Yorkshire, England).

**Table 2. Bacterial strains and plasmids used**

| **Strains** | **Origin or relevant characteristics** | **Source or reference** |
|---|---|---|
| *L. acidophilus* NCFM | Human intestinal isolate | (31) |
| NCK 1377 | NCFM::pTRK826 (*slpA* integrant) | (1) |
| NCK 1392 | NCFM containing pTRK669 | (29) |
| NCK 1398 | NCFM::pTRK685 (*lacL* integrant) | (29) |
| NCK 1661 | NCFM::pTRK833 (*fbpA* integrant) | (9) |
| NCK 1765 | NCFM::pTRK854 (*luxs* integrant) | This study |
| | | |
| *V*. *harveyi* | | |
| BB170 | *luxN*::Tn5 AI-1 sensor AI-2 sensor⁺ | (35) |
| | | |
| *E.coli* | | |
| EC1000 | RepA⁺ MC1000, Km^{r}, carrying a single copy of the pWV01 *repA;* host for pOR128-based plasmids | (25) |
| | | |
| Plasmids | | |
| pORI28 | Em^{r}, ori (pWV01), replicates only with *repA* provided in *trans* | (25) |
| pTRK669 | ori (pWV01), Cm^{r}, RepA⁺ | (29) |
| pTRK854 | 471 bp internal region of *luxS* (LBA1081) cloned into BglII-XbaI sites of pORI28 | This study |

### Tissue culture

The Caco-2 (ATCC HTB-37, Rockville, MD) cells were only used between the 40^{th} and 50^{th} passage. All reagents used in maintenance of Caco-2 cells were obtained from Gibco (Invitrogen Corp., Carlsbad, CA). The cells were routinely grown in 95% air-5% CO₂ atmosphere in Minimum Essential Medium (MEM) supplemented with 20% (v/v) inactivated (56°C, 30 min) fetal bovine serum (FBS), 0.10 mM non-essential amino acids and 1.0 mM sodium pyruvate. Monolayers were typsinized for 10 min, counted using a hemocytometer, and seeded at 1.3x10⁵ cells/well in 2.0 ml of cell culture medium. Cells were grown on 15 mm Thermanox plastic coverslips (Nalge Nunc International, Rochester, NY) in Costar 12-well tissue culture treated plates (Corning Inc., Acton, MA). The medium was replaced every two days and all adherence assays were performed after 14 days of incubation.

### Adherence Assay

Adhesion of *Lactobacillus* strains to Caco-2 cells was examined according to the method described previously (Buck et al. (2005) Appl. Environ. Microbiol. 71:8344-8351). Briefly, mid-log phase bacterial cells (OD₆₀₀ 0.6) were prepared in 10 ml MRS with 3.0 µg/ml Em to maintain selective pressure on integrants. Cells were removed by centrifugation for 10 min at 4000 x g, and washed twice with phosphate-buffered saline (PBS). Bacterial pellets were resuspended in 5 ml of fresh MRS prior to adherence. For adhesion adaptive conditions, cell pellets from mid-log phase cultures were resuspended in 1 ml of fresh MRS at ~1.0 x 10⁹ cfu/ml and incubated for 1 hr at 37°C. Cells were centrifuged again and resuspended in 5 ml fresh MRS prior to addition to the monolayers. Fifteen-day Caco-2 monolayers were washed twice with PBS and treated with a bacterial suspension at a concentration of 4 x 10⁸ CFU/ml. Bacteria were incubated on the monolayer for 1.5 hr at 37°C in a mixture (1:2 v/v) of MRS and cell-line culture medium. Following incubation, the monolayers were washed five times with PBS, fixed in methanol and Gram stained. Adherent bacterial cells were then enumerated microscopically. Duplicate coverslips were counted for each experiment. The final data presented collectively represents at least 3 independent experiments in duplicate. Total counts for each coverslip were used and adhesion was expressed as percent (%) of the control strain NCK1398 (Russell and Klaenhammer (2001) Appl. Environ. Microbiol. 67:4361-4364) which carries an insert in the *lacL* (β-galactosidase) gene. Using this control, all mutant cultures and the parental control could be prepared with Em to maintain selective pressure on the LuxS- integrant. The adhesion protocol is depicted in Figure 2.

### Adhesion adaptive response

Ten mL of log phase (OD₆₀₀ 0.7) cells were harvested by centrifugation and resuspended in either 1 or 10mL of fresh Mann-Rogosa Sharpe (MRS) growth medium and incubated for 1hr at 37C. Those resuspended in 10ml are represented as "control conditions" (Figure 1A) while those resuspended in 1 ml are the "adhesion adaptive conditions" (Figure 1B). For the purposes of the present invention, both the incubation conditions and the resuspension in the smaller (more concentrated) volume of media (e.g., 1 ml MRS for the AAR group versus 10 ml MRS for the control group) are considered adhesion adaptive conditions. The concentrated and incubated cells exhibited a significant increase in adhesion (Figure 1). The level of adherence for the concentrated and incubated cells was at least 20 fold greater than the non-concentrated and incubated control cells, even though the same number of bacterial cells was added initially to each well. In each experiment, cells were resuspended in 5 ml of fresh MRS and enumerated to determine the absolute concentration of cells added to the Caco-2 monolayer. Only adherence data resulting from the addition of 3.8 x 10⁸ - 4.2 x 10⁸CFU/ml was reported. Similar increases in bacterial adherence were not obtained by either 10 X concentration without incubation, or incubation (37°C, 1 hr) without concentration of the mid-log cells prior to adherence, or by incubation (37°C,1 hr) without concentration in MRS adjusted to pH 4.5 with lactic acid (data not shown). After the one hour incubation, the pH of the unconcentrated cells was 5.5, whereas the concentrated cells lowered the pH to 4.5. Therefore, a combination of cell concentration and decreased pH resulted in an adaptation of the *L*. *acidophilus* cells to a state significantly more amenable to adherence. We designated this phenomenon an Adhesion Adaptive Response (AAR).

Figure 3 shows the adhesion properties of control strain and selected mutant strains *of L. acidophilus* NCFM with and without exposure to the adhesion adaptive conditions. Data is reported as total cell count in 17 fields on a single coverslip. Each data point represents at least two experiments in duplicate. *L. acidophilus* NCFM containing an integration into the β-galactosidase gene acts as the "control" in order to maintain Em selection during growth of all mutants.

### Microarray analysis

A single 20 mL culture *of L. acidophilus* was grown in MRS to OD₆₀₀ of 0.6, divided into two 10 ml aliquots, and harvested by centrifugation for 8 min at 3,150 x g. One aliquot was resuspended in 10 ml of fresh MRS, the other resuspended in 1 ml of fresh MRS. Both cultures were then incubated for 1 hr at 37°C. Following incubation, cells were harvested by centrifugation and frozen immediately in a dry ice-ethanol bath. RNA isolation was conducted using TRIzol (Life Technologies, Rockville, MD) according to the protocol described previously (Azcarate-Peril et al. (2005) Appl. Environ. Microbiol. 71:5794-5804). RNA purity and concentration were determined by electrophoresis on agarose gels and standard spectrophotometer measurements.

Identical amounts (25 µg) of total RNA were aminoallyl-labeled by reverse transcription with random hexamers in the presence of amino-allyl dUTP (Sigma Chemical Co., St. Louis, MO), using Superscript II reverse transcriptase (Clontech Laboratories, Inc., Mountain View, CA) at 42°C overnight, followed by fluorescence-labeling of aminoallylated cDNA with *N*-hydroxysuccinimide-activated Cy3 or Cy5 esters (Amersham Pharmacia Biotech, Piscataway, NJ). Labeled cDNA probes were purified using the PCR Purification Kit (Qiagen, Valencia, CA). Coupling of the Cy3 and Cy5 dyes to the AA-dUTP labeled cDNA and hybridization of samples to microarrays were performed using standard protocols. Additional information on these protocols can be found on the TIGR website at www.tigr.org/tdb/microarray/protocolsTIGR.shtml. Fluorescence intensities were acquired using a Packard Bioscience ScanArray 4000 microarray scanner (Global Medical Instruments, Inc, Ramsey, MN) and processed as TIFF images. Signal intensities were quantified using the GSI Luminomics QuantArray 3.0 software package (PerkinElmer Life and Analytical Sciences, Inc., Boston, MA). Two slides (each containing triplicate arrays) were hybridized reciprocally to Cy3- and Cy5-labeled probes per experiment (dye swap). Spots were analyzed by adaptive quantitation (Azcarate-Peril *et al.,* 2005, *supra*)*.* The local background was subsequently subtracted from the recorded spot intensities. Data was median normalized. The median of the six ratios per gene was recorded. The ratio between the average absolute pixel values for the replicated spots of each gene with and without treatment represented the fold change in gene expression. Confidence intervals and *P* values on the fold change were also calculated with the use of a two-sample *t* test. *P* values of 0.05 or less were considered significant (Table 3).

**Table 3. Selected differentially expressed ORFs under adhesion adaptive conditions^{a}**

| **ORF#** 41 | Gene Annotation | Ratio¹ | **ORF#** 1046 | Gene Annotation | Ratio¹ |
|---|---|---|---|---|---|
| | ribonucleoside triphosphate reductase | 2.767 | 1046 | ABC transporter substrate-binding protein | 4.241 |
| **55** | **D-lactate dehydrogenase** | **3.225** | 1078 | bile salt hydrolase | 2.538 |
| **132** | **Putative transcr. reg. tetR family** | **0.157** | 1080 | MetE | 2.125 |
| 144 | n-acetylglucosamine-6-P deacetylase | 2.385 | 1081 | autoinducer-2 production protein LuxS | 1.852 |
| 154 | phosphonate ABC trans. (permease) | 0.489 | 1115 | amino acid permease | 4.056 |
| 160 | anaer. NTP reductase | 2.383 | 1119 | Putative inner membrane | 0.444 |
| 161 | anaer. NTP reductase or activator | 2.507 | **1122** | prot **DNA topoisomerase IV subunitB** | **0.328** |
| 165 | neutral endopeptidase | 2.348 | **1140** | **lysin** | **0.221** |
| **166** | **(K+) uptake protein** | **0.327** | **1177** | **iron-sulfur cofactor synthesis protein YrvO** | **0.430** |
| 199 | inosine-monophosph. dehydrogen. | 3.000 | **1178** | **[nucleolar protein]** | **0393** |
| 204 | aminopeptidase e | 2.881 | **1197** | **DNA primase** | **0353** |
| **205** | **heat shock low mol. weight** | **2.496** | 1208 | peptide methionine sulfoxide reductase msrA | 2.357 |
| **234** | carboxyvinyltransferase | **0.487** | **1243** | **DNA-specific exonuclease RecJ** | **0.457** |
| 262 | aa transporter | 0.459 | 1247 | heat shock protein DnaK | 3.532 |
| **266** | **d-ala-d-ala adding enzyme** | **0.321** | 1248 | cochaperonin GrpE, Hsp70 cofactor | 3.266 |
| **267** | **ATP-dep. RNA helicase ydbR** | **0.225** | 1249 | Heat inducible transcription repressor HrcA | 3.831 |
| **281** | **lys-trna synthetase lysrs** | **0.406** | **1269** | **translation elongation factor Ts** | **0.430** |
| **346** | **dna repair RadA** | **0.500** | **1270** | **30s ribosomal proteinS2** | **0.446** |
| 358 | transcr. antiterm. nusG | 0.485 | 1300 | oligopeptide ABC substrate binding prot oppA | 3.007 |
| 359 | 50S rib. prot L11 | 0.397 | 1341 | branched-chain amino acid aminotrans. ILVE | 3.354 |
| 395 | Putative dehydratase | 5.362 | **1376** | **transmembrane prot.** | **0.192** |
| 396 | oxalyl-coa decarboxylase | 5.525 | **1401** | **Nadh Peroxidase (Npx)** | **6.032** |
| **405** | **cochaperonin GroES** | **8.045** | **1433** | **dihydroxyacetone kinase** | **2.208** |
| **406** | **chaperonin GroEL** | **9.273** | **1457** | **galactose-1-epimerase (mutarotase)** | **3.434** |
| **493** | **aggregation promoting protein** | **0.295** | **1458** | **galactose-1-phosphate uridylyltransferase** | **3.315** |
| 523 | P-type ATPase | 2.750 | **1460** | **Mucus binding protein precursor** | **2.470** |
| 555 | **myosin-crossreactive antigen** | **6.833** | **1462** | **beta-galactosidase** | **3.765** |
| **617** | **ATP-dep. RNA helicase** | **0.498** | **1467** | **beta-galactosidase large subunit (lactase)** | **3.868** |
| 638 | ATP-dep.t Clp protease, ClpE | 6.577 | **1469** | **udp-glucose 4-epimerase** | **3.024** |
| 652 | >>H+/K+ ATPase to 657<< | 3.583 | 1551 | phosphonbosylamine-glycine ligase PUR2 | 0.414 |
| 655 | Phosphotrans. system II pthA | 2.187 | **1552** | **Phosphoribosylaminoimida zole. PUR9** | **0.207** |
| 672 | Phos. starvation induced prot YvyD | 2.345 | **1553** | **phosphoribosyl glycinamide PUR3** | **0.259** |
| 680 | glucan branching enzyme glgB | 3.643 | **1554** | **Phosphoribosyl. cyclo-ligase PUR5** | **0.219** |
| 682 | adenylyltransferase glgD | 3.489 | **1555** | **Phosphoribosylpyroph. amidotrans. PUR1** | **0.203** |
| 694 | ATP-dependent Clp protease P | 2.945 | **1556** | **Phosphoribosylformylglyc. synthase PURL** | **0.187** |
| **789** | **aminotranfserase - NifS family** | **0.480** | **1557** | **Phosphoribosylformylglyc. synthase PURQ** | **0387** |
| **791** | **16s pseudouridylate synthase** | **0.448** | **1559** | **Phosphoribosylaminoimid. synthase PUR7** | **0.243** |
| **822** | **trNA-methyltransferase** | **0.453** | 1564 | Putative membrane prot. | 0.318 |
| **847** | **clpX (stress related protease)** | **0.465** | 1566 | bacteriocin helveticin J | 2.762 |
| 851 | diaminopimelate decarboxylase | 3.351 | 1595 | glycerol uptake facilitator protein glpF | 0.132 |
| 852 | tetrahydrodipicolinate succinylase | 3.348 | **1632** | **ssdh** | **5.685** |
| 853 | amino acid amidohydrolase | 2.961 | 1665 | oligopeptide ABC trans. substrate binding | 2.936 |
| 854 | dihydrodipicolinate synthase | 3.637 | **1699** | **exodeoxyribonuclease** | **7.479** |
| 855 | dihydrodipicolinate reductase | 2.390 | **1743** | **Cell wall-associated hydrolase** | **0300** |
| **877** | **PTS** system **IIa** | **2.562** | **1744** | **Putative glycosidase** | **0.232** |
| 882 | transc. regulator (GntR family) | 2.163 | **1768** | **IctP lactate premease** | **0.288** |
| 892 | bile salt hydrolase | 1.815 | 1812 | alpha-glucosidase II | 2.952 |
| **911** | **aminopeptidase** | **3.671** | 1821 | **ABC transporter,** ATPase **component** | **0.362** |
| 913 | peroxidase | 1.988 | **1822** | **ABC transporter, ATPase component** | **0.399** |
| **925** | **transcr. regulator** | **2.036** | **1848** | **di-/tripeptide transporter** | **0.272** |
| 927 | Putative membrane prot. | 0.363 | **1869** | **beta-phosphoglucomutase** | **6.740** |
| **953** | **nucleotide phosphodiesterase** | **0.430** | **1870** | **maltose phosphorylase** | **10.17** |
| **986** | **Galactose mutarotase related** | **3.146** | 1879 | phosphomethylpyrimidine kinase | 2.192 |
| **994** | **aminoacyl-histidine dipeptidase** | **0.326** | 1883 | probable NLP/P60 family secreted protein | 0.388 |
| **995** | **amino acid permease** | **0.257** | **1893** | **GMP reductase** | **0.478** |
| **999** | **Na+-transporting ATP synthase** | **2.293** | **1910** | **ATP-dependent protease ClpE** | **2.571** |
| 1027 | oxidoreductase | 2.381 | **1943** | **Putative lipoprotein A/antigen precursor** | 0.444 |
| 1042 | ABC transporter permease | 3.010 | 1945 | sugar ABC transporter permease | 0.426 |
| 1044 | abc transporter | 2.670 | **1974** | pyruvate oxidase | **4.304** |
| 1045 | ABC transporter ATP-binding | 4.866 | **1999** | **glycyl-tRNA synthetase alpha chain** | **0.444** |

| | | | | | |
|---|---|---|---|---|---|
| ^{a}ORFs shown in bold in table 2 exhibit a similar expression pattern to a previous microarray analysis of *L*. *acidophilus* NCFM exposed to MRS acidified to pH 4.5 (3). | | | | | |

Microarray analysis *of L. acidophilus* NCFM exposed to adhesion adaptive conditions (Table 3) suggests increased production of an interspecies signal, autoinducer-2 (AI-2). The initial (*metK*) and final (*luxS*) genes in the pathway for the production of AI-2 from methionine (see Figure 4) are significantly induced. Additionally, an ABC transporter cluster putatively involved with the export of AI-2 was induced.

### Autoinducer-2 biosynthetic pathway

Further analysis of the genome of *L. acidophilus* NCFM revealed four ORFs (LBA1622, LBA0931, LBA0820, LBA1081) showing homology to each gene in the biosynthetic pathway for the production of AI-2 from methionine (Figure 4). MetK converts methionine to S-adenosylmethionine (SAM) and is putatively coded by LBA1622 (SEQ ID NO:21). A methyl group is removed from SAM by an SAM-dependent methyltransferase (encoded by LBA931, SEQ ID NO: 3) forming S-adenosylhomocysteine (SAH) which is subsequently detoxified by an MTA/SAH nucleosidase, Pfs (encoded by LBA820, SEQ ID NO:1), forming S-ribosylhomocysteine (SRH) and adenine. LuxS (encoded by LBA1081, SEQ ID NO:15) converts SRH to homocysteine and 4,5-dihydroxy 2,3-pentanedione which circularizes, incorporating boron, to form AI-2 (Winzer et al. (2002) Microbiology 148:909-922). Homocysteine is finally methylated back to methionine by MetE (encoded by LBA1080, SEQ ID NO:13) located directly upstream of the *luxS* homolog; LBA1081. A terminator between the two ORFs with a free energy of -16.2 kcal suggests that LBA1084 and LBA1081 are expressed separately.

Table 4 lists other lactobacilli, for which genomic sequence data is available, with the complete pathway for the production of AI-2 from methionine.

**Table 4**

| Organism | *nietK* | Methyl-transferase | *pfs* | *luxS* |
|---|---|---|---|---|
| *L. acidophilus* NCFM | 2 | 2 | 1 | 2 |
| *L. gasseri* | 1 | 5 | 1 | 1 |
| *L. johnsonii* | 1 | 6 | 1 | 1 |
| *L. plantarum* | 1 | 2 | 1 | 1 |

The number of predicted ORFs coding for proteins exhibiting similarity to each enzyme in the pathway for synthesis of AI-2 from methionine are listed for *L*. *acidophilus* NCFM (Altermann et al.(2005) Proc. Natl. Acad. Sci. USA, 102:3906-3912), *Lactobacillus gasseri, Lactobacillus johnsonii* NCC533 (Pridmore et al. (2004) Proc. Natl. Acad Sci. USA 101:2512-2517), and *Lactobacillus plantarum* WCFS1 (Kleerebezum et al. (2003) Proc. Natl. Acad. Sci. USA 100: 1990-1995). *L*. *acidophilus* NCFM is predicted to harbor two copies of *luxS,* but one of the ORFs does not contain expression signals such as promoter or ribosomal binding regions.

### AI-2 Production

The detection of autoinducer-2 from the supernatant of selected bacterial strains was performed as described previously (DeKeersmaecker and Vanderleyden, 2003, *supra)* with modifications as follows. The detection of AI-2 produced by lactic acid bacteria can be problematic due to the decreased pH of the spent culture supernatants, and catabolite repression by glucose of the lux operon in the reporter strain *V. harveyi* BB170 (DeKeersmaecker and Vanderleyden, 2003, *supra).* Accordingly, all *Lactobacillus* populations used for detection of autoinducer-2 were grown in modified MRS (mMRS) containing 1% galactose rather than glucose. At specified time points, 4 ml aliquots were collected, OD₆₀₀ measured, and cell-free supernatants isolated by centrifugation at 4,000 x g for 10 min. The pH of the supernatant was neutralized to pH 6.5 with 2N NaOH and filtered through a 0.2 µm membrane. Prepared supernatants were stored at 4°C until the end of the time course experiments and all samples from each culture were assayed together in separate 96-well microtiter plates. The reporter strain *V. harveyi* BB170 (Azcarate-Peril, 2005, *supra)* was grown overnight in autoinducer bioassay (AB) media, washed with and resuspended in fresh AB to OD₆₀₀ 0.5. Ten µl of sterile supernatant was mixed with 90 µl of a 1/1000 dilution of the reporter strain BB 170 in each well of a 96-well plate. Luminescence was measured at 30°C every 10 min for 6 h in a fluorescent microtiter plater reader (FLOUStar Optima, BMG Technologies, Durham, NC). Fold induction was calculated by averaging at least 6 standard time points and dividing the average value obtained from the wild type by the average value obtained from the LuxS⁻ mutant. Each sample was measured in at least 3 independent wells and each data point represents 3 independent samples.

In order to determine when AI-2 was produced during the growth phase of *L*. *acidophilus* NCFM, supernatants were harvested at 0, 3, 6, 9, 12, 16, 20, 24, 36, and 48 hrs from triplicate cultures. AI-2 production during the growth of *L. acidophilus* is shown in figure 5. Coinciding with a rapid drop in pH, a noticeable increase in AI-2 production occurs throughout the logarithmic phase of growth. Upon entrance into stationary phase the AI-2 activity in the supernatant maintains a constant level up to 48 hrs (Figure 5).

### Insertional inactivation of luxS gene

Using *L. acidophilus* NCFM chromosomal DNA as a template, a 471 bp internal fragment of LBA1081 (*luxS*) was amplified using PCR with primers 1081-IF (5'-GATCA GATCT AAGTT AAGGC ACCTT ACG-3', SEQ ID NO:23) and 1081-IR (5'-GATCT CTAGA TTTCG AATGG GTCAT CAC-3', SEQ ID NO:24). The internal fragment was cloned onto the integrative vector pORI28 (Law, et al. (1995) J. Bacteriol. 177:7011-7018) and subsequently transformed by electroporation into *L*. *acidophilus* NCFM containing the temperature sensitive helper plasmid pTRK669 (Russell and Klaenhammer, 2001, *supra*)*.* Steps were then carried out according to Russell and Klaenhammer, 2001, *supra* for selection of integrants. Successful integration of the plasmid was confirmed by PCR and Southern hybridization analysis of junction fragments using standard protocols.

The LuxS⁻ mutant strain, NCK 1765, was tested for the ability to adhere to Caco-2 cells using bacterial cells from mid-log phase of growth and cells exposed to AAR conditions. When the mid-log phase cells were added directly to the Caco-2 cells from mid-log phase, a 58% decrease in adhesion (Student's t-test, *P* < 0.001) was observed for the LuxS⁻ mutant strain (Figure 6) compared to the control. A derivative *of L. acidophilus* NCFM (NCK1398) harboring an integration in a β-galactosidase was used as the control so that antibiotic pressure could be maintained on the mutant and control.

### Conclusions

*L. acidophilus* NCFM responds to environmental conditions in a manner that dramatically increases the ability of the organism to adhere to intestinal epithelial cells, *in vitro.* Transcriptional microarray analysis of a population exposed to these adhesion adaptive conditions suggests that the production of a signaling molecule, AI-2, is increased. Analysis of the genome *of L. acidophilus* NCFM identified all genes necessary for the production of AI-2 from methionine. Production of AI-2 from *L*. *acidophilus* NCFM was confirmed using a *V. harveyi* reporter strain. Upon inactivation of the final gene in the pathway, *luxS,* a decrease in adhesion was observed in log phase cells, but no difference was observed in the adhesion adaptive response of the LuxS- mutant strain compared to the control. These data suggest that adhesion of lactobacilli to the intestinal epithelium may involve an intimate interplay of various factors. While AI-2 mediated signaling contributes to the adhesive ability *of L. acidophilus* NCFM, it is not the exclusive means of communication within the population responsible for the adhesion adaptive response. These data suggest that adhesion of lactobacilli to the intestinal epithelium may involve an intimate interplay of various factors. While AI-2 mediated signaling contributes to the adhesive ability of *L. acidophilus* NCFM, it is not the exclusive means of communication within the population responsible for the adhesion adaptive response.

### Example 2

Microarray analysis of gene expression affected by LuxS and AI-2 has been performed on a number of different microbial species in order to determine the function of LuxS on cellular processes. The influence of AI-2 on *E*. *coli* K-12 grown in the presence and absence of glucose revealed altered gene expression of genes related to the *lsr* operon, methionine metabolism, and carbon utilization (12, 32). Another study linked LuxS production with genes involved in cellular growth and division using *E*. *coli* microarrays (9). Transcriptional microarray analysis of a LuxS⁻ mutant of *Porphyromonas gingivalis* and its wild type implicated the participation of LuxS with the stress response of that organism (36). In addition to pathogenic bacterial strains, several non-pathogenic strains have demonstrated a phenotype associated with AI-2 production. For example, the ecological performance of *Lactobacillus reuteri* in the murine gastrointestinal tract was altered in a LuxS⁻ mutant (28). This study utilized a whole genome microarray to identify gene expression influenced by LuxS in *L. acidophilus* NCFM. Multiple genes involved with the stress response, growth, and metabolism were differentially expressed in a LuxS- mutant strain. Phenotypic analysis of a LuxS- mutant strain examined the influence of a *LuxS* mutation on the heat and bile tolerance *of L. acidophilus* NCFM.

### Bacterial strains and growth conditions.

*Lactobacillus* strains were cultivated anaerobically at 37°C or 42°C in MRS broth (Difco Laboratories Inc., Detroit MI) or, when appropriate, in MRS supplemented with 1.5% agar. For detection of AI-2, lactobacilli were grown in modified MRS, following the ingredients for MRS except substituting 1 % w/v galactose in place of glucose. *E. coli* was grown aerobically in Luria-Bertani (LB, Difco) medium or on LB medium supplemented with 1.5 % agar at 37°C. Brain Heart Infusion (BHI, Difco) medium supplemented with 1.5 % agar and 150 µg/ml erythromycin (Em) was used for selection of *E*. *coli* transformants. Autoinducer Bioassay (AB) media (5) was used for the propagation of all *Vibrio harveyi* strains. When appropriate, chloramphenicol (Cm, 5.0 µg/ml) and Em (5.0 µg/ml or 150 µg/ml) were used for selection. Colony forming units (CFU) per ml were determined with appropriate dilutions in 10 % MRS broth using a Whitley Automatic Spiral Plater (Don Whitley Scientific Limited, West Yorkshire, England).

**Table 5. Bacterial strains and plasmids used**

| **Strains** | **Origin or relevant characteristics** | **Source or reference** |
|---|---|---|
| *L. acidophilus* | | |
| NCFM | Human intestinal isolate | (24) |
| NCK 1392 | NCFM containing pTRK669 | (22) |
| NCK 1818 | NCFM containing luxS deletion | This study |
| NCK 1758 | NCFM with insertionally inactivated *labT* | (10) |
| NCK 235 | *Lactobacillus delbruekii* used as indicator strain in bacteriocin assay | ATCC 4797 |
| *V. harveyi* | | |
| BB170 | *luxN*.:Tn5 AI-1 sensor⁻ AI-2 sensor⁺ | (26) |
| *E. coli* | | |
| EC1000 | RepA⁺ MC1000, Km^{r}, carrying a single copy of the pWV01 *repA;* host for pOR129-based plasmids | (18) |
| Plasmids | | |
| pORI28 | Em^{r}, ori (pWV01), replicates only with *repA* provided in *trans* | (18) |
| pTRK669 | ori (pWV01), Cm^{r}, RepA⁺ | (22) |
| pTRK884 | pOR128 ligated to *luxS* containing a 97 bp deletion and introduced EcoRI restriction site | This study |

### DNA manipulation techniques.

Total *Lactobacillus* genomic DNA was isolated according to the method of Walker and Klaenhammer (31). Standard protocols were used for endonuclease restriction, ligation, DNA modification and transformation (23). Plasmid preparations for the purpose of screening *E. coli* transformants followed the method of Zhou *et al.* (37). Large scale plasmid preparations were performed with the QIAprep Spin kit according to the manufacturer's instructions (QIAGEN Inc., Valencia, CA). PCRs were carried out according to manufacturer's recommendations using a *Taq* DNA polymerase PCR system (Roche Applied Science, Indianapolis, IN). PCR primers were synthesized by Integrated DNA Technologies (Coralville, IA) and, when appropriate, restriction sites were designed into the 5' end of the primers to facilitate future cloning steps. DNA fragments were extracted from 1.0 % agarose gels using the Zymoclean Gel DNA Recovery kit (Zymo Research, Orange, CA). Electrocompetent *Lactobacillus* cells were prepared as described by Walker et al. (30). Southern hybridization of genomic DNA was carried out using standard protocols.

**Table 6. Primers used in this study**

| Primer | Sequence^{a} |
|---|---|
| LuxS-EF | 5'- GATCTCTAGATGACAGAAGACGATGAATG -3' (SEQ ID NO:25) |
| LuxS-ER | 5'- GATCAGATCTATTGCGACTAAGTTCAGAC -3' (SEQ ID NO:26) |
| LuxS-delF | 5'- GATCGAATTCTCGTTCGGTTGAACTAAACGTAAGTC -3' (SEQ ID NO:27) |
| LuxS-delR | 5'- GATCGAATTCCGGAACAGGATTCCACCTAATCGTTTG -3' (SEQ ID NO:28) |
| LuxS-XXF | 5'- GCCAACTTAGCCTTAAGCACTC -3' (SEQ ID NO:29) |
| LuxS-XXR | 5'- TTGTTCCTGCTCCTCAGCCTTC -3' (SEQ ID NO:30) |
| LuxS-delNF | 5'- TGCTITAGCAACTICAGTAG -3' (SEQ ID NO:31) |
| LuxS-delNR | 5'- TAAAGTTAAGGCACCTTACG -3' (SEQ ID NO:32) |

| | |
|---|---|
| ^{a}Restriction enzyme sites used for donning are underlined. | |

### Marker free inactivation of LuxS.

A LuxS⁻ mutant strain *of L. acidophilus* NCFM was constructed by deletion of 97 bp within *luxS* (LBA1081) according to the protocols described previously (2, 6). Briefly, a 1,243 bp fragment containing luxS was amplified using primers LuxS-EF and LuxS-ER (Table 6). That fragment was subsequently ligated to pORI28, at the XbaI and BglII sites. Transformants were sleected in *E*. *coli* EC1000 and pORI::LuxSbig was isolated. Inverse PCR (Expand High Fidelity PCR system, Roche Applied Science, Indianapolis, IN) was performed on that plasmid using primers LuxS-delF and LuxS-delR (Table 6) each containing EcoRI enzyme recognition sites at the 5' end. The resulting 2.7 kbp PCR product was cleaned using the Qiagen PCR purification kit and digested for 18 hrs at 37°C with EcoRI. Following gel purification with the Zymoclean Gel DNA Recovery Kit (Zymogen) the product was ligated to itself for 16 hrs at 16 °C. The resulting plasmid, pTRK884, was then transformed into *E*. *coli* EC1000, and plasmid construction confirmed with PCR amplification. *L. acidophilus* NCFM containing the temperature sensitive helper plasmid, pTRK669, was then electrotransformed with pTRK884 and integrants selected according to the method previously described by Russell and Klaenhammer (22). Selected integrants were passed six times in non-selective media, to allow the second cross-over event to occur, and then plated on MRS devoid of antibiotics. Colonies were screened for Em sensitivity and colony PCR conducted using primers LuxS-deINF and LuxS-delNR, flanking the deleted region. PCR amplification of the deletion region and Southern hybridization assays using an internal fragment of luxS as the probe confirmed the deletion via gene replacement in NCK1818.

Until recently, site-specific gene inactivation in lactobacilli has been performed using plasmid integration strategies. Maintenance of plasmid integration requires growth in selective media. However, both plasmid integration and selective growth may influence gene expression results obtained by microarray analysis. As such, a marker-free strategy was used to inactivate *luxS* (LBA1081) in *L. acidophilus* NCFM using a double-crossover homologous recombination approach. The *luxS* gene of the resulting mutant strain, NCK1818, lacked a 97 bp internal region, and contained an additional EcoRI restriction site. The deletion region was selected to disrupt the reading frame and result in a non-functional gene product. Gene replacement was confirmed by PCR analysis of the deleted region and Southern hybridization using an internal fragment of *luxS* as the probe. NCK1818 was Em sensitive and did not produce AI-2 as determined by the *V. harveyi* reporter assay (5). Previously, the pattern of AI-2 expression by *L. acidophilus* NCFM was determined using a *luxS* integration mutant as the negative control (8). The same pattern of AI-2 production during the growth of *L. acidophilus* NCFM was observed using NCK1818 as the negative control.

### AI-2 detection.

Detection of autoinducer-2 from the supernatant of selected bacterial strains was performed as described previously (8). Briefly, the supernatants from bacterial populations harvested at each specified time point were adjusted to pH 6.5 with 2N NaOH and filtered through a 0.2 µm membrane. The reporter strain *Vibrio harveyi* BB170 (3) was grown overnight in autoinducer bioassay (AB) media, washed with and resuspended in fresh AB to OD₆₀₀ 0.5. Ten µl of sterile supernatant from the test culture was mixed with 90 µl of a 1/1000 dilution of the reporter strain BB170 in each well of a 96-well plate. Luminescence was measured at 30°C every 10 min for 6 h in a fluorescent microtiter plate reader (FLOUStar Optima, BMG Technologies, Durham, NC). Fold induction was calculated by averaging at least 6 standard time points from the *V. harveyi* luminescence curve and dividing the average value obtained from the wild type by the average value obtained from the LuxS⁻ mutant. Time points were selected when luminescence was at a constant level before the reporter strain responded to the production of its own AI-2. Each sample was measured in at least 3 independent wells and each data point represents 2 independent experiments (Table 7).

### RNA Isolation.

Both *L. acidophilus* NCFM and NCK1818 were transferred two times from frozen stock cultures in semi-defined media using galactose as the primary carbon source to prevent catabolite repression by glucose of the lux operon in the *V. harveyi* reporter strain. A 400 ml culture of both strains was inoculated at 1 % from a stationary phase (16 hr) culture and allowed to grow at 37°C. At OD₆₀₀ 0.2, a 5 ml sample was obtained for CFU determination and pH measurement. Six 10 ml samples of each strain were then quick cooled on dry ice, and cell pellets harvested at 4°C by centrifugation for 10 min at 3,150 x g. The supernatant from each strain following centrifugation at every time point was retained for AI-2 detection. At the final two sampling points (OD₆₀₀ 0.7 and OD₆₀₀1.2) 5 ml samples were collected from each culture for plating and pH determination; two 10 ml samples were then harvested from each culture and treated as described above. RNA isolation from cell pellets was conducted as described previously (3) using TRIzol (Invitrogen Life Technologies, Rockville, MD). RNA purity and concentration were determined by electrophoresis on 1.0 % agarose gels and standard spectrophotometer measurements. RNA was isolated from two biological replicates of both strains.

**Table 7 Culture conditions of cell populations harvested for microarrav analysis^{a}**

| OD₆₀₀ | CFU/ml (±SD) | AI-2 activity (±SD) | pH (±SD) |
|---|---|---|---|
| 0.2 | 3.26 x 10⁶ (±4.27 x 10⁵) | 2.27 (±0.09) | 6.08 (±0.04) |
| 0.7 | 2.67 x 10⁷ (±3.13 x 10⁶) | 10.78 (±0.60) | 5.35 (±0.13) |
| 1.2 | 7.81 x 10⁷ (±1.22x10⁷) | 30.13 (±0.25) | 4.89 (±0.12) |

| | | | |
|---|---|---|---|
| ^{a}All values represent the mean and one standard deviation calculated from combined data obtained from both *L. acidophilus* NCFM and NCK1818 in three independent experiments. | | | |

### cDNA Production and Microarray Hybridization.

Gene expression analysis of the LuxS⁻ mutant strain and the wild type strain at each of the three selected sampling points was performed using a whole-genome DNA microarray (3). PCR products of 1,889 predicted ORFs from the *L. acidophilus* NCFM genome (1) were spotted in triplicate on ULTRA GAPS glass slides (Corning Inc., Acton, MA). First strand cDNA synthesis and labeling were performed with the SuperScript Indirect cDNA Labeling System (Invitrogen). Briefly, equal amounts (20 µg) of RNA were aminoallyl-labeled by reverse transcription with random hexamers in the presence of amino-modified nucleotides, using SuperScript III reverse transcriptase at 42°C for 3 hrs. First-strand cDNA was purified with S.N.A.P columns, precipitated, labeled with N-hydroxysuccinimide-activated Cy3 or Cy5 esters (GE Healthcare Life Sciences, Piscataway, NJ), and purified again with S.N.A.P columns. The resulting labeled cDNA was hybridized onto microarray slides according to the protocols outlined by TIGR (www.tigr.org/tdb/microarray/protocolsTIGRshtml). Hybridizations were performed according to a single round-robin pattern, so that all possible direct pair-wise comparisons were conducted in an unbalanced design. A total of fifteen different hybridizations were performed at three different sampling points (OD₆₀₀ 0.2, 0.7, and 1.2) and two different strains (*L. acidophilus* NCFM and NCK1818).

### Microarray Data Analysis.

Fluorescence intensities were acquired using a General Scanning ScanArray 4000 Microarray Scanner (Packard Biochip BioScience, Biochip Technologies LLC, Mass.) and processed as TIFF images. Signal intensities were quantified, including background subtraction, and spot replicates averaged using the QuantArray 3.0 software package (Packard BioScience). The resulting raw intensity data was imported into SAS (SAS Institute, Cary, NC), log₂ transformed and fit to the normalization mixed model analysis of variance (ANOVA) in order to center the data to the mean intensity. A gene-specific mixed model ANOVA was then performed on the normalized data in which dye, strain, and time were considered fixed effects and the array effect was considered a random effect (34). The resulting difference between least-square estimates for two different treatments is analogous to log₂-transformed ratio of gene expression between those two treatments. Differences were calculated for dye effect, strain effect, time effect, and the combined effect of strain and time, strain*time. A *t* test was performed using these differences and their standard errors, with *P* < 0.05 considered significant. Volcano plots were constructed for each comparison using the estimate and Log₁₀(P value) with JMP 5.0 (SAS Institute) in order to visualize contrast between treatments and statistical significance of the results.

In order to study the genes differentially expressed in response to LuxS production, a microarray design was developed that examined the differences in expression between the wild type and LuxS⁻ mutant in the early, middle, or late-exponential growth phase. AI-2 production during growth was previously determined for *L. acidophilus* NCFM (8). From that data, three growth points were selected for RNA isolation: OD₆₀₀ 0.2, before AI-2 has accumulated; OD₆₀₀ 0.7, during the rapid production of AI-2; and, OD₆₀₀ 1.2, when production of AI-2 slows and the level of AI-2 in the supernatant remains elevated but constant. RNA was isolated from both strains at each time point, in duplicate, and AI-2 activity determined using the *V. harveyi* reporter assay. In total, six points were compared against each other in every possible pair of comparisons (15 hybridizations). Analysis of expression data was performed using a two-stage mixed model ANOVA and least square mean ratios considered significant at a fold change of 1.8 and P < 0.05 (Table 9).

Each differentially expressed ORF at the first two time points were grouped according to their COG classification (Fig. 8). The two COG classifications with the highest number of overexpressed genes in NCFM, accounting for 42% of the total, were translation ribosomal structure and biogenesis (J), and replication, recombination, and repair (L). In contrast, 40% of the overexpressed genes in the LuxS⁻ mutant were categorized as function unknown (S). The increased expression of ) genes related to normal growth and metabolism in NCFM compared to the LuxS⁻ mutant suggests that LuxS positively influences the expression of many of these genes either directly or indirectly. It is not clear from these data the function of the genes whose expression was negatively influenced by LuxS. There were no significantly differentially expressed genes under the conditions tested in either NCFM or the LuxS⁻ mutant at late-log phase (OD₆₀₀ 1.2), when the level of AI-2 in the media is no longer increasing.

A round robin microarray design combined with mixed model analysis was used to compare the effect of strain, time and the combined effect of both strain and time (strain*time) on the gene expression of *L. acidophilus* NCFM. A strain to strain comparison revealed that, before AI-2 began to appreciably accumulate in the media (OD₆₀₀ 0.2), 84 ORFs were significantly overexpressed in NCFM (Table 8a) while only 13 ORFs were significantly overexpressed in the LuxS⁻ mutant (Table 8b). A The most highly induced genes in NCFM compared to the mutant at early-log phase (OD₆₀₀ 0.2) were *relA* (3.14 fold), putatively related to signal transduction, and IF-2 (3.14 fold), a putative translation initiation factor. At mid-log phase (OD₆₀₀ 0.7), as AI-2 was rapidly accumulating, only 3 ORFs were significantly overexpressed in NCFM (LBA1497, LBA1796, LBA1798) and 4 ORFs (LBA0026, LBA0089, LBA0568, and LBA1596) overexpressed in the LuxS- mutant. Interestingly, the only ORF that was significantly differentially expressed at both of the first two time points was *labT* (LBA1796), an ABC-transporter previously implicated with bacteriocin export (10). At early-log phase, *labT* was overexpressed in NCFM 2.53 fold, and at mid-log it was overexpressed 2.10 fold compared to the LuxS⁻ mutant. A response regulator (LBA1798), of a neighboring two component regulatory system, was also overexpressed in NCFM compared to the mutant at mid-log phase. In this regard, the LuxS⁻ strain was analyzed for bacteriocin production and found to elicit the same level of bacteriocin production as the wild type. Additionally, an isogenic *labT* mutant strain was tested for AI-2 production and found to produce AI-2 at the same level as the wild-type.

**Table8a. COG classification of differentially expressed genes**

| **Genes induced in *L. acidophilus* NCFM at early-log phase (OD₆₀₀ 0.2)** | | | | | |
|---|---|---|---|---|---|
| Gene Annotation^{a} | ORF | Ratio^{b} | Gene Annotation | ORF | Ratio |

| *Energy production and conversion* | | | | | |
|---|---|---|---|---|---|
| F1F0-ATPase subunit a | LBA 0772 | 1.92 | F1F0-ATPase subunit d | LBA 0775 | 1.98 |
| F1F0-ATPase subunit c | LBA 0773 | 1.99 | *lctP* | LBA 1768 | 1.97 |

| *Cell cycle control, cell division, chromosome partitioning* | | | | | |
|---|---|---|---|---|---|
| *spoIIIe* | LBA 0659 | 1.95 | *epsC* | LBA 1735 | 1.85 |
| *ftsZ* | LBA 0812 | 2.18 | | | |

| *Amino acid transport and metabolism* | | | | | |
|---|---|---|---|---|---|
| *asd2* | LBA 0857 | 1.81 | *pepP* | LBA 1336 | 2.50 |
| *pepT* | LBA 1190 | 2.28 | | | |

| *Nucleotide transport and metabolism* | | | | | |
|---|---|---|---|---|---|
| deoxyribose-p aldolase | LBA 0391 | 2.24 | UMP kinase | LBA 1268 | 2.46 |

| *Carbohydrate transport and metabolism* | | | | | |
|---|---|---|---|---|---|
| phosphoglucomutase | LBA 0687 | 1.91 | *treC* | LBA 1014 | 2.56 |

| *Lipid transport and metabolism* | | | | | |
|---|---|---|---|---|---|
| *thil* | LBA 0626 | 2.07 | *fabG* | LBA 0662 | 2.28 |
| *hmdH* | LBA 0627 | 2.15 | | | |

| *Translation, ribosomal structure and biogenesis* | | | | | |
|---|---|---|---|---|---|
| trprs | LBA 0209 | 1.94 | *hemK* | LBA 0768 | 2.42 |
| meth-trna synthetase | LBA 0213 | 2.15 | pseudouridylate synthase | LBA 0791 | 1.85 |
| ribosomal protein S17 | LBA 0300 | 2.24 | trna synthetase | LBA 0817 | 1.93 |
| ribosomal protein L14 | LBA 0301 | 2.34 | *gidA* | LBA 0982 | 2.00 |
| ribosomal protein L24 | LBA 0302 | 2.53 | tRNA ligase | LBA 1198 | 1.82 |
| ribosomal protein L36 | LBA 0314 | 2.24 | *nf* | LBA 1267 | 1.89 |
| ribosomal protein S13 | LBA 0315 | 2.02 | *miaA* | LBA 1503 | 1.94 |
| tRNA pseudouridine synthetase | LBA 0322 | 2.12 | *pheS* | LBA 1518 | 2.31 |
| *ampM* | LBA 0623 | 1.93 | *leuS* | LBA 1617 | 1.80 |
| *pcrf I* | LBA 0767 | 2.75 | GTP-binding protein | LBA 1824 | 2.20 |

| *Transcription* | | | | | |
|---|---|---|---|---|---|
| *gntR_* | LBA 0393 | 2.09 | *nusA* | LBA 1259 | 1.88 |
| fibronectin binding prot. | LBA 1148 | 1.84 | *parB* | LBA 1828 | 2.52 |

| *Replication, recombination and repair* | | | | | |
|---|---|---|---|---|---|
| *recF* | LBA 0004 | 1.88 | DNA primase | LBA 1197 | 2.66 |
| *gyrA* | LBA 0006 | 1.89 | transposase | LBA 1464 | 2.33 |
| DNA polymer. III | LBA 0376 | 1.89 | transposase | LBA 1487 | 1.92 |
| *dnlJ* | LBA 0529 | 1.89 | *dnaB* | LBA 1545 | 2.97 |
| *uvrB* | LBA 0688 | 2.35 | *mutM* | LBA 1549 | 1.90 |
| *radC* | LBA 0797 | 2.02 | transposase | LBA 1570 | 2.11 |
| *uvrC* | LBA 0946 | 1.96 | RNA helicase | LBA 0416 | 2.06 |
| DNA topoisomerase | LBA 0981 | 1.87 | *mutT* family protein | LBA 0819 | 2.89 |
| DNA topo.sub.B | LBA 1122 | 2.89 | | | |

| *Cell wall, membrane, and envelope biogenesis* | | | | | |
|---|---|---|---|---|---|
| *mraW* | LBA 0803 | 2.04 | *epsB* | LBA 1736 | 1.98 |
| *murG* | LBA 0809 | 2.16 | *gidB* | LBA 1829 | 1.90 |
| *bshA* | LBA 0892 | 1.94 | *dltD* | LBA 1923 | 2.29 |
| *eep* | LBA 1263 | 2.23 | | | |

| *Posttranslational modification, protein turnover, chaperones* | | | | | |
|---|---|---|---|---|---|
| *clpC* | LBA 0283 | 2.05 | *hslV* | LBA 0984 | 1.88 |
| *radA* | LBA | 2.04 | *dnaK* | LBA | 1.95 |
| | 0346 | | | 1247 | |
| *clpX* | LBA 0847 | 2.49 | *grpE* | LBA 1248 | 2.97 |

| *Signal transduction mechanisms* | | | | | |
|---|---|---|---|---|---|
| *luxS* | LBA 1081 | | *relA* | LBA 0932 | |
| *phoH* | LBA 1203 | | | | |

| *Defense mechanisms* | | | | | |
|---|---|---|---|---|---|
| *labT* | LBA 1796 | 2.53 | permease | LBA 1839 | 2.36 |

| *General Function or Function Unknown* | | | | | |
|---|---|---|---|---|---|
| putative transport protein | LBA 0635 | 2.07 | dihydroacetone kinase | LBA 1310 | 1.81 |
| putative hydrolase | LBA 0796 | 1.97 | helicase | LBA 1676 | 2.39 |
| GTP binding protein | LBA 0947 | 2.46 | ABC transporter | LBA 1783 | 2.25 |
| oxidoreductase | LBA 0950 | 2.39 | serine/threonine prot phase | LBA 1317 | 1.85 |
| phosphoglycerate dehydrogenase | LBA 0969 | 2.05 | hypothetical | LBA 1191 | 2.04 |
| O-GlcNAc transferase | LBA 0971 | 1.85 | conserved hypothetical protein | LBA 1204 | 2.10 |
| conserved hypothetical protein | LBA12 02 | 2.70 | hypothetical | LBA 1823 | 1.88 |

| | | | | | |
|---|---|---|---|---|---|
| *^{a}* Putative identification by manual annotation for that gene or ORF *^{b}* Least square means ratio of log₂ estimates between wild type *L. acidophilus* NCFM and the LuxS⁻ mutant strain. The cutoff ratio for the fold difference was 1.8 and *P* < 0.05. | | | | | |

**Table 8b. COG classification of differentially expressed genes**

| **Genes induced in *L. acidophilus* NCFM compared to NCK1818 at mid-log phase (OD₆₀₀ 0.7)** | | |
|---|---|---|
| Gene Annotation*^{a}* | ORF | Ratio*^{b}* |

| *Cell cycle control, cell division, chromosome partitioning* | | |
|---|---|---|
| unknown | LBA 1497 | 1.94 |

| *Transcription* | | |
|---|---|---|
| response regulator | LBA 1798 | 1.92 |

| *Defense mechanisms* | | |
|---|---|---|
| *labT* | LBA 1796 | 2.11 |

| **Genes repressed in *L. acidophilus* NCFM compared to NCK1818 at early-log phase (OD₆₀₀ 0.2)** | | |
|---|---|---|
| *Cell cycle control, cell division, chromosome partitioning* | | |
| hypothetical | LBA 1156 | 0.53 |

| *Amino acid transport and metabolism* | | |
|---|---|---|
| *ansA* | LBA 1687 | 0.51 |

| *Translation, ribosomal structure and biogenesis* | | |
|---|---|---|
| rib. prot. L11 | LBA 0359 | 0.52 |
| seryl-trna synthetase | LBA 1626 | 0.52 |
| kanamycin kinase | LBA 1348 | 0.54 |

| *Posttranslational modification, protein turnover, chaperones* | | |
|---|---|---|
| thioredoxin reductase | LBA 0422 | 0.51 |

| *Inorganic ion transport and metabolism* | | |
|---|---|---|
| K+ uptake protein | LBA 0166 | 0.47 |
| ABC transporter | LBA 0154 | 0.54 |

| *Secondary metabolites biosynthesis, transport and catabolism* | | |
|---|---|---|
| hypothetical | LBA 0644 | 0.53 |

| *Intracellular trafficking, secretion, and vesicular transport* | | |
|---|---|---|
| hypothetical | LBA 0448 | 0.52 |

| *General function or function unknown* | | |
|---|---|---|
| hypothetical | LBA 0031 | 0.50 |
| cons. hypothetical | LBA 0217 | 0.50 |
| hypothetical | LBA 0690 | 0.53 |
| unknown | LBA 1127 | 0.53 |
| *lysM* | LBA 1850 | 0.51 |
| cons. hypothetical | LBA 0100 | 0.55 |

| **Genes repressed in *L. acidophilus* NCFM compared to NCK1818 at mid-log phase (OD₆₀₀ 0.7)** | | |
|---|---|---|
| *Transcription* | | |
| *cadX* | LBA 0022 | 0.55 |
| *dinG* | LBA 1164 | 0.55 |

| *General function or function unknown* | | |
|---|---|---|
| malate permease | LBA 0568 | 0.49 |
| hypothetical | LBA 0026 | 0.53 |
| cons. hypothetical | LBA 0089 | 0.50 |
| unknown | LBA 0883 | 0.55 |
| unknown | LBA 1596 | 0.51 |

| | | |
|---|---|---|
| *^{a}* Putative identification by manual annotation for that gene or ORF *^{b}* Least square means ratio of log₂ estimates between wild type *L. acidophilus* NCFM and the LuxS⁻ mutant strain. The cutoff ratio for the fold difference was 1.8 and P < 0.05. | | |

**Table 9. Microarray Results**

| Log2 ratio | Neglog10 P | ORF | **Induced by LuxS at OD 0.2** | COG Classification |
|---|---|---|---|---|
| 0.908863 | 2.789058 | 4 | recF_4 | [L] Replication, recombination and repair |
| 0.91857 | 2.412259 | 6 | gyrA_6 | [L] Replication, recombination and repair |
| 0.906325 | 2.535795 | 157 | [hypo] 157 | |
| 0.952818 | 2.560997 | 209 | trprs_209 | [J] Translation, ribosomal structure and biogenesis |
| 1.102485 | 2.964208 | 213 | meth-trna synthetase_213 | [J] Translation, ribosomal structure and biogenesis |
| 1.034335 | 2.704386 | 283 | clpC_283 | [O] Posttranslational modification, protein turnover, chaperones |
| 1.161939 | 2.064488 | 300 | rib. prot. S17_300 | [J] Translation, ribosomal structure and biogenesis |
| 1.226609 | 2.042745 | 301 | rib. prot. L14_301 | [J] Translation, ribosomal structure and biogenesis |
| 1.338589 | 2.269549 | 302 | rib. prot. L24_302 | [J] Translation, ribosomal structure and biogenesis |
| 1.161217 | 2.172414 | 314 | rib. prot. L36_314 | [J] Translation, ribosomal structure and biogenesis |
| 1.013961 | 1.807102 | 315 | rib. prot. S13_315 | [J] Translation, ribosomal structure and biogenesis |
| 1.082782 | 3.417382 | 322 | tRNA pseudouridine synthetase 322 | [J] Translation, ribosomal structure and biogenesis |
| 1.028134 | 3.713038 | 346 | radA_346 | [O] Posttranslational modification, protein turnover, chaperones |
| 0.919267 | 2.837944 | 376 | DNA polymer. III_376 | [L] Replication, recombination and repair |
| 1.16463 | 3.348315 | 391 | deoxyribose-p aldolase 391 | [F] Nucleotide transport and metabolism |
| 1.066688 | 3.273812 | 393 | gntR_393 | [K] Transcription |
| 1.043288 | 2.293597 | 416 | RNA helicase_416 | [L] Replication, recombination and repair[K] Transcription[J] Translation, ribosomal structure and biogenesis |
| 0.916647 | 2.631659 | 529 | dnlJ_529 | [L] Replication, recombination and repair |
| 0.947941 | 2.281472 | 623 | ampM_623 | [J] Translation, ribosomal structure and biogenesis |
| 1.049686 | 2.779361 | 626 | thil_626 | [I] Lipid transport and metabolism |
| 1.102867 | 3.798347 | 627 | hmdH_627 | [I] Lipid transport and metabolism |
| 1.051708 | 3.163317 | 635 | transp. prot._635 | [R] General function prediction only |
| 0.96165 | 2.960769 | 659 | spollle_659 | [D] Cell cycle control, cell division, chromosome partitioning |
| 1.189911 | 2.670923 | 662 | fabG_662 | [I] Lipid transport and metabolism[Q] Secondary metabolites biosynthesis, transport and catabolism[R] General function prediction only |
| 0.934721 | 2.563706 | 687 | phosphoglucomutase_687 | [G] Carbohydrate transport and metabolism |
| 1.23033 | 3.536662 | 688 | uvrB_688 | [L] Replication, recombination and repair |
| 1.459675 | 4.012875 | 767 | pcrf I_767 | [J] Translation, ribosomal structure and biogenesis |
| 1.272925 | 3.062158 | 768 | hemK_768 | [J] Translation, ribosomal structure and biogenesis |
| 0.937439 | 1.518881 | 772 | f1f0-subunit a_772 | [C] Energy production and conversion |
| 0.990013 | 1.729401 | 773 | f1f0-subunit c_773 | [C] Energy production and conversion |
| 0.986363 | 2.065649 | 775 | f1f0-atpase d_775 | [C] Energy production and conversion |
| 0.890224 | 3.276722 | 791 | pseudouridylate synthase 791 | [J] Translation, ribosomal structure and biogenesis |
| 0.97628 | 2.169948 | 796 | [hydrolase]_796 | [R] General function prediction only |
| 1.01383 | 2.563551 | 797 | radC_797 | [L] Replication, recombination and repair |
| 1.027217 | 2.27023 | 803 | mraW_803 | [M] Cell wall_membrane_envelope biogenesis |
| 1.11393 | 3.597361 | 809 | murG_809 | [M] Cell wall_membrane_envelope biogenesis |
| 1.123854 | 2.793074 | 812 | ftsZ__812 | [D] Cell cycle control, cell division, chromosome partitioning |
| 0.947385 | 2.926403 | 817 | trna synthetase_817 | [J] Translation, ribosomal structure and biogenesis |
| 1.532751 | 3.25757 | 819 | [mutT fam.]_819 | [L] Replication, recombination and repair[R] General function prediction only |
| 1.315755 | 2.996801 | 847 | clpX_847 | [O] Posttranslational modification, protein turnover, chaperones |
| 0.85508 | 1.789406 | 857 | asd2_857 | [E] Amino acid transport and metabolism |
| 0.952627 | 1.944032 | 892 | Ser - bile salt hydrolase | |
| 1.653786 | 3.607024 | 932 | relA_932 | [T] Signal transduction mechanisms[K] Transcription |
| 0.971224 | 2.878558 | 946 | uvrC_946 | [L] Replication, recombination and repair |
| 1.296349 | 3.086563 | 947 | GTP binding prot_947 | [R] General function prediction only |
| 1.254893 | 3.559454 | 950 | oxidoreductase_950 | [R] General function prediction only |
| 1.036386 | 2.163564 | 969 | phosphoglycerate dehydrogenase 969 | [R] General function prediction only |
| 0.885552 | 2.936481 | 971 | [O-GlcNAc transferase] 971 | [R] General function prediction only |
| 0.900508 | 2.247997 | 981 | DNA topoisomerase_981 | [L] Replication, recombination and repair |
| 0.996435 | 3.260872 | 982 | gidA_982 | [J] Translation, ribosomal structure and biogenesis |
| 0.910308 | 2.240629 | 984 | hsIV_984 | [O] Posttranslational modification, protein turnover, chaperones |
| 1.353333 | 3.018549 | 1014 | treC_1014 | [G] Carbohydrate transport and metabolism |
| 0.927704 | 2.595697 | 1081 | luxS_1081 | [T] Signal transduction mechanisms |
| 1.531752 | 4.144249 | 1122 | DNA topo.sub.B_1122 | [L] Replication, recombination and repair |
| 0.883132 | 2.452041 | 1148 | fibronectin-binding prot. 1148 | [K] Transcription |
| 1.191554 | 2.814596 | 1190 | pepT_1190 | [E] Amino acid transport and metabolism |
| 1.028381 | 2.091427 | 1191 | hyp. prot. 1191 | [S] Function unknown |
| 1.410457 | 3.521746 | 1197 | DNA primase_1197 | [L] Replication, recombination and repair |
| 0.863943 | 1.567874 | 1198 | tRNA ligase_1198 | [J] Translation, ribosomal structure and biogenesis |
| 1.434644 | 3.023855 | 1202 | [cons. hypo. prot.]_1202 | [R] General function prediction only |
| 1.165812 | 2.784708 | 1203 | phoH_1203 | [T] Signal transduction mechanisms, |
| 1.067024 | 2.314515 | 1204 | [cons. hypo. prot.] 1204 | [S] Function unknown |
| 0.964942 | 2.007406 | 1247 | dnaK_1247 | [O] Posttranslational modification, protein turnover, chaperones |
| 1.572382 | 3.321345 | 1248 | grpE_1248 | [O] Posttranslational modifcation, protein turnover, chaperones |
| 1.654133 | 3.369962 | 1255 | IF2_1255 | [J] Translation, ribosomal structure and biogenesis |
| 0.912588 | 2.132805 | 1259 | nusA 1259 | [K] Transcription |
| 1.159717 | 4.364846 | 1263 | eep_1263 | [M] Cell wall_membrane_envelope biogenesis |
| 0.918947 | 2.23504 | 1267 | rrf_1267 | [J] Translation, ribosomal structure and biogenesis |
| 1.299436 | 2.811626 | 1268 | UMP kinase_1268 | [F] Nucleotide transport and metabolism |
| 0.856016 | 1.950837 | 1310 | dihydroacetone kinase 1310 | [R] General function prediction only |
| 0.883897 | 3.248319 | 1317 | serine/threonine prot. kinase_1317 | [R] General function prediction only[T] Signal transduction mechanisms[K] Transcription[L] Replication, recombination and repair |
| 1.31915 | 3.478031 | 1336 | PepP_1336 | [E] Amino acid transport and metabolism |
| 1.218258 | 2.953197 | 1464 | [transposase]_1464 | [L] Replication, recombination and repaid |
| 0.943904 | 2.598181 | 1487 | transposase_1487 | [L] Replication, recombination and repair |
| 0.959417 | 3.053409 | 1503 | miaA_1503 | [J] Translation, ribosomal structure and biogenesis |
| | 2.306305 | 1518 | pheS_1518 | [J] Translation, ribosomal structure and biogenesis |
| 1.570846 | 4.172276 | 1545 | dnaB_1545 | [L] Replication, recombination and repair |
| 0.927818 | 2.828632 | 1549 | mutM_1549 | [L] Replication, recombination and repair |
| 1.075383 | | 1570 | >>transposase to 1569<< 1570 | [L] Replication, recombination and repair |
| 0.851554 | 1.960626 | 1617 | leuS_1617 | [J] Translation, ribosomal structure and biogenesis |
| 1.255148 | 3.232223 | 1676 | helicase_1676 prediction | [R] General function only |
| 0.883658 | 2.930052 | 1735 | epsC_1735 division, | [D] Cell cycle control, cell chromosome partitioning |
| 0.986686 | 2.513637 | 1736 | epsB_1736 | [M] Cell wall_membrane_envelope biogenesis |
| 0.977143 | 2.051172 | 1768 | lctP_1768 | [C] Energy production and conversion |
| 1.170425 | 2.543868 | 1783 | ABC transporter_1783 | [R] General function prediction only |
| 1.340554 | 3.77469 | 1796 | plnG_1796 | defense mechanisms |
| 0.906982 | 2.589276 | 1823 | [hypo]_1823 | [S] Function unknown |
| 1.138859 | 3.244559 | 1824 | GTP-binding protein_1824 | [J] Translation, ribosomal structure and biogenesis |
| 1.330758 | 3.754055 | 1828 | parB_1828 | [K] Transcriptions |
| 0.923493 | 3.168131 | 1829 | gidB_1829 | [M] Cell wall_membrane_envelope biogenesis |
| 1.24023 | 3.628774 | 1839 | [permease]_1839 | [V] Defense mechanisms |
| 1.194538 | 2.650976 | 1923 | dltD_1923 | [M] Cell wall_membrane_envelope biogenesis |
| 1.139065 | 3.220796 | 1927 | [cons. hypo]_1927 | |
| | | | | |
| | | | | |
| | | | **Induced by LuxS at OD 0.7** | |
| 0.959451 | 2.040127 | 1497 | [unknown]_1497 | [D] Cell cycle control, cell division, chromosome partitioning |
| 1.078681 | 3.075442 | 1796 | plnG 1796 | defense mechanisms |
| 0.940321 | 2.056627 | 1798 | response regulator_1798 | [K] Transcription[T] Signal transduction mechanisms |
| | | | | |
| | | | | |
| | | | **Repressed by LuxS at OD 0.2** | |
| -0.99406 | 2.384669 | 31 | hyp. prot._31 | [R] General function prediction only |
| -0.87543 | 2.238665 | 100 | >>[cons. hypo]<< 100 | |
| -0.88016 | 1.472098 | 154 | ABC transporter_154 | [P] Inorganic ion transport and metabolism |
| -1.09721 | 3.710204 | 166 | K+ uptake protein_166 | [P] Inorganic ion transport and metabolism |
| -0.99234 | 2.213327 | 217 | [cons. hypo. prot.]_217 | [S] Function unknown |
| -0.9436 | 1.630565 | 359 | rib. prot. L11_359 | [J] Translation, ribosomal structure and biogenesis |
| -0.9722 | 2.001816 | 422 | thiored. reductase_422 | [O] Posttranslational modification, protein turnover, chaperones[C] Energy production and conversion |
| -0.94949 | 2.438256 | 448 | [hypo]_448 | [U] Intracellular trafficking, secretion, and vesicular transport |
| -0.90283 | 1.84467 | 644 | [hypo]_644 | [Q] Secondary metabolites biosynthesis, transport and catabolism |
| -0.92823 | 2.588025 | 690 | [hypo]_690 | [S] Function unknown |
| -0.90539 | 2.396951 | 1127 | [unknown] 1127 | |
| -0.90928 | 1.738096 | 1156 | [hypo]_1156 | [D] Cell cycle control, cell division, chromosome partitioning |
| -0.87686 | 2.866565 | 1348 | kanamycin kinase_1348 | [J] Translation, ribosomal structure and biogenesis |
| -0.95519 | 1.777933 | 1626 | seryl-trna synthetase 1626 | [J] Translation, ribosomal structure and biogenesis |
| -0.9827 | 3.538901 | 1687 | ansA_1687 | [E] Amino acid transport and metabolism[J] Translation, ribosomal structure and biogenesis |
| -0.9703 | 1.981019 | 1850 | [lysM] 1850 | |
| | | | | |
| | | | | |
| | | | **Repressed by LuxS at OD 0.7** | |
| -0.864 | 2.209396 | 22 | cadX_22 | [K] Transcription |
| -0.92222 | 1.848892 | 26 | [hypo]_26 | SPy2166 |
| -1.006 | 2.026077 | 89 | [cons. hypo]_89 | |
| -1.02247 | 2.659564 | 568 | malate permease_568 | [R] General function prediction only |
| -0.85612 | 1.659479 | 883 | [unknown]_883 | |
| -0.8652 | 2.413007 | 1095 | dinG_1164 | [K] Transcription[L] Replication, recombination and repair |
| -0.95934 | 1.583834 | 1596 | ::[unknown]:: | |

The trehalose hydrolase, *treC* (LBA1014), was overexpressed 2.56 fold in NCFM compared to the LuxS⁻ mutant strain at early-log phase. Previous research reported deficient growth on trehalose of a *L*. *acidophilus* TreC⁻ mutant (11). Therefore, the maximum growth rate *of L. acidophilus* NCFM and the LuxS⁻ mutant on mMRS media containing various sugars was measured in order to determine the possible influence of a *luxS* mutation on sugar utilization (Table10). Growth was not affected by the *luxS* mutation when the strains were grown on MRS or mMRS containing glucose or trehalose. However, the LuxS- mutant exhibited a lower maximum growth rate compared to NCFM on both lactose and sucrose.

**Table 10 Maximum specific growth rate (µ hr⁻¹) on various carbohydrate sources**

| Medium | | NCFM (µ ± SD) | | LuxS⁻ (µ ± SD) |
|---|---|---|---|---|
| MRS | | 0.45 (± 0.005) | | 0.42 (± 0.006) |
| mMRS*^{a}* | Glucose | 0.34 (±0.04) | | 0.32 (± 0.05) |
| mMRS*^{a}* | Trehalose | 0.28 (± 0.003) | | 0.29 (± 0.02) |
| mMRS*^{a}* | Lactose | 0.28 (±0.004) | * | 0.19 (± 0.006) |
| mMRS*^{a}* | Sucrose | 0.33 (±0.003) | * | 0.23 (± 0.003) |

| | | | | |
|---|---|---|---|---|
| *Identifies significantly different growth rates as determined by the Student's t test (P < 0.01) *^{a}* Medium composition described above | | | | |

The transcriptional profile of each strain from early to late-log phase was also examined (Fig. 9). A similar number of genes, 27 in NCFM and 41 in the LuxS⁻ mutant, increased in expression from early to mid-log phase in both strains. The expression of 12 genes increased in both strains and included sugar metabolism genes (LBA0874, LBA1012, LBA1812, and LBA1974), and a putative myosin-crossreactive antigen (LBA555). Only 24 genes in the wild type strain, compared to 97 genes in the mutant strain, decreased in expression from early to mid-log phase. Again, from middle to late-log phase, the expression of a similar number of genes increased in both strains, but 45 genes decreased in expression during this growth phase in the wild type, and only 15 in the LuxS⁻ mutant. These results suggest that AI-2 could be responsible for increasing or maintaining the expression of genes during the early stages of growth. When AI-2 is deficient in the media, the expression of a significant number of genes appears to be decreased during the early log phase.

### Stress experiments.

Both *L. acidophilus* NCFM and NCK1818 were grown in MRS broth at 37°C until the population reached OD₆₀₀ 0.2, 0.7 and 1.2, at which point cells were collected for stress tolerance experiments.

*Bile tolerance.* At the predetermined sampling points, each culture was diluted and plated in duplicate on MRS agar supplemented with 0.75, 1.0, or 2.0 % w/w Oxgall (BD Biosciences, San Jose, CA). The plates were incubated anaerobically for 48 hrs and CFU/ml and percent survival were calculated. Each assay was performed in triplicate.

*Heat tolerance.* Each strain was grown in 250 ml of MRS broth and at each sampling point, 10 ml of cells were harvested from each population by centrifugation at 3,150 x g for 10 min at 21°C. Following centrifugation, the supernatant was discarded and cell pellets resuspended in 10 ml fresh MRS, preincubated to 55°C. Samples were taken at 0, 10, 20, 30, and 45 min, diluted, and plated in duplicate. Each assay was performed in triplicate.

*Statistical analysis.* Data obtained from the above experiments were analyzed using the Student's *t* test with *P* < 0.05 considered significant.

The involvement of LuxS with various stress responses *of L. acidophilus* NCFM was tested. Growth on MRS agar supplemented with 2.0 % Oxgall (dehydrated fresh bile) significantly decreased the growth of the LuxS⁻ mutant compared to NCFM when bacterial populations were plated at OD₆₀₀ 0.2. When cells were plated from OD₆₀₀ 0.7 populations, a significant decrease in growth was observed in the LuxS⁻ strain on MRS supplemented with 1.0 % Oxgall. Cells harvested from the final time point, OD₆₀₀ 1.2, showed no difference in growth on Oxgall (Fig 10). These results indicate that AI-2 production correlates with bile tolerance through mid-log phase, but as the LuxS⁻ population reaches stationary phase, sensitivity to bile due to the absence of AI-2 is not present.

Additionally, when populations harvested from OD₆₀₀ 0.2 and OD₆₀₀ 0.7 were exposed to 55°C heat stress, the LuxS⁻ mutant was more sensitive (Fig 11). Cell populations of the LuxS⁻ mutant harvested from the final time point, OD₆₀₀ 1.2, did not show a significant decrease when compared to NCFM. This heat-stress survival pattern further implicates the involvement of AI-2 with stress response during the early and middle-log phases of growth, but not as the population approaches stationary growth phase where the cells were inherently more heat tolerant.

### Bacteriocin production

Five µl of both *L*. *acidophilus* NCFM and NCK1818 were spotted onto MRS agar and incubated at 37°C overnight in an anaerobic chamber. The following day, 100 µl of the indicator strain *Lactobacillus delbrueckii* (NCK235) was added to 10 ml of molten MRS overlay agar (0.75% w/v) and poured evenly onto the surface of the agar plate. After 24 hours of incubation, zones of inhibition indicating antagonistic activity of lactacin B, were evaluated.

### Growth curves

*L. acidophilus* NCFM and NCK1818 were both transferred three times from frozen stock cultures in MRS and modified MRS (mMRS). The mMRS media used in this study followed the ingredients for commercially available MRS (Becton, Dickinson and Company, Sparks, MD), replacing dextrose with 1 % w/v of either glucose, lactose, trehalose, or sucrose. Growth curves were performed at 37°C in 96 well plates (Corning) containing 200 µl of each supplemented semi-defined medium or MRS broth. Cultures were allowed to grow for 16 hours and OD₆₀₀ was measured every 15 min in triplicate wells. Plates were incubated at 37°C, and growth was automatically monitored by determining the changes in A₆₀₀ as a function of time using a FLUOStar OPTIMA microtiter plate reader (BMG Labtech). The maximum specific growth rate was calculated from the slope of a linear regression line during exponential growth with a correlation coefficient (*r*²) of 0.99. Each point represented the mean of three independent cultures.

### Conclusions

This application is the first to report a transcriptional analysis of the LuxS regulon at different stages of growth. Previous microarray studies of the gene expression modulated by AI-2 have either studied the response of a LuxS⁻ mutant strain to exogenous AI-2 (DeLisa, et al. (2001) Journal of Bacteriology 183:5239-5247) or identified a single growth point and analyzed the transcriptional difference between an insertional mutant and the wild type (Merritt, et al. (2003) Infect Immun 71:1972-9; Wang, et al. (2005) Journal of Bacteriology 187:8350-8360; Yuan, et al. (2005) Infect. Immun. 73:4146-4154). While these approaches can successfully identify genes regulated by AI-2 at single points, the expression profile only represents a snapshot of the LuxS regulon. In order to gain a more complete understanding of gene expression influenced by LuxS, we selected three time points throughout the growth phase *of L. acidophilus* NCFM for transcriptional analysis. At the first sampling point, OD₆₀₀ 0.2, AI-2 activity detected in the growth media was minimal as the populations were in the early stages of logarithmic growth phase. The second sampling point, during mid-log growth (OD₆₀₀ 0.7), was taken during the rapid accumulation of AI-2 in the media. Cells were harvested at OD₆₀₀ 1.2, late-log phase, for the final sampling point, which was approximately 30 minutes after AI-2 levels reached their peak in the media. These points were chosen to represent the gene expression before, during, and after the production of AI-2 as described above. The LuxS⁻ strain used in this study was a gene deletion mutant and therefore was expected to be free of any pleiotropic effects caused by integrations or addition of selected exogenous components to the media.

The largest number of differentially expressed genes was identified during the early-log phase of growth. A relatively high number of genes were induced in the wild type compared to the LuxS⁻ strain, indicating that AI-2 facilitates the expression of certain genes during the early phases of growth. The majority of these genes were related to transcription, translation, and replication. Three genes (LBA772, LBA773, and LBA775) of the operon encoding the F₁F₀-ATPase (Kullen and Klaenhammer (1999) Mol. Microbiol. 33:1152-1161) (LBA772-LBA779) were overexpressed in the wild type strain. The trehalose hydrolase (*treC,* LBA1014) also showed increased expression in the wild type, although no trehalose was present in the media. The absence of AI-2 did not affect the growth of NCFM on trehalose (Table 5), while neither a TreB⁻ (transporter) or TreC⁻ mutant strain were able to grow on trehalose (Duong, et al (2006) Appl. Environ. Microbiol. 72:1218-1225). The differential expression of these putative energy production systems further associates LuxS with growth and metabolism. Similar results obtained by DeLisa et al. (2001, Journal of Bacteriology 183:5239-5247) implicated AI-2 with regulation of cell division, DNA processing, and morphological processes.

In addition to normal growth and metabolic processes, LuxS affected the expression of various cell-surface factors. Two genes (LBA1735 and LBA1736) of a putative exopolysaccharide (EPS) operon were also differentially expressed in the wild type in early-log phase. A putative fibronectin-binding protein, previously shown to participate with adhesion to Caco-2 cells (Buck, et al. (2005) Appl. Environ. Microbiol. 71:8344-8351), was also induced during early-log phase by AI-2. Fibronectin is a component of the human intestinal extracellular matrix (ECM) and could be a target for the adhesion of bacterial cells in the intestinal environment (Kapczynski et al. (2000) Curr. Microbiol. 41:136-41). A LuxS⁻ mutant strain of *Lactobacillus reuteri* exhibited decreased ecological performance in the murine gastrointestinal tract (Tannock, 2005, *supra*). Regulating the expression of cell-surface factors early in the growth phase may pre-adapt the bacterium for interaction with a diverse microbial community or ecological performance in the intestinal tract.

Cell-surface associated molecules of intestinal microorganisms are recognized by the host and can be important in both the inflammatory response and maintenance of intestinal homeostatsis (Rakoff-Nahoum, et al. (2004) Cell 118:229-241). One of these bacterial surface molecules, lipoteichoic acid, is bound to the cellular membrane and extends through the cell wall to present itself in the environment of the bacteria. The *dltD* gene (LBA1923), a member of the *dlt* operon responsible for proper D-alanation of lipoteichoic acids in lactobacilli, was induced in the presence of LuxS at early-log growth phase. When a Dlt⁻ mutant of *Lactobacillus plantarum* was exposed to peripheral blood mononuclear cells (PBMCs), the secretion of proinflammatory cytokines TNFα and IL-12 was decreased (Grangette, et al. (2005) Proc. Natl. Acad. Sci. U. S. A 102:10321-10326). The expression of IL-10 by the PBMCs was increased following exposure to the Dlt⁻ mutant, skewing the cytokine profile to an anti-inflammatory response. Regulation by AI-2 of the structure of these lipoteichoic acids could play a role in host-microbe interactions in the intestinal tract.

AI-2 is also thought to participate in the regulation of stress responses in bacteria (Wen, et al. (2004) J. Bacteriol. 186:2682-91; Xavier, et al. (2005) J. Bacteriol. 187:238-248). Accordingly, the expression of multiple genes putatively related with stress responses were influenced by AI-2, including *dnaK* (LBA1247), *grpE* (LBA1248), and *clpX* (LBA847) involved with the removal of misfolded proteins and premature polypeptides produced during heat stress. Analysis of the influence of AI-2 on heat stress survival revealed that when compared to the wild type strain, the LuxS- mutant strain of *L. acidophilus* NCFM was more sensitive to 55°C ) heat stress (Fig 11). This sensitivity was only observed when the bacterial populations were stressed from early or mid-log growth phase. These results are consistent with the increased expression of the heat-shock response genes at the early stages of growth. In a recent study of *Porphyromonoas gingivalis,* a LuxS⁻ mutant strain was more resistant than the wild type to heat stress (Xavier, 2005 *supra*), suggesting strain-dependent alteration of stress response by AI-2

Intestinal bacteria must survive passage through the harsh conditions of the gastrointestinal tract in order to persist in the intestine. One of the hurdles that must be overcome is the exposure to bile in the gastric region. A putative bile salt hydrolase (LBA0892) was induced by LuxS at early-log phase. The LuxS⁻ mutant strain was tested for its tolerance to bile and found to be more sensitive when cultures were harvested from either early or mid-log growth phase. Cultures harvested from late log phase did not exhibit any differences in bile tolerance from the wild type. These results indicate that AI-2 could act to prepare the cell for stressful conditions by positively influencing the expression of genes that have a function with growth and survival in different environmental conditions.

AI-2 was reported to be a quorum sensing factor that accumulates as the environment as cell density increases, although the growth phase in which AI-2 affects gene expression has not been reported. Our results suggest that AI-2 acts on cell populations in the early stages of logarithmic growth, before appreciable accumulation of AI-2. The high number of genes that decreased in expression-from early to mid-log growth phase in the LuxS- mutant strain compared to the wild type supports the hypothesis that LuxS positively influences gene expression early in the growth phase. When gene expression was examined throughout the growth phase, LuxS seems to prepare the population for the stresses and interactive conditions naturally encountered during planktonic growth. It is clear that AI-2 impacts the expression of genes related to rapid growth and stress response of *L. acidophilus* NCFM, as well as possibly influencing host-microbe interactions.

Further preferred aspects and embodiments of the invention are disclosed in the following numbered paragraphs.
1. A method of increasing adhesion in a lactic acid bacteria comprising exposing said bacterium to adhesion adaptive conditions comprising incubating said cells at a concentration of at least 1 x 10⁹ cfu/ml for a time sufficient to increase the adhesion of said lactic acid bacteria.
2. The method of paragraph 1, wherein said adhesion adaptive conditions comprise a concentration of about 1 x 10⁹ cfu/ml of said cells for about 1 hour.
3. The method of paragraph 1, wherein said adhesion adaptive conditions increase the expression of at least one polynucleotide selected from the group consisting of SEQ ID NO: 19, 33, 35 or 37, or a polynucleotide having at least 90% sequence identity to the sequence of SEQ ID NO: 19, 33, 35 or 37.
4. The method of paragraph 2, wherein said method further comprises contacting said lactic acid bacterium to a substrate.
5. The method of paragraph 4, wherein said substrate comprises a cell of the gastrointestinal tract or the urogenital tract.
6. The method of paragraph 5, wherein said cell comprises an epithelial cell or a mucosal cell.
7. The method of paragraph 1, wherein the increased adhesion improves at least one probiotic property of said lactic acid bacterium.
8. The method of paragraph 1, wherein said bacterium is selected from the group consisting of *Lactobacillus acidophilus, L. gasseri, L. johnsonii,* and *L. plantarum.*
9. The method of paragraph 1, wherein said lactic acid bacteria expresses a therapeutic polypeptide.
10. The method of paragraph 9, wherein the therapeutic polypeptide is heterologous to the bacterium.
11. The method of paragraph 5, wherein the cell of the gastrointestinal tract or the urogenital tract is from a human, a domestic animal or an agricultural animal.
12. The method of paragraph 1, wherein the stress-tolerance of said lactic acid bacteria is improved.
13. A lactic acid bacterium produced by the method of paragraph 1.
14. A lactic acid bacterium produced by the method of paragraph 2.
15. A bacterium culture comprising the lactic acid bacterium of paragraph 13.
16. A method of delivering a therapeutic polypeptide to a subject comprising:
   a) providing a lactic acid bacteria having a nucleic acid encoding a therapeutic polypeptide;
   b) exposing said lactic acid bacterium to adhesion adaptive conditions comprising incubating said cells at a concentration of at least 1 x 10⁹ cfu/ml for a time sufficient to increase the adhesion of said lactic acid bacteria;
   c) administering said lactic acid bacteria of step (b) to the subject.
17. The method of paragraph 16, wherein said adhesion adaptive conditions comprise a concentration of about 1 x 10⁹ cfu/ml of said cells for about 1 hour.
18. The method of paragraph 16, wherein said bacterium is selected from the group consisting of *Lactobacillus acidophilus, L. gasseri, L.johnsonii,* and *L*. *plantarum.*
19. The method of paragraph 16, wherein the subject comprises a human, a domestic animal or an agricultural animal.
20. A method of inducing or enhancing autoinducer-2 production in a bacteria comprising introducing into said bacterium at least two heterologous nucleic acid molecules selected from the group consisting of:
   (a) (i) a nucleic acid molecule comprising a nucleotide sequence as set forth in SEQ ID NO: 1 ;
      (ii) a nucleic acid molecule that hybridizes to the complement of the nucleic acid of (a)(i) under stringent conditions, said stringent conditions comprise hybridization in 50% formamide, 1 M NaCl, 1 % SDS at 37°C, and a wash in 0.1 X SSC at 60°C to 65°C;
      (iii) a nucleic acid molecule having at least 90% identity to the nucleotide sequence set forth in SEQ ID NO: 1; or,
      (iv) a nucleic acid molecule encoding a polypeptide having at least 90% identity to SEQ ID NO: 2;
   (b) (i) a nucleic acid molecule comprising a nucleotide sequence as set forth in SEQ ID NO: 3;
      (ii) a nucleic acid molecule that hybridizes to the complement of the nucleic acid of (b) (i) under stringent conditions, said stringent conditions comprise hybridization in 50% formamide, 1 M NaCl, 1 % SDS at 37°C, and a wash in 0.1 X SSC at 60°C to 65°C; and
      (iii) a nucleic acid molecule having at least 90% identity to the nucleotide sequence set forth in SEQ ID NO: 3; or,
      (iv) a nucleic acid molecule encoding a polypeptide having at least 90% identity to SEQ ID NO: 4;
   (c) (i) a nucleic acid molecule comprising a nucleotide sequence as set forth in SEQ ID NO: 15;
      (ii) a nucleic acid molecule that hybridizes to the complement of the nucleic acid of (c)(i) under stringent conditions, said stringent conditions comprise hybridization in 50% formamide, 1 M NaCl, 1% SDS at 37°C, and a wash in 0.1 X SSC at 60°C to 65°C; and
      (iii) a nucleic acid molecule having at least 90% identity to the nucleotide sequence set forth in SEQ ID NO: 15; or,
      (iv) a nucleic acid molecule encoding a polypeptide having at least 90% identity to SEQ ID NO: 16;
   (d) (i) a nucleic acid molecule comprising a nucleotide sequence as set forth in SEQ ID NO: 21 ;
      (ii) a nucleic acid molecule that hybridizes to the complement of the nucleic acid of (d)(i) under stringent conditions, said stringent conditions comprise hybridization in 50% formamide, 1 M NaCl, 1% SDS at 37°C, and a wash in 0.1 X SSC at 60°C to 65°C; and
      (iii) a nucleic acid molecule having at least 90% identity to the nucleotide sequence set forth in SEQ ID NO: 21; or,
      (iv) a nucleic acid molecule encoding a polypeptide having at least 90% identity to SEQ ID NO: 22; and,
   (e) (i) a nucleic acid molecule comprising a nucleotide sequence as set forth in SEQ ID NO: 13;
      (ii) a nucleic acid molecule that hybridizes to the complement of the nucleic acid of (e)(i) under stringent conditions, said stringent conditions comprise hybridization in 50% formamide, 1 M NaCl, 1 % SDS at 37°C, and a wash in 0.1 X SSC at 60°C to 65°C; and
      (iii) a nucleic acid molecule having at least 90% identity to the nucleotide sequence set forth in SEQ ID NO: 13;
      (iv) a nucleic acid molecule encoding a polypeptide having at least 90% identity to SEQ ID NO: 14; wherein said bacterium produces greater amounts of autoinducer-2 than the corresponding control bacterium.
21. The method of paragraph 20, further comprising exposing said bacterium to adhesion adaptive conditions comprising incubating said cells at a concentration of at least 1 x 10⁹ cfu/ml for a time sufficient to increase the adhesion of said lactic acid bacteria.
22. The method of paragraph 21, wherein said adhesion adaptive conditions comprise a concentration of about 1 x 10⁹ cfu/ml of said cells for about 1 hour.
23. The method of paragraph 20, wherein said bacteria is a lactic acid bacteria.
24. The method of paragraph 20, wherein said bacteria is probiotic.
25. The method of paragraph 24, wherein the increased adhesion improves at least one probiotic property of said bacterium.
26. The method of paragraph 23, wherein said lactic acid bacterium is selected from the group consisting of *Lactobacillus acidophilus, L. gasseri, L. johnsonii,* and *L. plantarum.*
27. The method of paragraph 23, wherein said lactic acid bacteria expresses a therapeutic polypeptide.
28. The method of paragraph 27, wherein the therapeutic polypeptide is heterologous to the bacterium.
29. The method of paragraph 23, wherein the stress-tolerance of said lactic acid bacteria is improved.
30. A bacterium comprising at least two heterologous nucleic acid molecules selected from the group consisting of:
   (a) (i) a nucleic acid molecule comprising a nucleotide sequence as set forth in SEQ ID NO: 1;
      (ii) a nucleic acid molecule that hybridizes to the complement of the nucleic acid of (a)(i) under stringent conditions, said stringent conditions comprise hybridization in 50% formamide, 1 M NaCl, 1 % SDS at 37°C, and a wash in 0.1 X SSC at 60°C to 65°C; and
      (iii) a nucleic acid molecule having at least 90% identity to the nucleotide sequence set forth in SEQ ID NO: 1; or,
      (iv) a nucleic acid molecule encoding a polypeptide having at least 90% identity to SEQ ID NO: 2; (b) (i) a nucleic acid molecule comprising a nucleotide sequence as set forth in SEQ ID NO: 3;
      (ii) a nucleic acid molecule that hybridizes to the complement of the nucleic acid of (b)(i) under stringent conditions, said stringent conditions comprise hybridization in 50% formamide, 1 M NaCl, 1 % SDS at 37°C, and a wash in 0.1 X SSC at 60°C to 65°C; and
      (iii) a nucleic acid molecule having at least 90% identity to the nucleotide sequence set forth in SEQ ID NO: 3; or, (iv) a nucleic acid molecule encoding a polypeptide having at least 90% identity to SEQ ID NO: 4;
   (c) (i) a nucleic acid molecule comprising a nucleotide sequence as set forth in SEQ ID NO: 15;
      (ii) a nucleic acid molecule that hybridizes to the complement of the nucleic acid of (c)(i) under stringent conditions, said stringent conditions comprise hybridization in 50% formamide, 1 M NaCl, 1% SDS at 37°C, and a wash in 0.1 X SSC at 60°C to 65°C; and
      (iii) a nucleic acid molecule having at least 90% identity to the nucleotide sequence set forth in SEQ ID NO: 15; or, (iv) a nucleic acid molecule encoding a polypeptide having at least 90% identity to SEQ ID NO: 16;
   (d) (i) a nucleic acid molecule comprising a nucleotide sequence as set forth in SEQ ID NO: 21;
      (ii) a nucleic acid molecule that hybridizes to the complement of the nucleic acid of (d)(i) under stringent conditions, said stringent conditions comprise hybridization in 50% formamide, 1 M NaCl, 1% SDS at 37°C, and a wash in 0.1 X SSC at 60°C to 65°C; and
      (iii) a nucleic acid molecule having at least 90% identity to the nucleotide sequence set forth in SEQ ID NO: 21; or, (iv) a nucleic acid molecule encoding a polypeptide having at least 90% identity to SEQ ID NO: 22; and,
   (e) (i) a nucleic acid molecule comprising a nucleotide sequence as set forth in SEQ ID NO: 13;
      (ii) a nucleic acid molecule that hybridizes to the complement of the nucleic acid of (e)(i) under stringent conditions, said stringent conditions comprise hybridization in 50% formamide, 1 M NaCl, 1% SDS at 37°C, and a wash in 0.1 X SSC at 60°C to 65°C; and
      (iii) a nucleic acid molecule having at least 90% identity to the nucleotide sequence set forth in SEQ ID NO: 13;
      (iv) a nucleic acid molecule encoding a polypeptide having at least 90% identity to SEQ ID NO: 14; wherein said bacterium produces greater amounts of autoinducer-2 than the corresponding wild-type bacterium.
31. The bacteria of paragraph 30, wherein said bacteria is a lactic acid bacteria.
32. The bacteria of paragraph 30, wherein said bacteria is probiotic.
33. The bacteria of paragraph 31 , wherein said lactic acid bacterium is selected from the group consisting of *Lactobacillus acidophilus, L. gasseri, L. johnsonii,* and *L*. *plantarum.*
34. The bacteria of paragraph 31, wherein said lactic acid bacteria expresses a therapeutic polypeptide.
35. The bacteria of paragraph 34, wherein the therapeutic polypeptide is heterologous to the bacterium.
36. The bacteria of paragraph 31, wherein the stress-tolerance of said lactic acid bacteria is improved.
37. A bacterium culture comprising bacterium of paragraph 30.
38. A method of screening for environmental conditions that increase adhesion of a lactic acid bacterium to a substrate comprising:
   a) subjecting said bacterium to an environmental condition suspected of increasing adhesion; and,
   b) contacting said bacterium to the substrate; wherein an increase in adhesion of the bacterium subjected to said environmental condition to said substrate compared to adhesion of a control bacterium that has not been subjected to said environmental conditions indicates that the environmental condition is effective in increasing adhesion of the bacterium.
39. The method of paragraph 38, wherein said bacterium is selected from the group consisting of *Lactobacillus acidophilus, L. gasseri, L. johnsonii,* and *X. plantarum.*
40. The method of paragraph 38, wherein said substrate comprises a cell of the gastrointestinal tract or the urogenital tract.
41. The method of paragraph 40, wherein said cell comprises an epithelial cell or a mucosal cell.
42. A method of modulating adhesion in a bacterium comprising introducing into said bacterium at least one heterologous nucleic acid molecule selected from the group consisting of:
   a) a nucleic acid molecule comprising a nucleotide sequence as set forth in SEQ ID NO:1, 3, or 21;
   b) a nucleic acid molecule that hybridizes to the complement of the nucleic acid of (a) under stringent conditions, said stringent conditions comprise hybridization in 50% formamide, 1 M NaCl, 1% SDS at 37°C, and a wash in 0.1 X SSC at 60°C to 65°C;
   c) a nucleic acid molecule having at least 90% identity to the nucleotide sequence set forth in SEQ ID NO: 1, 3, or 21; or,
   d) a nucleic acid molecule encoding a polypeptide having at least 90% identity to SEQ ID NO:2, 4, or 22;
      wherein adhesion in said bacterium is modulated when compared to adhesion in a bacterium that does not comprise the heterologous nucleic acid molecule of (a), (b), (c), or (d).
43. The method of paragraph 42, wherein said bacterium is selected from the group consisting of *Lactobacillus acidophilus, L. gasseri, L. johnsonii,* and *L. plantarum.*
44. The method of paragraph 42, wherein said substrate comprises a cell of the gastrointestinal tract or the urogenital tract.
45. The method of paragraph 44, wherein said cell comprises an epithelial cell or a mucosal cell.

## Claims

1. . A method of modulating adhesion in a bacterium comprising introducing into said bacterium at least one heterologous nucleic acid molecule selected from the group consisting of:
a) a nucleic acid molecule comprising a nucleotide sequence as set forth in SEQ ID NO:1, 3, or 21;
b) a nucleic acid molecule that hybridizes to the complement of the nucleic acid of (a) under stringent conditions, said stringent conditions comprise hybridization in 50% formamide, 1 M NaCl, 1% SDS at 37°C, and a wash in 0.1 X SSC at 60°C to 65°C;
c) a nucleic acid molecule having at least 90% identity to the nucleotide sequence set forth in SEQ ID NO: 1, 3, or 21; or,
d) a nuleic acid molecule encoding a polypeptide having at least 90% identity to SEQ ID NO:2, 4, or 22;
wherein adhesion in said bacterium is modulated when compared to adhesion in a bacterium that does not comprise the heterologous nucleic acid molecule of (a), (b), (c), or (d).

2. . The method of claim 1, wherein said bacterium is selected from the group consisting of *Lactobacillus acidophilus, L. gasseri, L*. *johnsonii,* and *L. plantarum,*

3. . The method of claim 1, wherein said substrate comprises a cell of the gastrointestinal tract or the urogenital tract.

4. . The method of claim 3, wherein said cell comprises an epithelial cell or a mucosal cell.
